(19) Europäisches Patentamt
European Patent Office
Office européen des brevets

(11) Publication number: **0 315 456 B1**

# (12) EUROPEAN PATENT SPECIFICATION

(45) Date of publication of patent specification: **01.06.94**

(51) Int. Cl.5: **A61K 39/44**, C12N 15/00

(21) Application number: **88310372.3**

(22) Date of filing: **03.11.88**

The file contains technical information submitted after the application was filed and not included in this specification

## (54) Polysaccharide-modified immunoglobulins having reduced immunogenic potential or improved pharmacokinetics.

(30) Priority: **05.11.87 US 118150**

(43) Date of publication of application:
**10.05.89 Bulletin 89/19**

(45) Publication of the grant of the patent:
**01.06.94 Bulletin 94/22**

(84) Designated Contracting States:
**AT BE CH DE ES FR GB GR IT LI LU NL SE**

(56) References cited:
**WO-A-86/04145**
**GB-A- 1 541 436**

**CHEMICAL ABSTRACTS, vol. 84, no. 13, 29 March 1986, Columbus, OH (US); E. RUEDE et al., p. 356, no. 87841f&NUM;**

(73) Proprietor: **HYBRITECH INCORPORATED**
**11095 Torreyana Road**
**San Diego California 92126(US)**

(72) Inventor: **Fagnani, Roberto Cesare**
**8972 Caminito Fresco**
**La Jolla, CA 92037(US)**

(74) Representative: **Hudson, Christopher Mark et al**
**Erl Wood Manor**
**Windlesham Surrey GU20 6PH (GB)**

EP 0 315 456 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to an immunoglobulin, or fragment thereof, conjugated with modified low molecular weight polysaccharides, preferably an oxidized dextran. Such conjugated immunoglobulins have reduced immunogenicity but show substantially unaltered immunoreactivity and localization properties and substantially unaltered or improved pharmacokinetics in vivo, as compared to the unmodified immunoglobulin.

Monoclonal antibodies, or antibodies conjugated to toxins, drugs or radionuclides, have been used for the diagnosis and treatment of tumors in both animal models and clinical trials. A major difficulty in the clinical application of monoclonal antibodies, however, is that these foreign proteins, by nature being immunogenic, can initiate a immune response by the host against the monoclonal antibody. Such a response may inactivate the biological action of the antibodies and may cause the development of severe adverse pharmacological reactions, including allergic reactions or serum sickness.

Likewise, some human diseases are characterized by a deficiency of certain enzymes, hormones, or other functionally active proteins. These clinical conditions can be managed effectively with the administration of the corresponding foreign proteins. Examples of such conditions may include, for example, diabetes, which can be controlled effectively by the repeated administration of insulin.

In recent years, the therapeutic administration of foreign enzymes has been applied to a variety of human diseases such as the treatment with L-asparaginase in leukemias, adenosine-deaminase in severe combined immunodeficiency, streptokinase-streptodornase in myocardial infarctions and other cardiovascular conditions, and other diseases well characterized in modern medicine. The therapeutic administration of foreign enzymes, however, also has resulted in the immunological reaction of the host against such antigenic proteins, resulting in the inactivation of normal biological activity, the development of allergic reactions, and the rapid removal of the enzymes from the circulation.

In certain cases, the modification of enzymes with water-soluble polymers has been shown to make the enzymes less susceptible to in vivo degradation, increase the circulating half-life of the enzyme in the blood, and such modification may result in a protein of lowered immunogenicity which still retains enzymatic activity. The polymeric substance in such cases, preferably, is water-soluble and slowly degradable in vivo. Water-soluble polymers, including polyethylene glycol (PEG) and high molecular weight dextrans, have been used for such modification of enzymes. See, for example, U.S. Pat. No. 4,055,635; T. E. Wileman, et al., J. Pharm. Pharmacol., 38 264 (1986); G. Mitra, et al., Biotechnology and Bioengineering, 26 452 (1984); U.S. Pat. No. 4,179,337; U.S Pat. No. 3,639,213; J. E. Benbough, et al., Biochemical Pharmacol. 28 833 (1979).

As noted, the administration of antibodies for diagnostic or therapeutic purposes also can result in an immune response by the host, resulting in the loss of biological activity, rapid removal of the antibody from the circulation and severe allergic reactions. For diagnostic or therapeutic applications involving multiple dosing regimens over a period of time, the problem is especially great. Some attempts, including the modification of enzymes using water soluble polymers such as PEG and high molecular weight dextrans, have been made to avoid these undesirable consequences. See, for example, PCT Publication WO 86/04145. However, the adducts described in the prior art possess potentially undesirable characteristics. In particular, the use of high molecular weight polysaccharides and the coupling conditions employed yielded substantially polymeric adducts possessing unfavorable pharmacokinetics and resulting in substantially lowered biological activity. The need remains, therefore, for a useful means for reducing the immunogenicity of such immunoglobulins while maintaining normal or substantially unaltered immunoreactivity, and localization properties and substantially unaltered or improved pharmacokinetics.

In this regard, the present invention provides a substantially monomeric immunoreactive composition which comprises an immunoglobulin, or fragment thereof, said immunoreactive composition being covalently linked to a low molecular weight polysaccharide derivative, preferably an oxidized dextran. Such immunoreactive substances of the invention possess greatly reduced or abrogated immunogenicity while retaining substantially unaltered immunoreactivity, localization properties, and substantially unaltered or improved pharmacokinetics. In addition, the present invention also provides a method for reducing the immunogenicity generated by drugs (such as adriamycin, methotrexate, vinblastine, desacetylvinblastine, cis-platin and others) or metal chelating agents or other molecules, when such compounds are bound to a suitable immunoglobulin.

Further, formulations for in vivo use are provided, which comprise, as an active ingredient, a modified polysaccharide-conjugate immunoreactive composition of the invention, associated with a pharmaceutically-acceptable carrier, diluent or excipient therefor.

In yet a further embodiment of the invention, there is provided a method for the immunotherapy of a patient in need thereof, which comprises administering to said patient a therapeutically-effective amount of an immunoreactive composition of the invention.

As noted, the present invention provides an immunoreactive composition comprising an immunoglobulin, or fragment thereof, said immunoreactive composition being covalently linked to a low molecular weight polysaccharide derivative, preferably a modified dextran.

The preferred immunoreactive composition comprises an antigen-recognizing immunoglobulin (antibody), or fragment thereof. Particularly preferred immunoglobulins are those immunoglobulins which can recognize tumor associated antigens. As used, "immunoglobulin" may refer to any recognized class or subclass of immunoglobulins such as IgG, IgA, IgM, IgD, or IgE. Preferred are those immunoglobulins which fall within the IgG class of immunoglobulins. As noted, one skilled in the art will appreciate that the invention also encompasses the use of antigen recognizing immunoglobulin fragments. Such immunoglobulin fragments may include, for example, the Fab′, $F(ab')_2$, or Fab fragments, or other antigen recognizing immunoglobulin fragments. Such immunoglobulin fragments can be prepared, for example, by proteolytic enzyme digestion, for example, by pepsin or papain digestion, reductive alkylation, or recombinant techniques. The materials and methods for preparing such immunoglobulin fragments are well-known to those skilled in the art. See generally, Parham, J. Immunology, 131, 2895 (1983); Lamoyi et al., J. Immunological Methods, 56, 235 (1983); Parham, id., 53 133 (1982); and Matthew et al., id., 50 239 (1982).

The immunoglobulins or fragments thereof, when conjugated to a low molecular weight polysaccharide derivative, possess low or greatly reduced immunogenicity while retaining substantially unaltered immunoreactivity and localization properties, substantially unaltered or improved pharmacokinetics and, in a particularly preferred embodiment, the ability to localize at tumor sites in vivo.

The immunoglobulin can be derived from any species, preferably, however, from human, murine, or rabbit origins. Also, the immunoglobulin can be a "chimeric antibody" as that term is recognized in the art. Also, the immunoglobulin may be "bifunctional", that is, the antibody may have one arm having a specificity for one antigenic site, such as a tumor associated antigen while the other arm recognizes a different target, for example, a hapten which is, or to which is bound, an agent lethal to the antigen-bearing tumor cell. Such bifunctional antibodies are described, for example, in European Patent Publication, EPA 105360, to which those skilled in the art are referred. Further, for immunodiagnostic or immunotherapeutic purposes, the immunoglobulin portion of the adduct may be labelled with a radioisotope, for example, Tc, $^{125}I$, $^{90}Y$, $^{111}In$, $^{131}I$, or others, by means which are well known to those skilled in the art. A particularly useful method for labelling the immunoglobulin is by use of the chelating agents described in Meares, et al., U.S. Patent No. 4,622,420, which is incorporated herein by reference.

With specific reference to the administration of bifunctional antibodies, particularly those with at least one specificity to a hapten such as a radioisotope, including a radioisotope associated with a chelating agent, one skilled in the art will appreciate that the antibody/hapten complex to be administered to a patient can be formed in vitro prior to administration, or alternatively, the complex can be formed in vivo by separate administration of the hapten and the antihapten antibody. Additionally, if desired, the antihapten antibody may be permitted to distribute itself throughout the patient prior to administration of the hapten. Other means of accomplishing favorable biodistribution of the bifunctional antibody will be apparent to one skilled in the art. In this regard, the disclosure in European Patent Publication EP A 0217577 (April 8, 1988), incorporated herein by reference, is especially useful.

As noted, particularly preferred for use in the invention are monoclonal antibodies which are characterized in the art as hybrid or bifunctional antibodies. In accordance with the present invention, the hybrid antibodies have a dual specificity, preferably with one or more binding sites specific for the hapten of choice and one or more binding sites specific for a target antigen, for example, an antigen associated with a tumor, an infectious organism, or other disease state. Among such antigens may be mentioned tumor-associated antigens such as the antigen recognized by KS1/4, carcinoembryonic antigen (CEA), prostatic acid phosphatase (PAP), prostate specific antigen (PSA), human chorionic gonadotropin (HCG), ferritin, bombesin, melanoma associated antigens p97 and gp 240, as well as those noted below. Such hybrid or bifunctional antibodies may be derived either biologically, by cell fusion techniques, or chemically, especially with cross-linking agents or disulfide bridge-forming reagents, and may be comprised of whole antibodies and/or fragments thereof. Methods for obtaining such hybrid antibodies are disclosed in PCT application W08303679 (October 27, 1983) and European Application EPA 0 217 577, supra, both of which are incorporated herein by reference. Particularly preferred bifunctional antibodies are those synthetically prepared with cross-linking agents such as bis-(maleimido)-methyl ether ("BMME"), or with other cross-linking agents familiar to those skilled in the art.

As noted, also especially preferred for use in the present invention are chimeric monoclonal antibodies, preferably those chimeric antibodies having hapten-binding specificity or specificity toward a tumor associated antigen. As used herein, the term "chimeric antibody" refers to a monoclonal antibody comprising a variable region, i.e., binding region, and at least a portion of a constant region derived from a different species, usually prepared by recombinant DNA techniques. Chimeric antibodies comprising a murine variable region and a human constant region are especially preferred in certain applications of the invention, particularly human therapy, as such antibodies may be less immunogenic than purely murine monoclonal antibodies. Such murine/human chimeric antibodies are the product of immunoglobulin genes comprising DNA segments encoding murine immunoglobulin variable regions and DNA segments encoding human immunoglobulin constant regions. Methods for producing chimeric antibodies involve conventional recombinant DNA and gene transfection techniques well known to the art. Morrison, S.L., et al., Proc. Nat'l Acad. Sci., 81:6851 (1984). Particularly preferred are those chimeric antibodies which recognize the antigens outlined above for the bifunctional antibodies.

Encompassed by the term "chimeric antibody" is the concept of "humanized antibody", that is those antibodies in which the framework or "complementarity determine regions ("CDR") have been modified to comprise the CDR of an immunoglobulin of different specificity as compared to that of the parent immunoglobulin. In a preferred embodiment, a murine CDR is grafted into the framework region of a human antibody to prepare the "humanized antibody". Particularly preferred CDR'S correspond to those representing sequences recognizing the antigens noted above for the chimeric and bifunctional antibodies. The reader is referred to the teaching of EP A 0 239 400 (September 30, 1987), incorporated herein by reference, for its teaching of CDR modified antibodies.

In a most preferred embodiment of the invention, one skilled in the art will recognize that a bifunctional-chimeric antibody can be prepared which would have the benefits of lower immunogenicity of the chimeric or humanized antibody, as well as the flexibility, especially for therapeutic treatment, of the bifunctional antibodies described above. Such bifunctional-chimeric antibodies can be synthesized, for instance, by chemical synthesis using cross-linking agents and methods of the type described above. In any event, the present invention should not be construed as limited in scope by any particular method of production of bifunctional chimeric antibodies.

In addition, the invention encompasses within its scope immunoglobulins or immunoglobulin fragments to which are fused active proteins, for example, an enzyme of the type disclosed in Neuberger, et al., WO 86/01533, Published March 13, 1986. The disclosure of such products is incorporated herein by reference. Of course, in such a case, one skilled in the art will recognize that care must be taken to avoid dextran modification which would inactivate the normally active protein fused to the immunoglobulin. Whether such inactivation has occurred is a simple matter to determine and the methods outlined in Neuberger, et al., supra, would be sufficient for such purposes.

As noted, "bifunctional", "fused", "chimeric" (including humanized), and "bifunctional-chimeric" (including humanized) antibody constructions also include, within their individual contexts constructions comprising antigen-recognizing fragments. As one skilled in the art will recognize, such fragments could be prepared by traditional enzymatic cleavage of intact bifunctional, chimeric, humanized, or chimeric-bifunctional antibodies. If, however, intact antibodies are not susceptible to such cleavage, because of the nature of the construction involved, the noted constructions can be prepared with immunoglobulin fragments used as the starting materials; or, if recombinant techniques are used, the DNA sequences, themselves, can be tailored to encode the desired "fragment" which, when expressed, can be combined in vivo or in vitro, by chemical or biological means, to prepare the final desired intact immunoglobulin "fragment". It is in this context, therefore, that the term "fragment" is used.

Furthermore, the immunoglobulin (antibody), or fragment thereof, used in the present invention may be polyclonal or monoclonal in nature. Monoclonal antibodies are the preferred immunoglobulins, however. The preparation of such polyclonal or monoclonal antibodies now is well-known to those skilled in the art who, of course, are fully capable of producing useful immunoglobulins which can be used in the invention. See, e.g., G. Kohler and C. Milstein, Nature 256 495 (1975). In addition, hybridomas and/or monoclonal antibodies which are produced by such hybridomas and which are useful in the practice of the present invention are publicly available from sources such as the American Type Culture Collection, 12301 Parklawn Drive, Rockville, Maryland 20852 or, for example, Boehringer-Mannheim Biochemicals, P.O. Box 50816, Indianapolis, Indiana 46250.

Particularly preferred monoclonal antibodies are those which recognize tumor associated antigens. Such monoclonal antibodies, are not meant to be so limited, however, and may include, for example, the following:

| Antigen Site Recognized | Monoclonal Antibodies | Reference |
|---|---|---|
| Lung Tumors | KS1/4 | N. M. Varki, et al., Cancer Res. 44:681, 1984. |
| | 534,F8;604A9 | F. Cuttitta, et al., in: G. L. Wright (ed) Monoclonal Antibodies and Cancer, Marcel Dekker, Inc., N.Y., p. 161, 1984. |
| Squamous Lung Cancer | G1, LuCa2, LuCa3, LuCa4 | Kyoizumi et al., Cancer Res., 45:3274, 1985. |
| Small Cell Lung Cancer | TFS-2 | Okabe et al., Cancer Res. 45:1930, 1985. |
| Colon Cancer | 11.285.14 14.95.55 | G. Rowland, et al., Cancer Immunol. Immunother., 19:1, 1985. |
| | NS-3a-22,NS-10 NS-19-9,NS-33a NS-52a,17-1A | Z. Steplewski, et al., Cancer Res., 41:2723, 1981. |
| Carcinoembryonic Antigen (CEA) | MoAb 35 (or ZCE025) | Acolla, R.S. et al., Proc. Natl. Acad. Sci., (USA), 77:563, 1980. |
| | CEM231 | Hybritech, Inc., 11095 Torreyana Road San Diego, CA 92121 |
| Melanoma | 9.2.27 | T. F. Bumol and R. A. Reisfeld, Proc. Natl. Acad. Sci., (USA), 79:1245, 1982. |

| | | |
|---|---|---|
| p97 | 96.5 | K. E. Hellstrom, et al., Monoclonal Antibodies and Cancer, loc. cit. p. 31. |
| Antigen T65 | T101 | Boehringer-Mannheim, P.O. Box 50816, Indianapolis, IN 46250 |
| Ferritin | Antiferrin | Boehringer-Mannheim, P.O. Box 50816, Indianapolis, IN 46250 |
| | R24 | W. G. Dippold, et al., Proc. Natl. Acad. Sci. (USA), 77:6114, 1980. |
| Neuro-blastoma | Pl 153/3 | R. H. Kennet and F. Gilbert, Science, 203:1120, 1979. |
| | MIN 1 | J. T. Kemshead in Monoclonal Antibodies and Cancer, loc. cit. p. 49. |
| | UJ13A | Goldman et al., Pediatrics, 105:252, 1984. |
| Glioma | BF7,GE2,CG12 | N. de Tribolet, et al., in Monoclonal Antibodies and Cancer, loc. cit. p. 81. |

| | | |
|---|---|---|
| Breast Cancer | B6.2,B72.3 | D. Colcher, et al., in Monoclonal Antibodies and Cancer, loc. cit. p. 121. |
| Osteogenic Sarcoma | 791T/48, 791T/36 | M. J. Embleton, ibid, p. 181 |
| Leukemia | CALL 2 | C. T. Teng, et al., Lancet, 1:01, 1982. |
| | anti-idiotype | R. A. Miller, et al., N. Eng. J. Med., 306:517, 1982. |
| Ovarian Cancer | OC 125 | R. C. Bast, et al., J. Clin. Invest., 68:1331, 1981. |
| Prostate Cancer | D83.21, P6.2, Turp-27 | J. J. Starling, et al., in Monoclonal Antibodies and Cancer, loc. cit. p. 253. |
| Renal Cancer | A6H, D5D | P. H. Lange, et al., Surgery, 98:143, 1985. |

As one skilled in the art will recognize, drugs, for example, adriamycin, methotrexate, vinblastine, as well as metals chelated via a chelating agent, for example, when bound to immunoglobulins, can result in highly immunogenic entities. Such an immunoglobulin bound to a drug or chelated metal can be used in the immunoglobulin portion of the immunoreactive compositions of the invention.

As used, "substantially unaltered" immunoreactivity means that the covalently linked immunoreactive substance retains at least about 70% of the immunoreactivity of that observed for the free (i.e. non-linked) immunoglobulin or fragment used. Lower immunoreactivities also fall within the scope of the present invention but, as one skilled in the art will recognize, greater amounts of the linked immunoglobulin may have to be administered to accomplish the same result as that observed for more highly immunoreactive covalently linked products. In addition, the linkage may result in substantially unaltered or improved pharmacokinetics over the unlinked immunoglobulin, particularly in the case of an immunoglobulin fragment, and may allow one to use the covalently linked antibody, or fragment thereof, even if the immunoreactivity is lower than preferred. Such adducts, therefore, also should be considered within the scope of the invention.

In addition, the immunoreactive compositions of the invention have substantially unaltered or improved pharmacokinetics as compared to the non-linked immunoglobulin, or fragment used. In this context, the term "substantially unaltered or improved pharmacokinetics" is meant to indicate that the immunoreactive compositions have a biodistribution, plasma half-life, elimination rate, etc. similar to, the same as or improved over that of the unlinked substance so as to retain the ability to recognize the desired antigenic site and, if a tumor cell is involved, to target the desired cell in a manner like that of the unlinked immunoglobulin. As used, therefore, "improved pharmacokinetics" means a generally more favorable circulating plasma half-life, lack of abnormal non-specific accumulation in particular organ(s), such as the liver, the kidney, the nervous system etc., and a favorable body elimination rate.

Preferably, the compositions of the invention will provide generally improved pharmacokinetics. In particular, it is preferred that an immunoglobulin of the invention, or fragment thereof, possess low kidney uptake, as compared to the unmodified immunoglobulin or fragment, and most preferably, such immunoglobulin or fragment may possess an increased plasma half-life.

Thus, in an additional embodiment, there is provided a method for improving the pharmacokinetics of an immunoglobulin or antigen-recognizing fragment thereof, which comprises covalently linking an immunoglobulin, or antigen-recognizing fragment thereof, to a low molecular weight polysaccharide derivative.

"Immunogenicity" refers to the ability of a given molecule or a determinant thereof to induce formation of antibodies upon administration, whether in vivo or in vitro, and to bind to such antibodies. "Antigenicity" is defined as the ability of a given molecule or a determinant thereof to be recognized by an antibody. As used, therefore, "reduced immunogenicity", "reduced immunogenic potential", "lowered immunogenicity", "lowered immunogenic potential", or similar terms are meant to convey that the covalent polysaccharide-modified immunoglobulins of the present invention, relative to the non-linked immunoglobulins, produce a much lowered immune response upon administration to the host, including for example, development of anti-antibodies, allergic (anaphylactic) reactions, or serum sickness. Because of such reduced immunogenicity, the presently disclosed dextran-immunoglobulin adducts can be administered multiple times without resulting in such unfavorable immune responses.

The immunoglobin, or fragment thereof, is linked to a low molecular weight, modified polysaccharide. The preferred polysaccharide is a dextran of low molecular weight, preferably having a molecular weight of from about 3,000 to about 16,000. Of this molecular weight range, especially preferred are those dextrans with a molecular weight of from about 6,000 to about 16,000. Suitable dextrans are available commercially from, for example, Dextran Products Ltd., Scarborough, Ontario, Canada.

The immunoglobulins are linked covalently to the desired dextran through accessible amino groups present on the immunoglobulin. In particular, the preferred dextran starting material is activated by oxidizing the reactive hydroxyl groups on the dextran to active aldehyde groups, in the presence of a suitable oxidizing agent, preferably sodium periodate ($NaIO_4$).

When sodium periodate is used as the oxidizing agent, the activation reaction conveniently is carried out in buffer solution, for example, sodium borate buffer, at reduced temperature, typically at about 0°C to about room temperature. The reaction mixture is stirred in the dark for about 24 hours after which time the reaction may be stopped by destroying excess oxidation reagent, for example, by the addition of an excess of a compound containing free vicinal hydroxyl groups such as ethylene glycol. To obtain the desired activated dextran, the reaction mixture can be purified, for example, by dialysis against distilled water followed by lyophilization of the product.

Other means of activating the dextran so that it will react with the immunoglobulin, directly or through bridging linkages, will be apparent to those skilled in the art and are encompassed also within the teaching of the present invention.

After the dextran starting material has been activated, it can be mixed, in a suitable solution, for example, buffer solution at a pH of about 6.5 to about 7.5, with the desired immunoglobulin. In so doing, the aldehyde groups of the oxidized dextran can react with the available free amino groups of the immunoglobulin to form a Schiff base adduct.

The Schiff base adduct produced by the reaction above then is reduced in the presence of a suitable mild reducing agent. The preferred reducing agent for this purpose is sodium cyanoborohydride ($NaBH_3CN$). The reducing agent is used in slight excess to the dextran-immunoglobulin Schiff base adduct, and the reaction mixture is stirred at 0°C to about 25°C, until the desired degree of reduction has occurred.

In performing the reduction reaction, it has been found that certain reaction conditions are preferred so that the formation of undesirable high molecular weight polymeric aggregates is avoided. Avoidance of such polymers is desirable so that one obtains a product retaining optimal antibody reactivity and pharmacokinetics.

In particular, the reduction reaction should not continue for long periods of time, from about 15 minutes to about 3 hours, being preferred. Further, a reducing agent to dextran molar ratio not exceeding from about 1:1 to about 5:1 produces the desired substantially monomeric dextran-immunoglobulin adduct and largely prevents the formation of undesirable polymeric aggregates.

Further, the degree of dextran substitution can be varied according to the molar ratio of the antibody to dextran. In particular, a molar ratio of dextran to antibody of about 500:1 to about 2500:1 provides ideal derivatization conditions such that the higher the noted molar ratio, the greater the degree of dextran substitution. For intact antibody, the range of average substitution per molecule can be adjusted to be from about one dextran molecule per molecule of antibody, to over about 20, with the preferred substitution ranging from an average of about ten to twenty dextran molecules per molecule of intact antibody.

Under the reaction conditions noted above, high molecular weight polymeric aggregates are largely avoided and only moderate levels of dimerization (from about 10% to about 25% of total proteins) are observed. In particular, the degree of dimerization increases at dextran:antibody molar ratios of less than

about 1000:1 but decreases significantly at molar ratios of greater than about 2000:1. At this latter molar ratio only about 10% or less of dimerization is observed. Thus, as used, "substantially monomeric immunoreactive substance" means an immunoreactive composition containing no more than about 25% of total protein content as dimeric products resulting from the derivatization reaction. It bears emphasizing that polymeric aggregates should always be kept at a minimum level. The levels of such products can be determined easily by standard SDS-gel electrophoresis or gel filtration HPLC analysis.

After the reduction reaction, the dextran linked immunoglobulin can be separated from unreacted starting materials, including unreacted immunoglobulin and unreacted oxidized polysaccharide, by methods commonly available to one of ordinary skill in the art. Suitable methods may include chromatography, for example gel filtration chromatography, using generally available support resins such as Pharmacia Sephacryl S-300 or Sephadex G75 (Pharmacia, Piscataway, New Jersey), or ACA54 (IBF, Villeneuve-La-Garenne, France).

One skilled in the art may want to quantify the degree of dextran substitution on the final linked immunoglobulin of the invention. For the most accurate determination, one will realize that it is essential that the purification methods used to remove unreacted oxidized dextran from the oxidized dextran bound to the immunoglobulin be as effective as possible. Incomplete separation of these two species will result in the contamination of the adduct with free dextran oxidized, thereby resulting in an overestimation of the level of substitution.

A suitable assay for the quantification of dextran bound to the immunoglobulin, particularly an IgG, is the anthrone/sulfuric acid method. (Dreywood, R., Ind. Eng. Chem. Anal. Ed., 18 499 (1946); Morris, D.L., Science, 107 254 (1948)). Because of the low level of glycosylation of immunoglobulins of the G class (2-5% of the total molecular weight of IgG is composed of various sugars) and because of the low amount of antibody used in the assay, determination of the level of dextran binding with this method is not significantly affected by the protein backbone. This assay, therefore, provides a direct indication of the extent of dextran substitution.

The determination of dextran substitution also serves another important purpose. The initial oxidation of dextran results in the generation of aldehyde groups in the dextran molecule. For dextrans having a molecular weight of about 6000, the maximum theoretical number of aldehydes is about 67, assuming complete oxidation of every glucose subunit and the terminal hydroxyl group. Because only a relatively small number of these groups participate in the linkage with the antibodies, the purified immunoglobulin-polysaccharide adducts probably still contain a significant number of free aldehyde groups. In solution, these available groups can react with adjacent antibody molecules to form high molecular weight aggregates. Such aggregates exhibit undesirable pharmacokinetics because they tend to localize in the liver and other organs of the reticuloendothelial system. In this regard, see Figure 1b. In addition, such aggregates tend to precipitate out of solution with time, thus raising a concern about stability of the antibodies. This potential shortcoming can be avoided with the reduction of free aldehyde groups with a suitable reducing agent, preferably $NaBH_4$. By virtue of its relatively strong reducing activity, however, it is important that the amount of $NaBH_4$ be estimated carefully. This two-step reaction procedure (i.e. reduction of the Schiff bases and then any free aldehyde groups) is an essential condition for optimal modification of antibodies with dextran. Substitution of a strong reducing agent, such as $NaBH_4$, for the less active agent, such as $NaBH_3CN$, for the simultaneous one-step reduction of the Schiff base as well as free aldehyde groups on dextran molecules is not recommended. By virtue of its strong reducing ability, $NaBH_4$, for example, is known to reduce aldehydes as well as Schiff bases, thus requiring a gradual, step-wise incremental addition of this reagent to the antibody-dextran mixture after Schiff base formation.

Moreover, the large excess of dextran employed in the derivatization conditions would require the addition of a very large amount of $NaBH_4$ which could affect adversely the biological activity of the antibodies produced.

The novel modified polysaccharide immunoglobulin adducts of the invention are useful in in vivo immunodiagnosis and immunotherapy, particularly in the treatment of cancers if the immunoglobulin portion of the product has tumor antigen recognizing characteristics. Thus, in a further embodiment of the invention, there is provided a method for the immunotherapy of a patient in need thereof, which comprises administering to said patient a therapeutically-effective amount of an immunoreactive composition provided by the invention.

The immunodiagnostic/immunotherapeutic adducts of the invention are prepared preferably for use in formulations suitable for injection. Thus, there also is provided a pharmaceutical formulation, for example, an injectable preparation, comprising a dextran-immunoglobulin adduct of the invention together with a pharmaceutically-acceptable excipient, carrier or diluent therefor, such as are well known in the immunodiagnostic and immunotherapeutic arts. Such formulations are preferably in unit dosage form, each

dosage containing, for example, from about 0.01 to about 200 mg of the active immunoglobulin adduct. Preferred formulations contain from about 0.01 to about 100 mg of the active immunoglobulin adduct.

Typical pharmaceutically-acceptable excipients, diluents and carriers for parenteral formulations may include, for example, isotonic saline, dilute aqueous dextrose (eg. 5%), the polyhydric aliphatic alcohols or mixtures thereof, for instance glycerin, propylene glycol, polyethylene glycol, and the like. Parenteral solutions may also contain preservatives such as phenethyl-alcohol, methyl and propyl parabens, thimerosal and the like. If needed, about 0.05 to about 0.20 percent by weight of an antioxidant such as sodium metabisulfite or sodium bisulfite can be employed also. "Pharmaceutically-acceptable" refers to those excipients, diluents or carriers which are useful in the treatment or diagnosis of a warm-blooded animal. The novel adducts are effective over a wide dosage range depending on factors such as the immunoreactivity of the adduct involved, disease state being treated or to be diagnosed, manner of administration, age and condition of the patient as well as other factors to be determined by the physician.

| | |
|---|---|
| Figure 1a | shows the biodistribution of $^{125}$I-T101-dextran adducts. |
| Figures 1b, 1c and 1d | show the pharmacokinetics of $^{111}$In-T101 dextran adducts. |
| Figure 2 | shows an evaluation of the immunogenicity of T101-dextran adducts. |
| Figure 3 | shows the rate of clearance of T101 from rabbits immunized with T101, T101-dextran (10) and T101-dextran (17). |
| Figures 4a, 4b and 4c | show the biodistribution of rabbit anti-human ferritin ("RAF"), RAF-dextran (8) and RAF-dextran (16), respectively. |
| Figure 5a | shows the immunogenicity of RAF-dextran adducts. |
| Figure 5b | shows the long-term immunogenicity studies of RAF-dextran adducts. |
| Figure 6 | shows the immunogenicity of human IgG-dextran (m.w. 16,000) conjugates. |
| Figure 7 | shows the biodistribution of KS1/4 dextran adducts. |
| Figure 8 | shows the localization of KS1/4-dextran (10) adducts in a tumor xenograft. |
| Figures 9a and 9b | show the plasma kinetics and localization of ZCE025-Fab′-dextran adducts in a tumor xenograft. |
| Figures 10a and 10b | show the levels of ZCE025 $F(ab')_2$-dextran adducts in kidney and plasma, respectively. |
| Figure 11 | shows the tumor localization of ZCE025 $F(ab')_2$-dextran adducts. |
| Figures 12a and 12b | show the pharmacokinetics of CHA-CEM bifunctional antibody-dextran adducts. |
| Figure 13 | shows the tumor localization properties of CHA-CEM bifunctional antibody-dextran adducts. |
| Figures 14a and 14b | show the pharmacokinetics of ZCE025-dextran adducts. |
| Figure 15 | shows the tumor localization of ZCE025-dextran adducts. |
| Figures 16a and 16b | show the biodistribution of an anti-CEA chimeric antibody-dextran adduct. |
| Figure 17 | shows the restriction site and function map of pGCEMK. |
| Figure 18 | shows the restriction site and function map of pNCEMG1. |

One skilled in the art will recognize that other modifications and embodiments exist which will fall within the intended scope of the invention. Thus, the following specific Examples and Preparations are provided to further illustrate the invention but are not meant to be construed as limiting its scope in any way.

## PREPARATION I

## ACTIVATION OF DEXTRANS

Dextrans (Dextran Products Ltd., Scarborough, Ontario, Canada) having molecular weights of about 6,000 ± 500 or 16,000 ±1,000 are used. Activation is effected by oxidation with $NaIO_4$ (Glucose: $NaIO_4$ Molar ratio ≧ 1:2) for 24 hours in the dark at 4°C. Typically, 50 grams of dextran powder are dissolved in 500 ml of 10mM sodium borate buffer, pH 3.1. One hundred twenty grams of $NaIO_4$ in 1.12 L of borate buffer are added slowly, with constant stirring, over a 20 minute period of time. The reaction is allowed to proceed for 24 hours at 4°C in the dark, after which time the reaction may be quenched by adding to the mixture a 10-fold molar excess of ethylene glycol. The mixture then is maintained at room temperature for 2 hours with continuous stirring. The resulting reaction mixture then is dialyzed extensively against sterile distilled water for several days at 4°C. Dialysis is stopped when the outer buffer does not contain residual $NaIO_3$ or $NaIO_4$ as evidenced by a silver nitrate assay in which a few drops of a 1N $AgN0_3$ solution are added to an aliquot of the outer dialysis buffer. The presence of residual $NaI0_3$ or $NaI0_4$ is indicated by the formation of either a white or orange precipate, respectively. After dialysis, the activated dextran solution is

lyophilized to dryness and stored in a desiccator under vacuum until ready to use. Approximately 20-25 grams of activated dextran are recovered, with an approximate yield of 40-50% of starting material. Activated dextran is then characterized by NMR and IR spectroscopy, and by the anthrone/sulfuric acid colorimetric determination indicated in Example 1B.

EXAMPLE I

PREPARATION AND CHARACTERIZATION OF WATER-SOLUBLE CONJUGATES OF T101 MoAb WITH DEXTRAN, MW6000

A. PREPARATION OF THE CONJUGATES

Forty-five milligrams of T101, (commercially available from Boehringer-Mannheim Biochemicals, P.O. Box 50816, Indianapolis, IN 46250; Royston, I. et al., J. Immun. 125 725 (1980).) at 2-5 mg/ml of solution is made in 100 mM sodium phosphate buffer, pH 6.5-7.5. A solution of activated dextran, as prepared in Preparation I, is made by mixing, with gentle swirling, 1.98 grams of powder in 2 ml of the phosphate buffer until completely dissolved. The activated dextran solution then is added to the antibody mixture to make a final volume of 10 ml. Under these conditions, the aldehyde groups of the activated dextran can react with the free amino groups of the antibody to form Schiff bases.

A solution of $NaBH_3CN$, in 100 mM phosphate buffer, then is added, in slight excess, to the concentration of dextran, and the mixture is allowed to react at room temperature for two hours. Under these conditions, $NaBH_3CN$ can reduce Schiff bases to secondary amines, resulting in a covalent linkage of activated dextran to the antibody.

Upon termination of the reaction, T101-Dextran molecules are separated from unreacted dextran by using methods currently known to one skilled in the art. A suitable procedure for instance, is provided by gel filtration chromatography with resins such as Pharmacia Sephacryl S-300 or Sephadex G75 (Pharmacia, Piscataway, New Jersey), or ACA54 (IBF, Villeneuve-La-Garenne, France).

B. QUANTITATION OF DEXTRAN SUBSTITUTION OF T101

This procedure outlines how one determines the number of oxidized dextran molecules linked to each immunoglobulin adduct. For accurate determinations, it is essential that the purification methods, used in paragraph A. above, for the removal of unreacted dextran from dextran bound to T101, be as effective as possible. Incomplete separation of these two species will result in the contamination of the adduct with free dextran. This, of course, will result in overestimating the degree of substitution.

A standard curve is constructed by adding increasing amounts of activated dextran (10 to 100 µg) to 1 ml of sodium phosphate buffer, pH 7.0, in clean glass tubes in duplicate. To this volume, 3 ml of a freshly made anthrone solution (0.2% in 80% $H_2SO_4$) are added rapidly and tubes are mixed on a vortex mixer. Tubes then are transferred immediately to a boiling water bath for 15 minutes, and then cooled in an ice water bath for an additional 15 minutes. Color development is determined by reading the absorbance at 625 nm. in a spectrophotometer. With this assay, non-oxidized dextran or oxidized dextran which has been reduced with $NaBH_4$ does not produce any color. Fifty to 100 µg of either unmodified T101 or T101-dextran adducts then are similarly tested, and their absorbance at 625 nm. is plotted against the standard curve prepared from the previous activated dextran standard spectrophotometric determinations. The amount of activated dextran bound to T101 can be determined from the curve and the molar concentration then is calculated. The concentration of T101, can be determined easily by reading the absorbance at 280 nm and making standard calculations in a manner known in the art. The molar substitution of dextran (m.w. 6,000) per mole of T101 then can be calculated according to the following formula:

$$\frac{150,000}{6,000} \quad X \quad \frac{[\text{Dextran}]\ (\text{mg/ml})}{[\text{Antibody}]\ (\text{mg/ml})}$$

C. REDUCTION OF FREE ALDEHYDE GROUPS PRESENT ON THE IMMUNOGLOBULIN - DEXTRAN ADDUCTS

As noted earlier, any free aldehyde groups remaining on the immunoglobulin-dextran adduct can be detrimental to the quality of the final product because these moieties can lead to the formation of undesirable high molecular weight aggregates. To avoid this problem, the free aldehyde groups are reduced with a reducing agent, preferably $NaBH_4$. To determine the amount of reducing agent to use, the following procedure was used: A $NaBH_4$ dose-escalation experiment was performed by treating small aliquots of the T101-dextran adducts with increasing molar ratios of $NaBH_4$:per each subunit of the oxidized dextran, such as 2.5:1, 5:1, 10:1, 20:1, 30:1 and 40:1. The reaction was allowed to proceed for 2 hours at room temperature with gentle stirring, then for an additional 22 hours at 4°C. Samples then were dialyzed extensively against 100 mM sodium phosphate buffer, pH 7.0, to remove residual $NaBH_4$, and the extent of residual activation of oxidized dextran bound to the antibodies was determined with the anthrone/$H_2SO_4$ method described above. An amount of $NaBH_4$ corresponding to the $NaBH_4$:oxidized dextran molar ratio producing greater than 80% reduction in color development was found sufficient to treat adequately the bulk of T101-dextran adducts with regard to conferring optimal pharmacokinetics and stability properties. One skilled in the art will appreciate that this step should be evaluated carefully for each different antibody to identify the dose of $NaBH_4$ necessary to achieve optimal reduction of activated dextran bound to the antibodies without adversely affecting immunoreactivity or pharmacokinetics.

D. DETERMINATION OF THE IMMUNOREACTIVITY OF T101 DEXTRAN ADDUCTS

1) PREPARATION OF ANTIGEN SOURCE

The Human T-cell line T-8402 [Moore, G.E. et al., In Vitro (Rockville), 8:434, (1973)] known to possess an antigen recognized by T101 was grown to confluency in tissue culture using a suitable medium and harvested by gently scraping the surface of the culture flask with a rubber policeman. After washing three times with sterile phosphate buffered saline (PBS), pH 7.4, the cell pellet was lysed by mixing it with an equal volume of 2% NP-40 in PBS. Five microliters of the resulting cell lysate were spotted directly on clean, dry nitrocellulose discs, and allowed to air dry. Discs were then blocked for two hours at 37°C with a protein-rich, blocking solution, such as that described in Johnson, D., et al. Gene Anal. Techn. 1 3 (1984) (referred therein as "BLOTTO"). The discs then were washed 3 times with sterile PBS and stored until used at 4°C in PBS containing 0.02% $NaN_3$ to prevent bacterial contamination.

2) DESCRIPTION OF THE ASSAY

Nitrocellulose discs containing the T-8402 cell lysate were incubated in BLOTTO containing 1 ng of $^{125}I$-radiolabelled antibody having a specific activity of 10 $\mu$Ci/$\mu$g. After incubation overnight at 4°C, the total radioactivity was determined in a well-type gamma counter. The supernatants then were carefully removed and added to a second set of antigen-coated nitrocellulose discs for a second round of binding. Discs used in the first round were washed with PBS, pH 7.0, containing 0.1% Tween 20 and the radioactivity associated was determined in a gamma counter. The second round of incubation proceeded for 4 hours at 37°C, after which time the discs were again counted for determination of total radioactive content, washed, and bound radioactivity was determined as described above. The total percent immunoreactivity (%I.R.) was calculated according to the following formula:

$$\%\ I.R. = \left[ \frac{\text{Bound cpm 1st round}}{\text{Total cpm 1st round}} + \left( 1 - \frac{\text{Bound cpm 1st round}}{\text{Total cpm 1st round}} \right) X \ \frac{\text{Bound cpm 2nd round}}{\text{Total cpm 2nd round}} \right] X \ 100$$

The immunoreactivity of two representative preparations of T101 containing, respectively, an average of 10 ("T101-dextran (10)") and 17 ("T101-dextran (17)") dextrans per antibody molecules is shown in Table I. Thus, following the described procedure, T101-dextran adducts substantially retain immunoreactivity when compared to the immunoreactivity of the free T101 used as a control.

TABLE I

| | % IMMUNOREACTIVITY (% of Control) |
|---|---|
| T101 (Control) | 100 |
| T101 - Dextran (10) | 89 |
| T101 - Dextran (17) | 83 |

E. PHARMACOKINETICS OF T101-DEXTRAN ADDUCTS

The same preparations of T101-dextran described in (D) were radioiodinated with the chloramine-T method (Hunter, W.M., and Greenwood, F.C. Nature, 194 495-496 (1962). Pharmacokinetic studies were performed as described by Halpern, S.C., et al., Cancer Res. 43 5347-5355 (1983). Briefly, the modified antibodies were injected i.v. in normal mice, and the pharmacokinetics were evaluated at 4 hours post-injection. At this time, mice were sacrificed and weighed. Organs were removed, their weight recorded, and each organ was then washed in sterile PBS, pH 7.0, and fixed in formalin solution. Samples of blood, urine and feces also were collected and their weight similarly recorded. The organs, or a portion thereof, were transferred to clean tubes and their radioactive content was determined in a well-type gamma-counter.

Relative radioactive content of each organ was expressed as a percentage of injected dose per organ. As indicated in Figure 1a, both preparations of T101-dextran exhibited substantially the same biodistribution as that observed for the control. The abbreviations used in Figure 1a are as follows: BL = blood, KI = kidney; LV = liver; U = urine; F = feces. In additional studies, pharmacokinetic analysis was extended up to 48 hours post-injection; T101-dextran preparations exibited substantially unaltered biodistribution compared to that observed for the control.

These studies were repeated after storage for nine months at 4 °C. The pharmacokinetics of these preparations did not change appreciably, compared to the results shown in Figure 1a. These results indicate that dextran-modified T101 MoAb, at this level of modification, are stable upon storage in aqueous solution at 4 °C for at least nine months.

Figures 1b, 1c and 1d show similar post-administration studies at 4, 24 and 48 hours, respectively, using $^{111}$In labelled T101 (control), T101-modified with dextran (15 moles dextran/mole of immunoglobulin, "T101-dextran (15)") and T101-dextran (15) without subsequent reduction with $NaBH_4$. The indium label was placed on the antibody by the method of Reardon, et al., Nature, 316, 265 (1985), using a (S)-p-isothiocyanato derivative of diethylenetriaminopentaacetic acid ("DTPA") in place of the isothiocyanato-benzyl EDTA disclosed therein. The data are expressed as a percentage of injected dose per gram of tissue. The abbreviations used for the various tissues correspond to those defined below in Example II, paragraph A and are used throughout the remaining figures. These figures show that subsequent reduction with $NaBH_4$ is important for obtaining monomeric compositions of the invention and failure to use such reduced products may result in substantial alterations in the observed pharmacokinetics as compared either to the control unmodified immunoglobulin or the dextran-modified immunoglobulins of the invention.

F. EVALUATION OF IMMUNOGENICITY OF T101-DEXTRAN ADDUCTS

The immunogenicity of the T101-dextran adducts, prepared as described above, was measured by injecting these preparations in rabbits and measuring the rabbit anti-mouse response in serum samples by conventional ELISA assay. Three groups of seven rabbits each were injected with 100 $\mu$g of antibody preparation/injection. Samples of blood were taken weekly by ear-puncture and serum was collected by centrifugation. The ELISA assay (Engvall: Meth. Enzymol. 70 419-439 (1980)) was performed as follows: Microtiter well plates were coated with non-modified T101 monoclonal antibody, non-specific binding sites were blocked with bovine serum albumin, wells were exposed to serial dilution of individual experimental rabbit serum and washed thoroughly. Wells then were incubated with commercially available [Tago, Inc. Burlingame, California] goat anti-rabbit immunoglobulins conjugated to horseradish peroxidase, and color was developed with the addition of 1 mg/l ml ortho-phenyldiamine in 0.1% v/v hydrogen peroxide solution. Color development was quantified by measuring the absorbance at 490 nm with commercially available ELISA readers.

Results are shown in Figure 2. Panel A describes the anti-T101 reactivity in serum of rabbits immunized against T101 (control, (part I)), T101-dextran (10) (part II), and T101-dextran (17) (part III). Sera were tested

at 1:50 final dilution. After two immunizations (shown by arrows) 7/7 or 100% of the control rabbits responded against T101. In contrast, four out of seven (or 57%) of the rabbits injected with T101-dextran (10) and only one out of 7 (or 14%) rabbits injected with T101-dextran (17) elicited responses against T101. Thus, dextran modification of T101 MoAb resulted in the effective reduction of immunogenicity of these antibodies.

Panel B of Figure 2 describes the ability of the rabbits unresponsive to either of the T101-dextran preparations to respond to a subsequent challenge of modified T101. The purpose of this experiment is two-fold: First, to ascertain whether the lack of response against T101-dextran preparations was due to lack of immunological competence of those rabbits, and secondly, to evaluate whether the administration of T101-dextran induces tolerance against the native protein. As shown in panel B, all rabbits unresponsive to either preparation of T101-dextran elicited a strong immune response against unmodified T101, indicating that these rabbits were fully immunocompetent.

The production of host antibodies against an immunogen results in the rapid clearance of the antigen from the circulation. Thus, to confirm further the results shown in Figure 2, the rate of clearance of T101 from the plasma of rabbits immunized with unmodified T101 or with T101-dextran was determined. One hundred $\mu$g of unmodified T101 were injected i.v. in rabbits which had been immunized previously with T101-dextran (10) and T101-dextran (17), but which had failed to mount a response against these antigens (--△▲--, --◊♦--). Sera were collected at the times indicated in Figure 3 and tested for the presence of circulating T101 with the ELISA assay described previously. Controls included rabbits positive against unmodified T101 (--O-●--), and virgin, non-immunized rabbits

(-⬢-⬡-).

Rabbits immunized against unmodified T101 rapidly cleared T101 from the circulation since no detectable T101 could be found in these sera as early as 6 hours post-injection. On the other hand, rabbits unresponsive to the immunization of T101-dextran (10) or T101-dextran (17), as well as control, non-immunized rabbits did not remove T101 from the circulation. Moreover, in both the experimental as well as the control groups, the elimination kinetics of T101 from the blood was not affected, being primarily first order, with an approximate half-life of 68 hours. This evidence shows the lack of an immune response against T101 in rabbits immunized with T101-dextran, and further confirms the reduction of immunogenicity of the dextran modified antibody.

G. LACK OF ANTI-DEXTRAN RESPONSE IN RABBITS IMMUNIZED WITH T101-DEXTRANS

The data described in paragraph (F) indicates that dextran-modification of T101 can reduce substantially the immunogenicity of the free antibody. Dextrans of high molecular weight (>95,000), however, are known to be immunogenic themselves, especially when bound to protein carriers. (Kabat, E.A., and Berg, D., J. Immunology, vol. 70, 514, 1953. and Richter, W. and Kagedal, L., Int. Arch. Allergy, vol. 42, pp 885-902, 1972). To evaluate whether the administration of T101-dextran in rabbits could elicit an anti-dextran response, sera of rabbits injected with T101-dextran (10) or T101-dextran (17) were tested against either T101-dextran or BSA-dextran in the ELISA assay described earlier. The results are shown in Table II. Sera from rabbits that did not respond against immunization with T101-dextran (10) or T101-dextran (17) were also negative when tested against BSA-dextran. In addition, sera positive against immunization with unmodified T101, T101-dextran (10) or T101-dextran (17) were similarly negative when tested against BSA-dextran. These data indicated a lack of immunogenicity of protein-bound dextran derivative and confirm that the T101-dextran adducts, themselves, lack any significant immunogenic characteristics.

TABLE II

| EVALUATION OF ANTI-OXIDIZED-DEXTRAN RESPONSE IN RABBITS INJECTED WITH T101-DEXTRAN | | | |
|---|---|---|---|
| POSITIVE CONTROL | SERA TESTED AGAINST (OD. 490 nm) | | |
| | T101 | T101-DEXTRAN(10) | BSA-DEXTRAN(10) |
| R anti-Mouse Ab | 1.91 | 0.340 | 0.009 |
| R anti-BSA Ab | 0.065 | 0.004 | 2.15 |
| SERA NEGATIVE AGAINST | | | |
| T101-DEXTRAN (10) | 0.124 | 0.062 | 0.078 |
| T101-DEXTRAN (17) | 0.004 | 0.009 | 0.100 |
| SERA POSITIVE AGAINST | | | |
| T101 | >2.00 | 1.58 | 0.105 |
| T101-DEXTRAN (10) | 2.15 | 1.08 | 0.062 |
| T101-DEXTRAN (17) | >2.00 | 0.61 | 0.09 |

EXAMPLE II

PREPARATION OF WATER-SOLUBLE CONJUGATES OF POLYCLONAL RABBIT ANTI-HUMAN FERRITIN-IgG WITH DEXTRAN, MW6000

Dextran, having a molecular weight of about 6000, was activated as described in Preparation 1. Polyclonal rabbit anti-human ferritin IgG ("RAF") was purified from rabbit immune serum according to the method of Fahey and Terry [Handbook of Experimental Immunology, pp. 8.1-8.15, D.M. Weir (Ed.,)].

The purified RAF antibody then was modified with activated dextran according to the method described in Example 1.

A. PHARMACOKINETICS OF RAF-DEXTRAN ADDUCTS

The in vivo organ biodistribution of RAF (control), and two preparations of RAF-dextran adducts, containing about 8 dextran molecules per antibody molecule and 16 dextran molecules per antibody molecule (as determined by the method described in Example I) are shown in Figures 4a, 4b, and 4c, respectively. The antibodies were radiolabelled with indium and the study was performed as described in Example I. In these figures, BL = blood; FEM = Femur (which is designated as BO in Figure 4b and 4c); BO = bone; HT = heart; KI = kidney; LV = liver; LG = lung; MU = muscle; SK = skin; SP = spleen; I = intestine; U = urine; F = feces; GI = gastro-intestinal tract (which is designated as I in Figure 4b). Further, in Figures 4b and 4c, biodistributions were performed at 4, 24, 48 and 72 hour intervals post-injection while in determining the data for Figure 4a, samples were taken at 4 hours and 48 hours post-injection. As indicated in the figures, both preparations of RAF-dextran possessed pharmacokinetics that are not significantly different from that of the control, unmodified antibodies. In addition, the dextran modification resulted in a desirable substantial increase in plasma levels of the antibodies at the earlier time-points. These studies were repeated after storage at 4°C for three months and produced identical results.

B. EVALUATION OF IMMUNOGENICITY OF RAF-DEXTRAN ADDUCTS

The immunogenicity of RAF-dextran adducts was measured by injecting these preparations in mice and measuring the extent of the mouse-anti-rabbit response in serum samples by the ELISA assay as described earlier. An optimal immunogenic dose of 10 μg of RAF per mouse was identified in a previous dose-escalation experiment, in which increasing amounts of RAF antibodies (1 to 500 μg per mouse) were injected and the extent of the mouse-anti-rabbit responses were evaluated by the ELISA assay.

Three groups of 5 mice each were injected with 10 μg of unmodified RAF (-O-), or RAF modified with about 8 dextrans/Ab ("RAF-dextran (8)")(-△-) or with about 17 dextrans/Ab ("RAF-dextran (17)")(-●-). The symbols used correspond to those used in Figures 5a and 5b which show the results of these studies.

Blood samples were collected weekly and serum separated by centrifugation. Sera were tested at 1:500 final dilution. Upon a single administration of unmodified RAF, mice elicited a strong immune response. When RAF was modified with either about 8 or 17 dextran molecules per molecule of antibody and similarly injected, they failed to elicit significant immune responses above background levels. To further confirm these results, relative titres of these sera were determined as described by Engvall, Meth. Enzymol 70 419-439 (1980). Briefly, sera were serially diluted and assayed in the ELISA assay as described earlier. Relative titres were expressed as the reciprocal of the dilution capable of generating 50% of the maximum response. With this assay, sera of mice immunized with unmodified RAF exhibited titres in excess of 1/1,200. However, sera of mice immunized with RAF-Dextran (8) or RAF-Dextran (17) did not exhibit appreciable titres (less than 1/50). The results of these studies, over a 36 week period of time, are shown in Figure 5b. In Figure 5b, the solid arrows denote the i.v. injection of each of the noted compositions whereas the open arrows indicate later challenges with unmodified RAF. Therefore, the modification of RAF with dextran molecules resulted in the effective reduction of the immunogenic content of these antibodies. In addition, mice unresponsive to either preparation of RAF-dextran elicited an immune response against challenges with unmodified RAF, indicating that these mice were fully immunocompetent.

C. LACK OF ANTI-DEXTRAN IMMUNE RESPONSE IN MICE IMMUNIZED WITH RAF-DEXTRAN ADDUCTS

The data described in paragraph B indicates that modification of RAF antibodies with oxidized dextran can reduce substantially the immunogenicity of these antibodies. To evaluate whether the administration of RAF-dextran adducts in mice could elicit an anti-dextran immune response, sera of mice immunized with RAF-dextran (8) and RAF-dextran (17) were tested for their ability to respond against either RAF-dextran (17) or BSA-dextran (10) in the ELISA assay described earlier. As shown in Table III, sera of mice that did not respond against immunizations with RAF-dextran (8) or RAF-dextran (17), as described in paragraph B, were similarly negative when tested against either RAF-dextran or BSA-dextran. These results indicate that binding of the oxidized dextran (mw of about 6000) to the polyclonal RAF fails to induce an immune response against the modified dextran, further confirming the evidence provided earlier.

TABLE III

| EVALUATION OF ANTI-DEXTRAN RESPONSE IN MICE INJECTED WITH RAF-DEXTRAN | | |
|---|---|---|
| POSITIVE CONTROLS | SERA TESTED AGAINST THESE ANTIGENS: (OD 490 nm) | |
| | RAF-DEXTRAN | BSA-DEXTRAN |
| Mouse anti-Rabbit | 2.1 | N.T.* |
| Rabbit anti-BSA | N.T.* | 1.66 |
| MOUSE SERA IMMUNIZED WITH: | | |
| RAF | 0.123 | 0.000 |
| RAF-Dextran (8) | 0.265 | 0.131 |
| RAF-Dextran (17) | 0.215 | 0.040 |

* N.T. = Not Tested

EXAMPLE III

PREPARATION OF POLYCLONAL IgG-DEXTRAN (M.W. 16,000) ADDUCTS

Dextran having a molecular weight of about 16,000 ±1,000 was activated as decribed in Preparation I. Polyclonal immunoglobulin G from pooled, normal human serum was purified according to the standard method of Fahey and Terry [Handbook of Experimental Immunology, pp. 8.1-8.15, D. M. Weir (Ed.)]. Briefly, the immunoglobulins were purified from whole serum by precipitation with either 33% ammonium sulfate or 18% sodium sulfate, followed by anion-exchange chromatography with DEAE columns.

Purified polyclonal IgG was conjugated with dextran according to the procedure described in Example I. The immunogenicity was measured by injecting polyclonal IgG-dextran preparations in mice, and serum

samples were collected weekly. An optimal immunogenic dose of 10 μg of human IgG per mouse was identified in a 40 dose-escalation experiment as described in Example II. Unmodified, polyclonal human IgG control preparations were injected in a separate group of mice and the mouse anti-human response was similarly measured. The results of this study are shown in Figure 6. Upon a single injection of 10 μg polyclonal human IgG control (●) mice developed a substantial immune response. The response was shown to peak at week 4-5 post-administration, and subsequently returned to baseline levels. Human polyclonal IgG, modified with dextran and similarly injected (—, in Figure 6), failed to elicit an immune response at any time during the experiment (up to week 10). The conjugation of polyclonal human IgG with dextran having a molecular weight of about 16,000, therefore, significantly reduces the immunogenic potential of these antibodies.

## EXAMPLE IV

### PREPARATION OF WATER-SOLUBLE CONJUGATES OF KS1/4 007B MoAb WITH DEXTRAN, MW6000

Dextran of MW6000 was activated as described in Preparation I. Purified MoAb KS1/4 007B was modified with activated dextran as described in Example I to produce two preparations of KS1/4 007B-dextran, one containing about 10 dextran molecules per antibody molecule and another containing about 15 dextran molecules per molecule of antibody.

### A. DETERMINATION OF IMMUNOREACTIVITY OF KS1/4 MoAb DEXTRAN, MW6000

The immunoreactivity assay was performed essentially as described previously in Example 1D, with the exception that cell line LS174-T [B.H. Tom, *et al.*, *In Vitro* 12:180-191, 1976. American Type Culture Collection, Rockville, Maryland. Deposit No. ATCC CL188] was used. This cell line possesses the antigen(s) recognized by KS1/4 007B. The results of this study are shown in Table IV.

TABLE IV

| IMMUNOREACTIVITY OF KS1/4-007B-DEXTRAN | |
|---|---|
| | % OF CONTROL |
| KS1/4-007B (CONTROL) | 100 |
| KS1/4-007B-DEXTRAN (10) | 91 |
| KS1/4-007B-DEXTRAN (15) | 87 |

### B. PHARMACOKINETICS OF KS1/4 007B-DEXTRAN ADDUCTS

The *in vivo* biodistribution of radiolabelled KS1/4-007B adducts is shown in Figure 7. Unmodified KS1/4-007B or KS1/4-007B modified with about 10 dextran molecules per antibody were radiolabelled and injected intravenously in normal Balb/c mice. The symbols used have the same meanings as already defined (See Example 2A). These studies were performed as previously described in Example I. KS1/4 007B-dextran exhibited pharmacokinetics substantially unaltered from that of the control, unmodified antibodies. In addition, the dextran modification results in a potentially desirable increase in plasma levels of the antibodies shortly after injection.

### C. EVALUATION OF IN VIVO TUMOR-TARGETING ABILITY OF KS1/4 007B-DEXTRAN ADDUCTS

*In vivo* tumor localization studies were performed in nude mice carrying subcutaneous implants of the human colon carcinoma cell line T-380 which was found to possess the antigen(s) recognized by KS1/4 007B. Briefly, Balb/C Nu/Nu mice were injected subcutaneously with a minced preparation of T-380 tumor implants and maintained for approximately two weeks, or until the tumor implant had grown to a size of approximately 0.5 grams as described by P.L. Hagan *et al.*, *J. Nuc. Med.*, 26 1418-1423 (1985). Mice were kept in a sterile environment to prevent complications due to bacterial or viral infections. Unmodified KS1/4 007B or KS1/4 007B modified with about 10 dextran molecules per molecule of antibody were radiolabelled and injected intravenously in tumor-bearing nude mice. Tumor localization and organ biodistribution studies

were performed as described in Example I. Data are expressed as a percentage of injected dose per gram of tissue. As shown in Figure 8, KS1/4 007B-dextran (10) retained substantial tumor localization activity compared to control unmodified KS1/4. In this figure, TU = "tumor". The remaining symbols have the same meanings as defined in Example 2A. This study indicates that modification of the antibody with dextran does not adversely affect the ability of KS1/4 007B to localize in the tumor xenograft in vivo.

## EXAMPLE V

## PREPARATION OF WATER-SOLUBLE CONJUGATES OF ANTI-CEA ZCE025 Fab′ MoAb WITH DEXTRAN MW6000

Dextran MW6000 was activated as described in Preparation I. Purified murine ZCE025 [Acolla et al., Proc. Natl. Acad. Sci. (USA), 77:563, 1980] was treated with Pepsin, 1% final solution in acetate buffer, pH 4.0 for 24 hours to generate $F(ab')_2$ fragments [Stanworth, D.R., and Turner, M.W., pp. 10.11-10.24, Handbook of Experimental Immunology, Weir, D.M. (Ed.,)]. The resulting $F(ab')_2$ fragments were purified from residual intact antibodies and low molecular weight products by gel filtration chromatography, such as Pharmacia Sephadex G200. Purified $F(ab')_2$ fragments then were reduced to Fab′ by treatment with cysteine, and subsequently treated with iodoacetic acid to block free disulfide groups and prevent reassociation to $F(ab')_2$. Fab′ fragments then were dialyzed to remove reagents. These fragments then were modified with activated dextran as described in Example I. This generated monomeric adducts possessing, respectively, 8 dextran molecules/Fab′ ("ZCE025-Fab′-Dextran(8)") and 5 dextran molecules/Fab′ ("ZCE025-FAb′-Dextran(5)").

### A. DETERMINATION OF IMMUNOREACTIVITY OF ZCE025 Fab′ DEXTRAN ADDUCTS

1) PREPARATION OF ANTIGEN SOURCE

Briefly, plastic beads were covalently coated with a murine monoclonal antibody raised against CEA. Beads were then coated with CEA by incubation with CEA-containing serum to give a final concentration of approximately 100 µg CEA per bead. Another set of beads were similarly treated with serum that did not contain CEA to provide the control. After overnight incubation at 4°C, beads were washed with PBS, pH 7.0, containing 0.1% Tween 20, and stored at 4°C in PBS with 0.02% $NaN_3$ to prevent bacterial contamination until ready for use.

2) DESCRIPTION OF THE ASSAY

CEA-coated beads as well as negative beads prepared as above were incubated with 1 ng of $^{125}I$-radiolabelled antibody having a specific activity of 10 µCi/µg. After incubation at 37°C overnight, beads were processed as described in Example 1D. The total percentage immunoreactivity was calculated as described in Example 1D. Results are shown in Table V. After modification with oxidized dextran, ZCE025 Fab′ was shown to retain approximately 50% of the original immunoreactivity as compared to the unmodified ZCE025 Fab′ control.

TABLE V

| IMMUNOREACTIVITY OF ZCEO25-Fab′-DEXTRAN | |
|---|---|
| | % I.R. |
| Fab′ (CONTROL) | 51 |
| Fab′-DEXTRAN (5) | 26 |
| Fab′-DEXTRAN (8) | 24 |

### B. EVALUATION OF IN VIVO TUMOR-TARGETING ABILITY OF ZCE025 Fab′-DEXTRAN ADDUCTS

In vivo tumor localization studies were performed in nude mice carrying subcutaneous implants of the human colon carcinoma cell line T-380 as described in Example IV. Unmodified Fab′ fragments as well as ZCE025 Fab′-dextran (8) and ZCE025 Fab′-dextran (5) were radiolabelled and injected intravenously in tumor-bearing nude mice. Plasma elimination kinetics were evaluated at 4, 14, 24, and 48 hours post-administration. Tumor localization and organ biodistribution were performed as described in Example I, at 4,

and 48 hours post-injection. As shown in Figure 9a, ZCE025 Fab′ modified with either 5 or 8 molecules of activated dextran possessed significantly elevated plasma levels as consequence of increased molecular weight. In particular, the plasma half-life was shown to increase substantially, from about 7 hours for the unmodified fragment to about 16-19 hours for either Fab′-dextran preparation. Tumor-localization and organ biodistribution studies were performed with ZCE025 Fab′-dextran (5). As shown in Figure 9b, dextran-modification of Fab′ fragment resulted in a marked decrease (up to 10 fold) in kidney uptake, and consequently, a decrease in urine secretion. As a consequence of decreased kidney excretion, plasma levels were substantially increased, as already evidenced in Figure 9a. Tumor localization values were also comparable to the unmodified Fab′. Dextran modification of the ZCE025 Fab′ fragments not only markedly improved the observed pharmacokinetics of the unmodified form of the antibody fragments by reducing the elimination rate by the kidneys, but also did not adversely affect its ability to localize at the tumor site in vivo.

EXAMPLE VI

PREPARATION OF WATER-SOLUBLE CONJUGATES OF ANTI-CEA ZCE025 $F(ab')_2$ MoAb WITH DEXTRAN, MW6000

$F(ab')_2$ fragments of the murine MoAb ZCE025 were produced and purified as described in Example V. Dextran, MW6000, was activated as described in Preparation 1. The $F(ab')_2$ fragments were modified with oxidized dextran as described in Example I. This generated monomeric adducts possessing, respectively, 1 dextran molecule per $F(ab')_2$ ("ZCE025 $F(ab')_2$-Dextran (1)") and 4 dextran molecules per $F(ab')_2$ ("ZCE025 $F(ab')_2$-Dextran (4)").

A. DETERMINATION OF IMMUNOREACTIVITY OF ZCE025 $F(ab')_2$-DEXTRAN ADDUCTS

Immunoreactivity studies were performed as described in Example V, paragraph A. The results of this study are shown in Table VI. These results show that after modification with oxidized dextran, ZCE025 F-(ab′)$_2$ fragments retain substantial immunoreactivity as compared to the unmodified $F(ab')_2$ control.

TABLE VI

| IMMUNOREACTIVITY OF ZCE025 $F(ab')_2$-DEXTRAN ADDUCTS | | |
|---|---|---|
| | % I.R. | % OF CONTROL |
| $F(ab')_2$ (Control) | 87 | 100 |
| $F(ab')_2$-Dextran (1) | 78 | 89 |
| $F(ab')_2$-Dextran (4) | 65 | 75 |

B. PHARMACOKINETICS OF ZCE025 $F(ab')_2$-DEXTRAN ADDUCTS

Unmodified ZCE025 $F(ab')_2$ fragments, as well as ZCE025 $F(ab')_2$-Dextran(1) and ZCE025 $F(ab')_2$-Dextran (4) were radiolabelled and injected intravenously in Balb/c mice. Plasma elimination kinetics and organ biodistribution were performed at 1, 4, 14, 24 and 48 hours post-administration. Figure 10a shows kidney tissue uptake. This figure clearly shows that ZCE025 $F(ab')_2$ fragments, modified with either 1 or 4 moles of dextran, exhibited a marked decrease in uptake by the kidney, thus substantially improving the pharmacokinetics of these fragments. The uptake of ZCE025 $F(ab')_2$-Dextran(4) was essentially reduced to background level, or the amount of radiolabelled product in the blood that circulates into the vascular compartment of the organ at time of sacrifice. Figure 10b shows the modified plasma levels of the $F(ab')_2$ fragments compared to the unmodified fragments. This figure indicates that dextran-modified fragments possessed elevated plasma half-lives, perhaps as a consequence of the increased molecular weight. In particular, the plasma half-lives were, respectively, 21 hours for the unmodified $F(ab')_2$ fragment, and 23-24 hours for the dextran-modified fragments. The distribution of these adducts in the remaining organs was not substantially altered as compared to the unmodified fragments.

C. EVALUATION OF IN VIVO TUMOR-TARGETING ABILITY OF ZCE025 F(ab')$_2$-DEXTRAN ADDUCTS

Unmodified ZCE025 F(ab')$_2$, as well as ZCE025 F(ab')$_2$-Dextran(1) and ZCE025 F(ab')-Dextran(4) were radiolabelled and injected intravenously in nude mice carrying subcutaneous implants of the human colon carcinoma cell line T-380 (see Example IV). Tumor-localization studies were performed at 1, 4, 14, 24 and 48 hours post-administration as described in Example IV, paragraph C. As shown in Figure 11, ZCE025 F(ab')$_2$-Dextran(1) and ZCE025 F(ab')$_2$-Dextran(4) substantially retained the ability to localize to the tumor xenografts.

The results shown in Figures 10a, 10b and 11 indicate that dextran modification of ZCE025 F(ab')$_2$ fragments markedly improved the pharmacokinetics of the unmodified fragments by increasing the half-life in plasma and reducing the rate of elimination by the kidney without adversely affecting its ability to localize to tumor xenografts in vivo.

EXAMPLE VII

PREPARATION OF WATER-SOLUBLE CONJUGATES OF CHA-CEM BIFUNCTIONAL ANTIBODIES WITH DEXTRAN, MW6000

Murine CHA-CEM bifunctional antibody ("BFA") is an antibody possessing two specificities, one directed toward carcinoembryonic antigen, the other to a hapten, a derivative of benzyl EDTA. This chemically linked bifunctional antibody can be synthesized by chemical reaction of an Fab' fragment of the murine MoAb CHA 255, directed against a derivative of benzyl EDTA, and a second Fab' fragment of the murine MoAb CEM231, directed against CEA. Briefly, each MoAb was digested with pepsin to generate F(ab')$_2$ fragments and then subsequently reduced to their corresponding Fab' fragment, as described in Example V. CHA 255 Fab' fragments were then reacted with the homobifunctional cross-linking agent bis-(maleimido)-methylether ("BMME"), excess reagent was removed, and subsequently reacted with CEM 231 Fab' fragments to generate CHA-CEM bifunctional antibodies. An alternate cross-linking procedure, using dithionitrobenzoic acid, as well as the present BFA procedure, are described in European Patent Application 86307031.4, published as EP A 0 217 577 on April 8, 1987, the teaching of which is incorporated herein by reference.

Dextran, MW6000, was activated as described in Preparation 1 and the CHA-CEM BFA was modified with oxidized dextran as described in Example I. This generated monomeric adducts possessing, respectively, 2 or 8 moles of dextran per mole of BFA ("BFA-Dextran(2)" and "BFA-Dextran(8)"), respectively.

A. DETERMINATION OF IMMUNOREACTIVITY ON CHA-CEM BFA-DEXTRAN ADDUCTS

Because the CHA-CEM bifunctional antibody possesses two different antigenic specificities, the immunoreactivity of each arm of the BFA molecule was determined separately.

1) DETERMINATION OF IMMUNOREACTIVITY OF THE ANTI-CEA ARM

Immunoreactivity of the anti-CEA arm of BFA was determined as described in Example V, paragraph A.

2) DETERMINATION OF IMMUNOREACTIVITY OF THE ANTI-HAPTEN ARM

The chelating agent (S)-4-[3-(hydroxyethyl)] thioureidobenzyl EDTA ("EOTUBE") (J. Nuc. Med., 29 835 (1988)) was trace-labelled with $^{111}$In. One-hundred fifty pmoles of $^{111}$In-EOTUBE were mixed with 100 pmoles of CHA-CEM or CHA-CEM-Dextran and incubated 30 minutes at room temperature. The BFA-$^{111}$In-EOTUBE complex was then separated from the free $^{111}$In-EOTUBE by centrifugation over a 30,000 MW cut-off membrane, such as the commercially available Centrifree (Amicon Co., Lexington, MA), and the filtrate (A) was collected. A sample containing $^{111}$In-EOTUBE in the absence of BFA was set-up, similarly treated, and the filtrate (B) collected. Total percentage of reactivity was expressed according to the following formula:

$$\% \text{ reactivity} = \left(1 - \frac{B-A}{A}\right) \times 1.5 \times 100$$

The immunoreactivity of CHA-CEM BFA and CHA-CEM-Dextran adducts is shown in Table VII. After modification, CHA-CEM Dextran adducts were shown to retain substantial immunoreactivity as compared to unmodified CHA-CEM.

TABLE VII

| IMMUNOREACTIVITY OF CHA-CEM-DEXTRAN ADDUCTS | | |
|---|---|---|
| | Anti-Hapten Arm % I.R. | Anti-CEA Arm % I.R. |
| CHA-CEM (CONTROL) | 102 | 68 |
| CHA-CEM DEXTRAN (2) | 98 | 52 |

B. PHARMACOKINETICS OF CHA-CEM BFA-DEXTRAN ADDUCTS

Unmodified CHA-CEM BFA, or antibody modified with either 2 or 8 moles of dextran per mole of BFA were radiolabelled with $^{111}In$. Briefly, $^{111}In$-labelled EOTUBE was mixed with BFA or BFA-Dextran as described above. The $^{111}In$-EOTUBE-BFA or the $^{111}In$-EOTUBE-BFA-Dextran complexes were then injected intravenously into Swiss CD1 mice. This labelling method exploits the affinity of the anti-hapten arm toward $^{111}In$ bound to EOTUBE and obviates the need to chemically link the $^{111}In$-chelating agent to the antibody. Plasma determination and organ biodistribution were determined at 4 and 24 or at 4 and 48 hours post-administration, respectively. As shown in Figures 12a and 12b, the modification of BFA with dextran resulted in a significantly reduced uptake of this antibody into the kidney, without substantially altering the biodistribution in the other organs tested. Thus, as noted previously with dextran modified ZCE025 $F(ab')_2$ or Fab′ fragments, the modification of CHA-CEM BFA with dextran resulted in substantially improved pharmacokinetics.

C. EVALUATION OF IN VIVO TUMOR-TARGETING ABILITY OF CHA-CEM BFA-DEXTRAN ADDUCTS

Tumor localization experiments were performed in nude mice carrying subcutaneous implants of the human colon carcinoma cell line T-380 (see Example IV). CHA-CEM BFA, or antibody modified with 2 moles of dextran per mole of BFA were injected intravenously in nude mice and allowed to localize to T-380 implants in vivo [S. E. Halpern, et al., Cancer Res., 43 5347-5355 (1983)]. Twenty-four hours post-administration, $^{111}In$ labelled EOTUBE was administered intravenously. Tumor-localization was determined 48 hours post-administration of $^{111}In$-EOTUBE, under conditions which had been optimized in previous experiments. Results are shown in Figure 13. CHA-CEM BFA modified with dextran possessed tumor-localization properties substantially unchanged relative to that of unmodified BFA while eliminating significant uptake by the kidney.

EXAMPLE VIII

PREPARATION OF WATER-SOLUBLE CONJUGATES OF ZCE025 MoAb WITH DEXTRAN, MW6000

Dextran of MW6000 was activated as described in Preparation I. Purified murine MoAb ZCE025 (Example V) was modified with oxidized dextran as described in Example I to produce preparations containing about 4, 7, 9, 13 and 15.5 moles of dextran per mole of antibody.

A. DETERMINATION OF IMMUNOREACTIVITY OF ZCE025 MoAb DEXTRAN ADDUCTS

The immunoreactivity assay was performed as described in Example V, paragraph A. The results of this study are shown in Table VIII. After modification with oxidized dextran, antibodies were shown to retain substantial immunoreactivity as compared to unmodified ZCE025.

TABLE VIII

| IMMUNOREACTIVITY OF ZCE025-DEXTRAN | | |
|---|---|---|
| | % I.R. | % of Control |
| ZCE025 (CONTROL) | 80.2 | 100 |
| ZCE025-DEXTRAN (4) | 82.1 | 102 |
| ZCE025-DEXTRAN (9) | 71.4 | 89 |
| ZCE025-DEXTRAN (15.5) | 58.0 | 72 |
| In a separate experiment, the immunoreactivity of ZCE025 modified with 7 and 13 moles of dextran per mole of antibody was determined. The results are as follows: | | |
| | % I.R. | % of Control |
| ZCE025 (CONTROL) | 82 | 100 |
| ZCE025-DEXTRAN (7) | 75.2 | 91.7 |
| ZCE025-DEXTRAN (13) | 60.3 | 73.5 |

B. PHARMACOKINETICS OF ZCE025-DEXTRAN ADDUCTS

Unmodified ZCE025 MoAb, as well as ZCE025 modified with 7, 9, 13, and 15.5 moles of dextran per mole of antibody were radiolabelled and injected intravenously into Balb/c mice. Biodistribution was performed at 4 and 48 hours post-administration and the results are shown in Figures 14a and 14b, respectively. As shown in these figures, dextran-modified preparations of ZCE025 exhibited pharmacokinetics substantially unaltered from that of the control, unmodified antibodies. In addition, the dextran modification results in a potentially desirable increase in plasma levels of the antibodies.

C. EVALUATION OF IN VIVO TUMOR-TARGETING ABILITY OF ZCE025-DEXTRAN ADDUCTS

Unmodified ZCE025, as well as antibody modified with 7, 9, 13 and 15.5 moles of dextran per mole of antibody were radiolabelled and injected intravenously into nude mice carrying a subcutaneous implant of the human colon carcinoma cell line T-380 (see Example IV). Tumor localization studies were evaluated 48 hours post-administration as described in Example IV, paragraph C. As shown in Figure 15, dextran-modified ZCE025 retained the ability to localize specifically to the tumor xenografts. However, a decrease in the tumor uptake of the antibody, related to the level of dextran-modification, was observed.

EXAMPLE IX

PREPARATION OF WATER SOLUBLE CONJUGATES OF CHIMERIC MONOCLONAL ANTIBODIES WITH DEXTRAN, MW6000

Preparation of the Chimeric Antibody

A human-mouse chimeric MoAb XCEM449 was prepared as described in U.S. Serial No. 165,856 (March 9, 1988) (Attorney Docket 1000-77 and H-7587), incorporated herein by reference. Murine hybridoma cells, designated as CEM 231.6.7, were used to derive and clone genomic DNA for variable light and heavy chain variable regions. Murine hybridoma CEM 231.6.7 was deposited with the American Type Culture Collection, Rockville, Maryland, under the acession number ATCC HB 9620. The cloned genomic DNA from murine hybridoma CEM 231.6.7, and human peripheral blood lymphocytes, were used for the construction of the chimeric genes.

Transfection of chimeric genes was accomplished by electroporation techniques, essentially as described by Toneguzzo, F., et al., Moleclar and Cell Biol., 6:703-706 (1986); and Chu, G. et al., Nucleic Acid Res., 15:1311-1325 (1987). The host cells, SP2/0-Ag14 hybridoma cells, were the recipients of the chimeric genes. The SP2/0-Ag14 hybridoma cells are available from the American Type Culture Collection, Rockville, Maryland under the accession number ATCC CRL 1581.

A. ISOLATION OF DNA FROM CEM 231.6.7 MURINE HYBRIDOMA

Genomic DNA from CEM 231.6.7 was isolated by procedures essentially as described by Pellecer et al., Cell, 41:133 (1978). Approximately 1 x $10^8$ cells were collected by centrifugation (10 minutes, 800 rpm, IEC clinical centrifuge). Cells were then washed twice in Phosphate Buffered Saline (PBS). Cells were then resuspended in 4 ml of 10 mM Tris-HCl (PH 8.0) 2 mM EDTA, 40 mM NaCl (TEN), and lysed by the addition of 200 µl of 10% SDS and 42 µl of Protease K at 20 mg/ml (Sigma Chemicals, St. Louis, Missouri). The sample was incubated overnight at 37°C. DNA was extracted twice with an equal volume of phenol:choloroform:isoamyl alcohol (25:24:1 ratio) and twice more with an equal volume of chloroform:isoamyl alcohol (24:1 ratio) and dialyzed against 10 mM Tris-HC1 (pH 8.0, 1 mM EDTA) (TE Buffer) overnight. The DNA was then treated for 2 hours with RNAse A at 50 µg/ml (Sigma Chemicals, St. Louis, Missouri), followed by Protease K at 200 µg/ml for one hour at 37°C. DNA was extracted again as detailed above and dialyzed overnight. Following dialysis, DNA was ethanol precipitated by adding 1/10th volume of 2 M Na acetate and 2 volumes of 95% ethanol. After 30 minutes at -20°C, the DNA was centrifuged at 8000 rpm for 30 minutes. The pellet was resuspended in TE buffer at a final concentration of 955 µg/ml.

B. CONSTRUCTION OF A GENOMIC LIBRARY FOR CEM 231.6.7

Partial DNA digests of the CEM 231 genomic DNA were prepared by taking 10 µg of DNA in 11 µl TE buffer, 20 µl of $H_2O$, 15 µl of 10X Core Restriction Digest Buffer (500 mM, pH 8.0, 100 mM $MgCl_2$, 500 mM NaCl) then aliquoted into tubes. Restriction enzyme MboI was added to each tube in units increasing from .0038 to .5 units/µl and incubated at 37°C for one hour. Aliquots of the samples then were run on a 0.7% TBE (89 mM Tris 89 mM Borate 2 mM EDTA) agarose gel and electrophoresed overnight at 40 volts. From the photograph of this gel it was determined which digestion fractions contained DNA in the correct molecular weight range (12-24 kb). Genomic DNA (200 µg) then was digested using the MboI units defined by the experiment described above (scaled up 20X) with an hour incubation at 37°C. This DNA was used to construct the EMBL-3 phage library using EMBL-3 phage DNA, commercially available from Stratagene, Inc. (La Jolla, California), as described in the manufacturer's protocol. This protocol follows the techniques described in several laboratory manuals (e.g., Basic Methods in Molecular Biology, edited by L. G. Davis, M.D. Dibner and J.F. Battey, Elsevier, New York (1986)). Following isolation on sucrose gradients, the large molecular weight CEM 231.6.7 DNA (12-24 kb) was ligated into the EMBL-3 phage arms using 100 ng of DNA, 100 ng of pre-isolated EMBL-phage arms, .5 µl of 10X Ligase Buffer (500 mM Tris-HCl, pH 8; 70 mM $MgCl_2$; 10 mM dithiothreitol (DTT) and T4 DNA ligase (2 units) at 4°C, overnight. Packaging was performed using Stratagene's commercially available Gigapack-Gold in vitro packaging system in accordance with the manufacturer's product protocol and using the host E. coli strain P2.392 supplied by Stratagene, Inc. Gigapack Gold packaging mix (500 µl) and 5 µl of the ligated phage were incubated at 22°C for 2 hours. Phage dilution buffer (0.5 ml) (per liter, 5.8 g NaCl; 2 g $MgSO_4$-$6H_2O$; 50 ml 1M Tris-HCl, pH 7.5; 5 ml 2% gelatin) was added. Phage then were titered using standard protocols as described in Molecular Cloning, edited by T. Maniatis, E.F. Fritsch and J. Sambrook, Cold Spring Harbor Laboratories, Cold Spring Harbor, New York (1982). Following titration, the phage library was plated at a density of 20,000 plaques/100 mM using P2.392 cells and incubated at 37°C overnight.

C. IDENTIFICATION AND ISOLATION OF RECOMBINANT PHAGE ϕMLCE-10 INCLUDING MURINE CEM 231.6.7 V KAPPA GENE

For the CEM 231.6.7 light (kappa) chain, 4.8 x $10^5$ recombinant phage in E. coli P2.392, commercially available from Stratagene, Inc. (La Jolla, California) were screened according to the protocol in Molecular Cloning, supra. This was done using murine kappa and murine $J_L$ probes derived from plasmids obtained from Dr. Marc Shulman, University of Toronto, Toronto, Canada or synthetic probes whose sequence was derived from the General Bank Data Base (NIH Accession #J00545). The kappa oligonucleotide probe used comprised the sequence:
5′-AGA-TGG-ATA-CAG-TTG-GTG-CAG-CAT-CAG-CCC-3′ and was synthesized by Molecular Biosystems, Inc. (San Diego, California).

Duplicate filter lifts were made and phage hybridizing to both probes were analyzed by double southern blots as described in Molecular Cloning, supra, probed with the murine kappa and $J_L$ probes which were radiolabelled with $^{32}$p. Hybridizatins were performed as described in Molecular Cloning, supra. A single CEM 231.6.7 phage clone was selected on the basis of its hybridizing to both probes, indicating a light chain variable region gene rearrangement to a position directly contigous to the C kappa gene region. This

recombinant phage was designated ϕMLCE-10.

D. CONSTRUCTION OF RECOMBINANT PLASMID ϕMLCE-10 INCLUDING CEM 231.6.7 MURINE V KAPPA GENE

ϕMLCE-10 DNA was isolated and 20 μg were digested with BamHI (1 unit/μg) using React Buffer #3 from Gibco-BRL (Gaithersburg, Maryland) in a total of 220 μl. A ~10 kb BamHI fragment was isolated by electrophoresis of the BamHI digested DNA in a .75% TBE agarose gel containing .5 μg/ml of ethidium bromide at 40 V overnight. Following visualization on a UV transparent light box the ~10 kb fragment was electrophoresed onto DEAE 81 (Schleicher and Schuell, Keene, New Hampshire) paper followed by elution in 1M NaCl and ethanol precipitation. The eluted fragment was then resuspended in 6 μl of TE buffer. The fragment was ligated to 50 ng of BamHI digested pBR322, available from the American Type Culture Collection (ATCC Designation 31344) by using 1 μl (50 ng) of pBR322 DNA, 6 μl (300 ng) of BamHI restricted (~10 kb fragment) recombinant phage DNA, 1 μl of 10X Ligase Buffer and 1 μl of T4 DNA ligase as described above. E. coli HB101 competent cells, available from Gibco-BRL (Gaithersburg, Maryland), were transformed using standard procedures. HB101 cells were first thawed on ice; then 10 μl of the ligation reaction were mixed with 200 μl of cells and incubated on ice for 30 minutes. Following this, cells were heat shocked for 90 seconds at 42°C, then returned to ice for 2 minutes. One ml of LB broth (per liter 10 g NaCl, 5 g yeast extract, 10 g tryptone) was added and the cells incubated in a New Brunswick air shaker for one hour at 225 rpm, 37°C. Two hundred μl were then plated on LB-agarose with ampicillin (50 μg/ml) and incubated overnight at 37°C. Ampicillin resistant colonies were screened using colony screening methods as detailed in Molecular Cloning, supra, and Grunstein, M. and Hogness, D., Proc. Nat. Acad. Sci., USA, 72:3961 (1975). Again the $J_L$ and Kappa murine probes were used to identify the recombinant pBR322 plasmid, designated as ϕMLCE-10 and was deposited under the Budapest Treaty with the American Type Culture Collection on March 4, 1988 (ATCC Deposit #67639).

E. ISOLATION OF HUMAN DNA

Whole blood was obtained from individuals homozygous for the human gamma haplotypes fbn and azg in 30 ml samples. Buffy coat cells were harvested from the blood samples by centrifugation at 2500 rpm in a Sorvall GLC-2B at 22°C. The buffy coat cells were removed with a pasteur pipet and washed once with PBS. The cell pellets were resuspended in 500 μl of 10 mM Tris-HCl (pH 8.0), 40 mM NaCl and lysed by the addition of 30 μl 10% SDS and 6 μl 20 mg/ml Protease K (Sigma Chemicals, St. Louis, Missouri). The sample was incubated 15 hours at 37°C. The DNA was extracted twice with an equal volume of phenol:chloroform:isoamyl alcohol (25:24:1), twice more with chloroform:isoamyl alcohol (24:1) and dialyzed against 10 mM Tris-HCl (pH 8.0), 1mM EDTA. The DNA was then treated for two hours with 50 μg/ml RNAse A (Sigma Chemicals) followed by redigestion with 200 μg/ml Protease K for 1 hour. The DNA was extracted and dialyzed as described above and the concentration determined by $OD_{260}$ to range from 100-200 mg/ml.

F. CONSTRUCTION OF A HUMAN GENOMIC PHAGE LIBRARY

Human DNA of the fbn haplotype (210 μg as isolated above) was partially digested with MboI, DNA fragments in the molecular weight range 12-24 kb isolated, and cloned into EMBL-3 phage as described in Section B above.

G. ISOLATION OF RECOMBINANT PLASMID pHFK-1

In order to isolate the human kappa constant region gene, the EMBL-3 library, in the manner described in Section B, was screened with a human kappa probe supplied by Dr. P. Hieter, Johns Hopkins University, Baltimore, Maryland, the sequence of which is available from the NIH Data Base, accession number J00241. A total of $5x10^5$ recombinant phage were screened as described above. A single clone was isolated and designated ϕHKF-1. Fifteen μl of ϕHKF-1 DNA were digested with BamHI and HindIII in React Buffer #3 (Gibco-BRL, Gaithersburg, Maryland). A ~5.2 kb fragment was isolated on DEAE 81 paper and ligated into the cloning vector pBR322 as described in Section D above. Ampicillin resistant recombinant colonies were again identified using the human kappa probe; a single clone was isolated and designated pHFK-1. pHFK-1 was deposited under the Budapest Treaty with the American Type Culture Collection on March 4, 1988 (ATCC Deposit #67637.

## H. CONSTRUCTION OF PLASMID pHKCE-10 INCLUDING CEM 231.6.7 $V_L$ KAPPA GENE AND HUMAN C KAPPA GENE

A ~3.8 kb HindIII fragment from pMLCE-10 containing the entire CEM 231.6.7 variable kappa region was further subcloned into the HindIII site of plasmid pHKF-1, described in Section E, using the methods described in Section D to form a chimeric plasmid having the murine CEM 231.6.7 variable light region fused to a human constant kappa region gene. 1 µg of pMLCE-10 DNA was digested with HindIII using React Buffer #2 (Gibco-BRL, Gaithersburg, Maryland) at 1 unit/µg of DNA. 1.2 µg of pHKF1 was digested in a similar manner. The pMLCE-10 digested DNA was electrophoresed as above and the ~3.8 kb HindIII fragment was isolated onto DEAE 81 paper and eluted in 5 µl of TE. One µg (2 µl) of HindIII digested pMLCE-10 were ligated to 600 ng of HindIII digested pHKF-1 in the presence of 10X Ligation Buffer, 10 mM ATP and 2 units of T4 DNA ligase of total volume of 10 µl. The ligation was performed at 12°C overnight and HB101 competent cells from BRL were used in the plasmid transformation, as described above. The plasmid designated as pHKCE-10 was identified by restriction mapping of the plasmid DNA.

## I. CONSTRUCTION OF PLASMID pGCEMK INCLUDING CHIMERIC LIGHT CHAIN IMMUNOGLOBULIN GENES

The eukaryotic expression vector containing the murine $V_L$ region fused to the human kappa gene was constructed using the vector pSV2gpt, available from the American Type Culture Collection, (ATCC Designation #37145). One µg of pSV2gpt DNA was digested with the restriction enzyme EcoRI using 1 unit/µg of DNA in Reaction Buffer #3 (Gibco-BRL, Gaithersburg, Maryland). The EcoRI fragments were blunt-ended by adding 10 µl of 5 mM each of the 3 deoxyribonucleotides dTTP, dGTP, dCTP and dATP, 2 units of Klenow enzyme and a 10X buffer (0.5 M Tris-HCl, pH 7.5; 0.1 M $MgCl_2$; 10 mM dithiothreitol) for a total of 50 µl, as described in Molecular Cloning, supra. The reaction proceeded for 30 minutes at room temperature and was followed by a ligation reaction in which phosphorylated ClaI linkers (2 µg) (New England BioLabs, Beverly, Massachusetts) were ligated to the 500 ng of EcoRI blunted-ended pSV2gpt in order to create a new ClaI site in the final vector. The ClaI linker sequence was d(pCATCGATG). Ligation reactions were carried out as described earlier. Following ligation, excess linker was removed by electrophoresis and the linear pSV2gpt-ClaI fragment was isolated on DEAE 81 paper as described above. The isolated DNA was self-ligated and HB101 competent cells were transformed as described earlier and the ampicillin resistant colonies analyzed by restriction enzyme digestion.

The resulting vector, pSV2gpt-Cla, was then digested with ClaI and BamHI restriction enzymes (1 unit/µg of DNA). The chimeric vector pHKCE-10 was digested also with these two enzymes. The ~4.5 kb pSV2gpt ClaI-BamHI fragment and the ~9 kb ClaI-BamHI pHKCE-10 fragment were isolated on DEAE 81 paper as described. These fragments were ligated as described above, using 375 ng of the ~9 kb fragment insert DNA and 200 ng of the ~4.5 kb vector DNA. Following transformation of HB101, a recombinant plasmid, designated as pGCEMK, was identified by restriction mapping of the plasmid DNA. This plasmid, which is the chimeric expression vector used to produce the CEM chimeric light chain polypeptide, is shown with restriction sites in Figure 17.

## J. ISOLATION OF THE RECOMBINANT PHAGE φMHCE-30 INCLUDING CEM 231.6.7 $V_h$ GENE

In order to identify the CEM 231.6.7 gamma chain, the EMBL-3 library described above was screened with two murine heavy chain probes, one representing the $J_h$ 3-4 regions, was obtained from Dr. Phil Tucker, University of Texas, Dallas, Texas, and the other representing sequences in the murine gamma-1 gene. This latter probe comprises the sequence:
5′-CTG-TAC-ATA-TGC-AAG-GCT-TAC-AAC-3′ as determined from the General Bank Data Base (NIH Accession #J00453) and was synthesized by Molecular Biosystems, Inc. (San Diego, California). A total of $4.8 \times 10^5$ recombinant phage plaques were screened in duplicate using these two probes to identify the clone containing both the $J_h$ region and the gamma region. Again, as with the kappa clone, a phage containing sequences for these two regions indicates that the DNA has been rearranged, thus identifying the expressed immunoglobulin gene in the cell line CEM 231.6.7. A single CEM 231.6.7 clone was selected on the basis of its containing such rearranged DNA and was designated φMHCE-30.

K. CONSTRUCTION OF pMHCE-30 INCLUDING CEM 231.6.7 $V_h$ GENE

By restriction mapping, a ~5.0 kb SstI fragment from ϕMHCE-30 was identified which contained the heavy chain variable region sequences as well as the major intron containing the murine heavy chain enhancer. To subclone this fragment into a plasmid, 10 μg of the phage DNA was digested with the restriction enzyme SstI in Reaction Buffer #2 (BRL-Gibco, Gaithersburg, Maryland) and the ~5.6 kb fragment was isolated following electrophoresis by the DEAE 81 method described earlier. The Bluescript vector M13-SK+, commercially available from Stratagene, Inc. (La Jolla, California) was also digested with SstI. The vector and isolated ~5.6 kb insert DNA were ligated at a ratio of 1:10 in a total of 10 μl as detailed above. Following transformation of HB101 competent cells, the desired recombinant was identified by restriction digest mapping, and designated as pMHCE-30. pMHCE-30 was deposited under the Budapest Treaty with the American Type Culture Collection on March 4, 1988 (ATCC Deposit #67640).

L. ISOLATION OF HUMAN DNA

Human DNA was isolated as described above.

M. CONSTRUCTION OF A HUMAN PLASMID LIBRARY

Human DNA of the azg haplotype was digested as 10 μg aliquots using 30 units each of the restriction endonucleases BamHI and HindIII in Reaction Buffer #3 (Gibco-BRL, Gaithersburg, Maryland) (50 mM Tris-HCl, pH 8.0; 10 mM $MgCl_2$; 100 mM NaCl) in a total volume of 200 μl. The digested fragments were concentrated to 20 μl by ethanol precipitation and separated on a 0.6% low-gelling temperature agarose (FMC) gel run for 15 hrs at 50 mAmps. DNA fragments in the size range ~6-7 kb were excised from the gel. The cloning vector pUC18 (described by Yanisch-Perron C., et al., Gene 33:103 (1985) and available commercially from Boehringer-Mannheim Biochemicals, Indianapolis, IN and New England Biolabs, Beverly, MA) was digested with BamHI and HindIII as described above. The human DNA fragments (150 ng) were ligated into pUC18 (50 ng) in a total reaction volume of 400 μl containing 30 mM Tris-HCl (pH 7.6), 10 mM $MgCl_2$, 5 mM dithiothreitol, 1 mM ATP, and 1 μl T4 DNA ligase (2 units) for 72 hours at 15° C. Half (100 ng) of the ligated DNA sample was used to transform 500 μl of freshly prepared competent E. coli M15 cells and the resulting transformants were plated onto X-gal, IPTG, AMP plates (4 μg/ml X-gal, 2 μg/ml IPTG, 100 μg/ml Ampicillin).

N. ISOLATION OF RECOMBINANT PLASMID pHG1Z

Ampicillin resistant pUC18 colonies containing the recombinant human DNA were screened by the method described above using a human Gamma-2 probe supplied by T. Honjo, University of Osaka, Japan and described by Takahashi, N. et al., Cell., 29:671-679 (1982). A clone containing a ~7.5 kb insert corresponding to the human Gamma-1 gene was identified and designated HyHG1. This same ~7.5 kb HindIII-BamHI fragment containing the human gamma constant region gene was subsequently re-cloned into pBR322 using the same procedure described in Section G. The pBR322 vector containing the human gamma-1 gene was designated pHG1Z and was deposited under the Budapest Treaty with the American Type Culture Collection of March 4, 1988 (ATCC Deposit #67638).

O. CONSTRUCTION OF pHGCEM-30 INCLUDING CEM 231.6.7 $V_h$ GENE AND HUMAN GAMMA-1 GENE

The murine variable heavy chain region was fused to the human gamma-1 gene in the following manner. Ten μg of pMHCE-30 was digested with ClaI (1 unit/μg) and then partially digested with HindIII to give a ~5.3 kb fragment containing $V_h$ and the major intron. Partial digests were performed by using only 0.1 unit/μg of DNA and a digestion time of 1 hour at 37° C. One μg of the plasmid pHGZ-1, described above, containing the human gamma-1 gene also was digested with ClaI and HindIII. The ~5.3 kb fragment from pMHCE-30 was isolated from a TBE gel using the DEAE 81 protocol described earlier. This fragment was ligated into the ClaI-HindIII site of pHGZ-1 by using 600 ng of the insert and 200 ng of the vector DNA in a ligation mixture of 10 μl total volume. Ligation reactions were carried out as detailed above. The recombinant plasmids resulting from transformation of HB101 were analyzed by restriction digest mapping in order to identify a plasmid containing the murine $V_h$ region fused to a human gamma-1 gene, and was designated pHGCEM-30.

P. CONSTRUCTION OF pNCEMG1 INCLUDING CHIMERIC HEAVY CHAIN IMMUNOGLOBULIN GENES

The chimeric Ig gene was inserted to the eukaryotic expression vector essentially as detailed in Section H. The vector used was pSV2neo, available from the American Type Culture Collection (ATCC Designation #37149). A ClaI site was added to this vector exactly as described for the pSV2gpt vector. Following this, 1 µg of pSV2neo-Cla DNA was digested with the enzymes ClaI and BamHI using 1 unit/µg of DNA. The plasmid pHGCEM-30 was digested with ClaI and then partially digested with BamHI (0.1 unit/µg) in order to obtain a fragment of ~12.7 kb which contained the chimeric $V_h$ and gamma-1 regions. This fragment was isolated on DEAE 81 paper and eluted in 10 µl of TE Buffer. The ligation was performed using 50 ng of vector DNA, 400 ng of the ~12.7 kb insert DNA, 10X ligation buffer, 10 mM ATP and T4 DNA ligase, as described above, at 12°C overnight. HB101 cells were transformed and the correct recombinant plasmid constituting the chimeric expression vector pNCEMG1 was identified. Plasmid pNCEMG1, which constitutes the recombinant expression vector used to express chimeric heavy chain immunoglobulin genes, is shown in Figure 18.

Q. TRANSFECTION OF CHIMERIC LIGHT CHAIN GENE WITH THE CHIMERIC CONSTRUCT pGCEMK

The light chain immunoglobulin plasmid used for transfection was pGCEMK, as described above. The pGCEMK plasmid, containing the chimeric variable light ($V_k$) CEM gene fused to the human kappa gene, was first transfected into SP2/0 hybridoma cells by the electroporation techniques referenced above. The SP2/0-Ag14 cells were grown in media containing 5% FCS and maintained in a log phase of growth for the three days preceeding electroporation. Twenty µg of the plasmid vector pGCEMK were linearized using the restriction enzyme PvuI (1 unit/µg) and the Reaction Buffer #7 (Gibco-BRL, Gaithersburg, Maryland). At the time of transfection the SP2/0 cells were collected by centrifugation in an IEC clinical centrifuge - 800 rpm, 10 minutes at room temperature. Cells then were washed 3X in Hanks Buffered Saline Solution (Gibco Laboratories, Grand Island, New York) with 6 mM Dextrose and resuspended at a final concentration of 3.0 x $10^7$ cells/ml. Cells (0.3 ml) were aliquoted into cuvettes at a density of 1 x $10^7$/0.3 ml and the linearized DNA was added. The mixture was maintained on ice for 10 minutes. Electroporation was done using the .8 mm gap electrode (P/N 472) and the BTX 100 Transfector (BTX, Inc. San Diego, California). Conditions were 3 pulses, 100 µseconds each at 300 volts. The electroporated cells were then resuspended in media at a density of 2 x $10^5$/ml (in T75 flasks) for 72 hours (37°C, 5% $CO_2$). Cells then were plated in the appropriate antibiotic at a density of 5 x $10^4$/ml in 24 well plates; SP2/0 cells containing pGCEMK were plated in HMAX 1.0 Media (50 ng/ml Hypoxanthine, 250 ng/ml Mycophenolic Acid and 50 µg/ml Xanthine), available from Sigma, St. Louis, Missouri, at 1 µg/ml. Two hundred µl of supernatant were collected from each well containing HMAX resistant colonies. This supernatant then was assayed for the presence of a human kappa constant region gene which would indicate expression of the chimeric immunoglobulin genes of pGCEMK.

R. IDENTIFICATION OF SP2/0 CELLS SECRETING CHIMERIC CEM 231.6.7

Transfected SP2/0 cells expressing the chimeric CEM-human kappa genes were identified by a standard enzyme-linked immunosorbent assay (ELISA), as described by Engvall, E. and Perlmann, P., Immunochemistry, 8:871-874 (1971), for human kappa sequences.

The purpose of this assay was to identify those cells secreting the chimeric kappa chain polypeptide coded for by the pGCEMK plasmid vector which was constructed from murine variable regions isolated from the murine hybridoma CEM 231.6.7 and fused to the human gamma-1 gene. A 5 µg/ml solution of goat anti-human kappa chain (Tago, Burlingame, California, #4106) in 10 mM sodium phosphate, pH 7-8, was prepared. Each well of a 96 well plate was coated with 50 µl of this solution. The plates were then incubated overnight at 37°C. Plates were then rinsed throughly in $H_2O$ and PBS + 0.1% Tween (w/v). Fifty µl of the supernatant fractions were added to each well, and incubated for 2 hours at room temperature. Plates were again rinsed as detailed above. A goat anti-human kappa chain alkaline phosphatase conjugate (Tago, Inc., Burlingame, California, #2496) was diluted 1:1000 in the same medium as the supernatant material. 100 µl were added per well and allowed to incubate for 1 hour at room temperature. Plates were rinsed as above. The alkaline phosphatase substrate was prepared as per package instruction, 1 tablet per 3 ml of distilled $H_2O$ and 150 µl of this substrate was added to each well and allowed to incubate 30 minutes at 37°C. The reaction was quenched with 50 µl of 300 mM EDTA and then the absorbance was read at 405 nM. Those supernatants showing the highest levels of kappa expression were identified and the cells from the corresponding wells were pooled and expanded for introduction of the chimeric construct

pNCEMG1.

S. TRANSFECTION OF CHIMERIC KAPPA PRODUCING CELLS WITH THE HEAVY CHAIN CHIMERIC CONSTRUCT pNCEMG1

The heavy chain immunoglobulin plasmids used for transfection into SP2/0 cells was pNCEMG1, derived from constructs as detailed above. The populations of cells expressing the chimeric CEM-human kappa genes which were pooled were next electroporated with the plasmid constructs containing the chimeric CEM heavy chain genes. As for the kappa gene electroporation, the SP2/0 chimeric kappa producing cells (SP2/0-K) were maintained at log phase of growth for the three days preceeding the electroporation. Twenty micrograms of the plasmid DNA pNCEMG1 was linearized with the enzyme PvuI in React Buffer #6 (Gibco-BRL, Gaithersburg, Maryland). Cells were collected, washed and resuspended at a density of 3 x $10^7$ cells/ml. The DNA was added and the mixture held on ice for 10 minutes preceeding the electroporation. Conditions used were 1 pulse at 5 $\mu$seconds, 250 volts. Cells were plated at 2.5 x $10^5$/ml in HH2, 5% FCS plus HMAX 1.0 for 72 hours at 37°C, 5% $CO_2$. These cells then were plated at 5 x $10^4$/ml in 24 well plates in medium containing HMAX 1.0 and G418 antibiotic (Geneticin, Gibco-BRL, Gaithersburg, Maryland) at an active concentration of 500 $\mu$g/ml. Selection was maintained for 14 days at which time those wells with HMAX/G418 resistant colonies were identified for further analysis.

T. IDENTIFICATION AND ANALYSIS OF CHIMERIC ANTIBODY SP2/0 CELLS SECRETING CEM-CHIMERIC ANTIBODY

Screening assays were performed to identify transfected SP2/0 cells which expressed both the chimeric CEM light and heavy chain immunoglobulin genes, producing antibody which binds CEA. The assay procedures used for the detection of antibodies directed against CEA were standard solid phase radioimmunoassays, essentially as described by Wang, R., et al., Immunol. Methods, 18:157-164 (1977).

The following reagents were added to wells of a microtiter plate and incubated overnight, at room temperature, with mixing: 25 $\mu$l cell culture supernatant, 50 $\mu$l $^{125}$I-CEA (affinity purified), 20 $\mu$l of Sepharose bound goat anti-human IgG and 25 $\mu$l cell culture media. Immune complexes bound to the Sepharose-anti human IgG were collected onto paper filters. Filters were counted in a gamma counter. The radioimmunoassays resulted in the identification of a chimeric anti-CEA specific antibody, which was designated as XCEM449.

U. PREPARATION OF THE CHIMERIC ANTIBODY-DEXTRAN ADDUCTS

Dextran, MW6000, was activated as described in Preparation 1. The XCEM449, as prepared above, was modified with oxidized Dextran as described in Example I. This generated monomeric adducts possessing 10 moles of dextran per mole of antibody.

V. DETERMINATION OF IMMUNOREACTIVITY OF CHIMERIC XCEM499-DEXTRAN ADDUCTS

The immunoreactivity assay was performed as described in Example V, paragraph A. The results of this study are shown in Table IX.

TABLE IX

| IMMUNOREACTIVITY OF XCEM499-DEXTRAN | | |
|---|---|---|
| | % I.R. | % of Control |
| XCEM449 (CONTROL) | 91.3 | 100 |
| XCEM449 DEXTRAN (14) | 67.6 | 74 |

W. PHARMACOKINETICS OF CHIMERIC XCEM449-DEXTRAN ADDUCTS

Unmodified chimeric XCEM449, as well as antibody modified with 10 moles of dextran per mole of antibody were radiolabelled and injected intravenously in Balb/c mice. Biodistribution studies were performed at 4 and 48 hours post-administration. The results of these studies are shown in Figures 16a and 16b, respectively. As shown in these figures, dextran-modified XCEM449 chimeric antibody exhibited pharmacokinetics substantially unaltered from that of control, unmodified chimeric antibody.

EXAMPLE X

PREPARATION OF ANTI-DEXTRAN ANTIBODIES

Dextran, MW6000, was activated as described in Preparation 1. Bovine Thyroglobulin (Sigma Chemical Co., St. Louis, MO) was modified with 30 moles of dextran per mole of protein as described in Example 1. Bovine serum albulin (BSA, Sigma Chemical Co., St. Louis, MO) was modified with 10 moles of dextran per mole of protein. Six subcutaneous immunizations were performed in mice over a 4-month period with 250 µg of thyroglobulin-Dextran emulsified with complete Freund's adjuvant. Sera were collected two weeks after each immunization and tested for the presence of anti-dextran antibodies with ELISA as described in Example 2, paragraph B. Microtiter plate wells were coated with thyroglobulin, thyroglobulin-Dextran, BSA and BSA-Dextran. Assays were performed as described, with a preparation of HRP-conjugated, goat anti-mouse immunoglobulin (TAGO, Inc., Burlingame, CA) as a source of secondary antibodies. Results are shown in Table X. Sera were tested at serial dilutions. Relative titres were defined as the reciprocal of the dilution resulting in 50% of maximum response. After repeated immunizations with thyroglobulin-dextran under these rigorous conditions, the sera of these mice exhibited reactivity against thyroglobulin and, to a much less extent, against thyroglobulin-dextran. However, no specific reactivity was detected against dextran, as evidenced by lack of reactivity against dextran molecules bound to BSA, as well as lack of non-specific reactivity against the carrier protein.

TABLE X

| DETERMINATION OF ANTI-DEXTRAN ANTIBODIES | |
|---|---|
| SUBSTRATE | TITRES |
| Thyroglobulin | 100,000 |
| Thyroglobulin-dextran | 10,000 |
| BSA | <50 |
| BSA-dextran | <50 |

These observations show that repeated administrations of dextran-modified proteins in mice consistently fail to trigger a specific anti-dextran immune response.

**Claims**

**Claims for the following Contracting States : AT, BE, CH, DE, FR, GB, IT, LI, LU, NL, SE**

1. A substantially monomeric immunoreactive composition with lowered immunogenic potential, said composition comprising an immunoglobulin, or antigen-recognizing fragment thereof, covalently linked to a low molecular weight polysaccharide derivative.

2. An immunoreactive composition as claimed in claim 1, in which said immunoglobulin, or antigen-recognizing fragment thereof, retains immunoreactivity and localization properties substantially unaltered from that of the free immunoglobulin, or antigen-recognizing fragment thereof.

3. An immunoreactive composition as claimed in claim 1 or 2 in which the covalently linked immunoglobulin or fragment possesses substantially unaltered or improved pharmacokinetics.

4. An immunoreactive composition, as claimed in claim 3, in which the low molecular weight polysaccharide derivative is an oxidized polysaccharide.

5. An immunoreactive composition as claimed in Claim 4 in which the oxidized polysaccharide is a low molecular weight oxidized dextran.

6. An immunoreactive composition, as claimed in claim 5, in which the average molecular weight of the oxidized dextran is from about 3,000 to about 16,000.

7. An immunoreactive composition, as claimed in claim 6, in which the average molecular weight of the oxidized dextran is from about 6,000 to about 16,000.

8. An immunoreactive composition, as claimed in claim 7, in which the average molecular weight of the oxidized dextran is about 6,000.

9. An immunoreactive composition, as claimed in any one of claims 5 to 8, which comprises an average from about one to about twenty oxidized dextran molecules per molecule of immunoglobulin or antigen-recognizing fragment thereof.

10. An immunoreactive composition, as claimed in claim 9, in which the immunoglobulin, or antigen recognizing fragment thereof, is a polyclonal or monoclonal antibody or antigen-recognizing fragment thereof.

11. An immunoreactive composition as claimed in claim 10 in which the immunoglobulin, or fragment thereof, is bound to a drug or a metal chelating agent.

12. An immunoreactive composition, as claimed in claim 11, in which the drug is adriamycin, methotrexate, vinblastine, or desacetylvinblastine, or cisplatin.

13. An immunoreactive composition, as claimed in claim 10, in which the immunoglobulin, or antigen-recognizing fragment thereof, is a monoclonal antibody or an antigen-recognizing fragment thereof.

14. An immunoreactive composition, as claimed in any one of claims 1 to 13, in which the immunoglobulin or antigen-recognizing fragment thereof, recognizes a tumor associated antigen.

15. An immunoreactive composition, as claimed in any one of claims 1 to 14, in which the immunoglobulin or an antigen recognizing fragment thereof, is T101, KS1/4, 9.2.27, RAF, ZCE025, or an antigen-recognizing fragment thereof.

16. An immunodiagnostic or immunotherapeutic formulation which comprises, as an active ingredient, an immunoreactive composition, as claimed in any one of claims 1 to 15, 20 to 30 or 32 to 40, associated with a pharmaceutically-acceptable carrier, diluent, or excipient therefor.

17. An immunoreactive composition, as claimed in any one of claims 1 to 15 or 20 to 28, for use in immunotherapy.

18. A process for preparing an immunoreactive composition as claimed in any one of claims 1 to 15, which comprises reducing with a suitable reducing agent a free aldehyde group present on the polysaccharide portion of the covalently linked immunoglobulin or antigen-recognizing fragment thereof, said linked immunoglobulin or an antigen recognizing fragment having been prepared by reacting a free immunoglobulin or immunoglobulin fragment with an oxidized polysaccharide and then reducing the resulting Schiff base adducts with a mild reducing agent.

19. A process as claimed in claim 30, in which the suitable reducing agent is sodium borohydride.

20. An immunoreactive composition as claimed in any one of claims 1 to 15 in which the immunoglobulin or fragment thereof is a bifunctional antibody or fragment thereof.

21. An immunoreactive composition as claimed in any one of claims 1 to 15 in which the immunoglobulin or immunoglobulin fragment thereof is a chimeric antibody or fragment thereof.

22. An immunoreactive composition as claimed in claim 20 in which the bifunctional antibody or fragment thereof is a synthetic bifunctional antibody or fragment thereof.

**23.** An immunoreactive composition as claimed in claim 22 in which the synthetic bifunctional antibody or fragment thereof is a synthetic covalently cross-linked antibody or fragment thereof.

**24.** An immunoreactive composition as claimed in claim 21 in which the chimeric antibody or fragment thereof is a humanized antibody, or fragment thereof.

**25.** An immunoreactive composition as claimed in claim 21 in which the chimeric antibody is a chimeric bifunctional antibody, or fragment thereof.

**26.** An immunoreactive composition as claimed in claim 25 in which the chimeric bifunctional antibody is a synthetic covalently cross-linked chimeric-bifunctional antibody, or fragment thereof.

**27.** An immunoreactive composition as claimed in claim 25 in which the chimeric bifunctional antibody, or fragment thereof, is humanized.

**28.** An immunoreactive composition as claimed in claim 26 in which the synthetic antibody, or fragment thereof, is humanized.

**29.** An immunoreactive composition as claimed in any one of claims 1 to 15 or claims 20 to 28 in which the covalently linked immunoglobulin, or fragment thereof, has reduced kidney uptake characteristics.

**30.** An immunoreactive composition as claimed in claim 29 in which the immunoglobulin or fragment thereof, has an increased plasma half-life.

**31.** A method for improving the pharmacokinetics of an immunoglobulin, or antigen-recognizing fragment thereof, which comprises covalently linking an immunoglobulin, or antigen-recognizing fragment thereof, to a low molecular weight polysaccharide derivative.

**32.** A substantially monomeric immunoreactive composition possessing improved pharmacokinetics said composition comprising an immunoglobulin, or antigen-recognizing fragment thereof, covalently linked to a low molecular weight polysaccharide derivative.

**33.** A composition as claimed in claim 32 in which said immunoglobulin, or antigen recognizing fragment thereof, is a bifunctional antibody or fragment thereof.

**34.** A composition as claimed in claim 32 which comprises a chimeric antibody or fragment thereof.

**35.** A composition as claimed in claim 34 which is a chimeric-bifunctional antibody, or fragment thereof.

**36.** A composition as claimed in claim 34 which is humanized.

**37.** A composition as claimed in claim 35 which is humanized.

**38.** A composition as claimed in claim 32 which possesses reduced kidney uptake characteristics.

**39.** A composition as claimed in claim 32 which has an increased plasma half-life.

**40.** A composition as claimed in claim 38 which has an increased plasma half-life.

**Claims for the following Contracting States : ES, GR**

**1.** A process for preparing a substantially monomeric immunoreactive composition with lowered immunogenic potential, said composition comprising an immunoglobulin, or antigen-recognizing fragment thereof, covalently linked to a low molecular weight polysaccharide derivative said process comprising reducing with a suitable reducing agent a free aldehyde group present on the polysaccharide portion of the covalently linked immunoglobulin or antigen-recognizing fragment thereof.

2. A process as claimed in claim 1 in which said covalently linked immunoglobulin, or antigen-recognizing fragment thereof, is prepared by reacting a free immunoglobulin, or fragment thereof, with an oxidized low molecular weight polysaccharide, followed by reduction, with a mild reducing agent, the resulting Schiff base adduct.

3. A process for preparing an immunoreactive composition as claimed in claim 1 or 2 in which said immunoglobulin, or antigen-recognizing fragment thereof, retains immunoreactivity and localization properties substantially unaltered from that of the free immunoglobulin, or antigen-recognizing fragment thereof.

4. A process for preparing an immunoreactive composition as claimed in any of claims 1 to 3 in which the covalently linked immunoglobulin or fragment possesses substantially unaltered or improved pharmacokinetics.

5. A process for preparing an immunoreactive composition as claimed in Claim 1, 3 or 4 in which the polysaccharide derivative is a low molecular weight oxidized dextran.

6. A process for preparing an immunoreactive composition, as claimed in claim 5, in which the average molecular weight of the oxidized dextran is from about 3,000 to about 16,000.

7. A process for preapring an immunoreactive composition, as claimed in claim 6, in which the average molecular weight of the oxidized dextran is from about 6,000 to about 16,000.

8. A process for preparing an immunoreactive composition, as claimed in claim 7, in which the average molecular weight of the oxidized dextran is about 6,000.

9. A process for preparing an immunoreactive composition, as claimed in any one of claims 5 to 8, which comprises an average from about one to about twenty oxidized dextran molecules per molecule of immunoglobulin or antigen-recognizing fragment thereof.

10. A process for preparing an immunoreactive composition, as claimed in claim 9, in which the immunoglobulin, or antigen recognizing fragment thereof, is a polyclonal or monoclonal antibody or antigen-recognizing fragment thereof.

11. A process for preparing an immunoreactive composition as claimed in claim 10 in which the immunoglobulin, or fragment thereof, is bound to a drug or a metal chelating agent.

12. A process for preparing an immunoreactive composition, as claimed in claim 11, in which the drug is adriamycin, methotrexate, vinblastine, or desacetylvinblastine, or cisplatin.

13. A process for preparing an immunoreactive composition, as claimed in any one of claims 1 to 9, in which the immunoglobulin, or antigen-recognizing fragment thereof, is a monoclonal antibody or an antigen-recognizing fragment thereof.

14. A process for preparing an immunoreactive composition, as claimed in any one of claims 1 to 13, in which the immunoglobulin or antigen-recognizing fragment thereof, recognizes a tumor associated antigen.

15. A process for preparing an immunoreactive composition, as claimed in any one of claims 1 to 14, in which the immunoglobulin or an antigen-recognizing fragment thereof, is T101, KS1/4, 9.2.27, RAF, ZCE025, or an antigen-recognizing fragment thereof.

16. A process for preparing an immunoreactive composition as claimed in any one of claims 1 to 15 in which the immunoglobulin or fragment thereof is a bifunctional antibody or fragment thereof.

17. A process for preparing an immunoreactive composition as claimed in any one of claims 1 to 15 in which the immunoglobulin or immunoglobulin fragment thereof is a chimeric antibody or fragment thereof.

**18.** A process for preparing an immunoreactive composition as claimed in claim 16 in which the bifunctional antibody or fragment thereof is a synthetic bifunctional antibody or fragment thereof.

**19.** A process for preparing an immunoreactive composition as claimed in claim 18 in which the synthetic bifunctional antibody or fragment thereof is a synthetic covalently cross-linked antibody or fragment thereof.

**20.** A process for preparing an immunoreactive composition as claimed in claim 17 in which the chimeric antibody or fragment thereof is a humanized antibody, or fragment thereof.

**21.** A process for preparing an immunoreactive composition as claimed in claim 17 in which the chimeric antibody is a chimeric bifunctional antibody, or fragment thereof.

**22.** A process for preparing an immunoreactive composition as claimed in claim 21 in which the chimeric-bifunctional antibody is a synthetic covalently cross-linked chimeric-bifunctional antibody, or fragment thereof.

**23.** A process for preparing an immunoreactive composition as claimed in claim 21 in which the chimeric bifunctional antibody, or fragment thereof, is humanized.

**24.** A process for preparing an immunoreactive composition as claimed in claim 22 in which the synthetic antibody, or fragment thereof, is humanized.

**25.** A process for preparing an immunoreactive composition as claimed in any one of claims 1 to 24 in which the covalently linked immunoglobulin, or fragment thereof, has reduced kidney uptake characteristics.

**26.** A process for preparing an immunoreactive composition as claimed in claim 25 in which the immunoglobulin or fragment thereof, has an increased plasma half-life.

**27.** A process as claimed in any one of claims 1 to 26 in which the reducing agent for the free aldehyde group is sodium borohydride and the reaction is carried out at about 0° C. to about 25° C.

**28.** A process for preparing an immunodiagnostic or immunotherapeutic composition which comprises, admixing an immunoreactive composition, as defined in any one of claims 1 to 26 with a pharmaceutically-acceptable carrier, diluent, or excipient therefor.

**Patentansprüche**

**Patentansprüche für folgende Vertragsstaaten : AT, BE, CH, DE, FR, GB, IT, LI, LU, NL, SE**

**1.** Im wesentlichen monomere immunreaktive Zusammensetzung mit verringertem immunogenen Potential, wobei diese Zusammensetzung ein Immunglobulin oder antigenerkennendes Fragment hiervon enthält, das kovalent an ein Polysaccharidderivat mit niedrigem Molekulargewicht gebunden ist.

**2.** Immunreaktive Zusammensetzung nach Anspruch 1, worin dieses Immunglobulin oder antigenerkennende Fragment hiervon die Immunreaktivität behält und die Lokalisierungseigenschaften zu denen von freiem Immunglobulin oder eines antigenbindenden Fragments hiervon im wesentlichen unverändert sind.

**3.** Immunreaktive Zusammensetzung nach Anspruch 1 oder 2, worin das kovalent gebundene Immunglobulin oder Fragment eine im wesentlichen unveränderte oder verbesserte Pharmakokinetik aufweist.

**4.** Immunreaktive Zusammensetzung nach Anspruch 3, worin das Polysaccharidderivat mit niedrigem Molekulargewicht ein oxidiertes Polysaccharid ist.

**5.** Immunreaktive Zusammensetzung nach Anspruch 4, worin das oxidierte Polysaccharid ein oxidiertes Dextran mit niedrigem Molekulargewicht ist.

6. Immunreaktive Zusammensetzung nach Anspruch 5, worin das durchschnittliche Molekulargewicht des oxidierten Dextrans etwa 3 000 bis etwa 16 000 beträgt.

7. Immunreaktive Zusammensetzung nach Anspruch 6, worin das durchschnittliche Molekulargewicht des oxidierten Dextrans etwa 6 000 bis 16 000 beträgt.

8. Immunreaktive Zusammensetzung nach Anspruch 7, worin das durchschnittliche Molekulargewicht des oxidierten Dextrans etwa 6 000 beträgt.

9. Immunreaktive Zusammensetzung nach einem der Ansprüche 5 bis 8, die im Durchschnitt etwa ein bis etwa zwanzig oxidierte Dextranmoleküle pro Molekül Immunglobulin oder antigenerkennendem Fragment hiervon enthält.

10. Immunreaktive Zusammensetzung nach Anspruch 9, worin das Immunglobulin oder antigenerkennende Fragment hiervon ein polyklonaler oder monoklonaler Antikörper oder ein antigenerkennendes Fragment hiervon ist.

11. Immunreaktive Zusammensetzung nach Anspruch 10, worin das Immunglobulin oder antigenerkennende Fragment hiervon an ein Arzneimittel oder einen Metallchelatbildner gebunden ist.

12. Immunreaktive Zusammensetzung nach Anspruch 11, worin das Arzneimittel Adriamycin, Methotrexat, Vinblastin oder Desacetylvinblastin oder Cisplatin ist.

13. Immunreaktive Zusammensetzung nach Anspruch 10, worin das Immunglobulin oder antigenerkennende Fragment hiervon ein monoklonaler Antikörper oder ein antigenerkennendes Fragment hiervon ist.

14. Immunreaktive Zusammensetzung nach einem der Ansprüche 1 bis 13, worin das Immunglobulin oder antigenerkennende Fragment hiervon ein tumorassoziiertes Antigen erkennt.

15. Immunreaktive Zusammensetzung nach einem der Ansprüche 1 bis 14, worin das Immunglobulin oder antigenerkennende Fragment hiervon T101, KS1/4, 9.2.27, RAF, ZCE025 oder ein antigenerkennendes Fragment hiervon ist.

16. Immundiagnostische oder immuntherapeutische Formulierung, welche als aktiven Bestandteil eine immunreaktive Zusammensetzung nach einem der Ansprüche 1 bis 15, 20 bis 30 oder 32 bis 40 zusammen mit einem pharmazeutisch annehmbaren Träger, Verdünnungsmittel oder Hilfsstoff enthält.

17. Immunreaktive Zusammensetzung nach einem der Ansprüche 1 bis 15 oder 20 bis 28 zur Verwendung in der Immuntherapie.

18. Verfahren zur Herstellung einer immunreaktiven Zusammensetzung nach einem der Ansprüche 1 bis 15, gekennzeichnet durch die Reduktion einer auf dem Polysaccharidteil des kovalent gebundenen Immunglobulins oder eines antigenerkennenden Fragments hiervon vorkommenden freien Aldehydgruppe mit einem geeigneten Reduktionsmittel, wobei das gebundene Immunglobulin oder antigenerkennende Fragment durch Umsetzung eines freien Immunglobulins oder Immunglobulinfragments mit einem oxidierten Polysaccharid und anschließende Reduktion des entstehenden Addukts einer Schiffschen Base mittels eines milden Reduktionsmittels hergestellt wurde.

19. Verfahren nach Anspruch 30, worin das geeignete Reduktionsmittel Natriumborhydrid ist.

20. Immunreaktive Zusammensetzung nach einem der Ansprüche 1 bis 15, worin das Immunglobulin oder das Fragment hiervon ein bifunktioneller Antikörper oder ein Fragment hiervon ist.

21. Immunreaktive Zusammensetzung nach einem der Ansprüche 1 bis 15, worin das Immunglobulin oder das Immunglobulinfragment hiervon ein chimärer Antikörper oder ein Fragment hiervon ist.

22. Immunreaktive Zusammensetzung nach Anspruch 20, worin der bifunktionelle Antikörper oder das Fragment hiervon ein synthetischer bifunktioneller Antikörper oder ein Fragment hiervon ist.

23. Immunreaktive Zusammensetzung nach Anspruch 22 worin der synthetische bifunktionelle Antikörper oder das Fragment hiervon ein synthetisch kovalent quervernetzter Antikörper oder ein Fragment hiervon ist.

24. Immunreaktive Zusammensetzung nach Anspruch 21, worin der chimäre Antikörper oder das Fragment hiervon ein humanisierter Antikörper oder ein Fragment hiervon ist.

25. Immunreaktive Zusammensetzung nach Anspruch 21, worin der chimäre Antikörper ein chimärer bifunktioneller Antikörper oder ein Fragment hiervon ist.

26. Immunreaktive Zusammensetzung nach Anspruch 25, worin der chimäre bifunktionelle Antikörper ein synthetisch kovalent quervernetzter chimärer bifunktioneller Antikörper oder ein Fragment hiervon ist.

27. Immunreaktive Zusammensetzung nach Anspruch 25, worin der chimäre bifunktionelle Antikörper oder das Fragment hiervon humanisiert ist.

28. Immunreaktive Zusammensetzung nach Anspruch 26, worin der synthetische Antikörper oder das Fragment hiervon humanisiert ist.

29. Immunreaktive Zusammensetzung nach einem der Ansprüche 1 bis 15 oder 20 bis 28, worin das kovalent gebundene Immunglobulin oder Fragment hiervon reduzierte Nierenaufnahmeeigenschaften aufweist.

30. Immunreaktive Zusammensetzung nach Anspruch 29, worin das Immunglobulin oder Fragment hiervon eine erhöhte Plasmahalbwertszeit aufweist.

31. Verfahren zur Verbesserung der Pharmakokinetik eines Immunglobulins oder eines antigenerkennenden Fragments hiervon, gekennzeichnet durch die kovalente Bindung eines Immunglobulins oder antigenerkennenden Fragments hiervon an ein Polysaccharidderivat mit niedrigem Molekulargewicht.

32. Im wesentlichen monomere immunreaktive Zusammensetzung, die eine verbesserte Pharmakokinetik aufweist, wobei diese Zusammensetzung ein an ein Polysaccharidderivat mit niedrigem Molekulargewicht kovalent gebundenes Immunglobulin oder antigenerkennendes Fragment hiervon enthält.

33. Zusammensetzung nach Anspruch 32, worin dieses Immunglobulin oder antigenerkennende Fragment hiervon ein bifunktioneller Antikörper oder ein Fragment hiervon ist.

34. Zusammensetzung nach Anspruch 32, die einen chimären Antikörper oder ein Fragment hiervon enthält.

35. Zusammensetzung nach Anspruch 34, die einen chimären bifunktionellen Antikörper oder ein Fragment hiervon enthält.

36. Zusammensetzung nach Anspruch 34, die humanisiert ist.

37. Zusammensetzung nach Anspruch 35, die humanisiert ist.

38. Zusammensetzung nach Anspruch 32, die reduzierte Nierenaufnahmeeigenschaften aufweist.

39. Zusammensetzung nach Anspruch 32, die eine erhöhte Plasmahalbwertszeit aufweist.

40. Zusammensetzung nach Anspruch 38, die eine erhöhte Plasmahalbwertszeit aufweist.

**Patentansprüche für folgende Vertragsstaaten : ES, GR**

1. Verfahren zur Herstellung einer im wesentlichen monomeren immunreaktiven Zusammensetzung mit verringertem immunogenen Potential, wobei diese Zusammensetzung ein Immunglobulin oder antigenerkennendes Fragment hiervon enthält, das kovalent an ein Polysaccharidderivat mit niedrigem Molekulargewicht gebunden ist, und das Verfahren gekennzeichnet ist durch die Reduktion einer auf dem Polysaccharidteil des kovalent gebundenen Immunglobulins oder antigenerkennenden Fragments hiervon vorkommenden freien Aldehydgruppe mit einem geeigneten Reduktionsmittel.

**2.** Verfahren nach Anspruch 1, worin das kovalent gebundene Immunglobulin oder antigenerkennende Fragment hiervon durch Umsetzung eines freien Immunglobulins oder Immunglobulinfragments hiervon mit einem oxidierten Polysaccharid niedrigen Molekulargewichts und anschließende Reduktion des entstehenden Konjugats einer Schiffschen Base mittels eines milden Reduktionsmittels hergestellt wird.

**3.** Verfahren zur Herstellung einer immunreaktiven Zusammensetzung nach einem der Ansprüche 1 oder 2, worin dieses Immunglobulin oder antigenerkennende Fragment hiervon die Immunreaktivität behält und die Lokalisierungseigenschaften zu denen von freiem Immunglobulin oder einem antigenbindenden Fragment hiervon im wesentlichen unverändert sind.

**4.** Verfahren zur Herstellung einer immunreaktiven Zusammensetzung nach einem der Ansprüche 1 bis 3, worin das kovalent gebundene Immunglobulin oder Fragment eine im wesentlichen unveränderte oder verbesserte Pharmakokinetik aufweist.

**5.** Verfahren zur Herstellung einer immunreaktiven Zusammensetzung nach einem der Ansprüche 1, 3 oder 4, worin das Polysaccharidderivat ein oxidiertes Dextran mit niedrigem Molekulargewicht ist.

**6.** Verfahren zur Herstellung einer immunreaktiven Zusammensetzung nach Anspruch 5, worin das durchschnittliche Molekulargewicht des oxidierten Dextrans etwa 3 000 bis etwa 16 000 beträgt.

**7.** Verfahren zur Herstellung einer immunreaktiven Zusammensetzung nach Anspruch 6, worin das durchschnittliche Molekulargewicht des oxidierten Dextrans etwa 6 000 bis etwa 16 000 beträgt.

**8.** Verfahren zur Herstellung einer immunreaktiven Zusammensetzung nach Anspruch 7, worin das durchschnittliche Molekulargewicht des oxidierten Dextrans etwa 6 000 beträgt.

**9.** Verfahren zur Herstellung einer immunreaktiven Zusammensetzung nach einem der Ansprüche 5 bis 8, die im Durchschnitt etwa ein bis etwa zwanzig oxidierte Dextranmoleküle pro Molekül Immunglobulin oder antigenerkennendem Fragment hiervon enthält.

**10.** Verfahren zur Herstellung einer immunreaktiven Zusammensetzung nach Anspruch 9, worin das Immunglobulin oder antigenerkennende Fragment hiervon ein polyklonaler oder monoklonaler Antikörper oder ein antigenerkennendes Fragment hiervon ist.

**11.** Verfahren zur Herstellung einer immunreaktiven Zusammensetzung nach Anspruch 10, worin das Immunglobulin oder Fragment hiervon an ein Arzneimittel oder einen Metallchelatbildner gebunden ist.

**12.** Verfahren zur Herstellung einer immunreaktiven Zusammensetzung nach Anspruch 11, worin das Arzneimittel Adriamycin, Methotrexat, Vinblastin oder Desacetylvinblastin oder Cisplatin ist.

**13.** Verfahren zur Herstellung einer immunreaktiven Zusammensetzung nach einem der Ansprüche 1 bis 9, worin das Immunglobulin oder antigenerkennende Fragment hiervon ein monoklonaler Antikörper oder ein antigenerkennendes Fragment hiervon ist.

**14.** Verfahren zur Herstellung einer immunreaktiven Zusammensetzung nach einem der Ansprüche 1 bis 13, worin das Immunglobulin oder antigenerkennende Fragment hiervon ein tumorassoziiertes Antigen erkennt.

**15.** Verfahren zur Herstellung einer immunreaktiven Zusammensetzung nach einem der Ansprüche 1 bis 14, worin das Immunglobulin oder antigenerkennende Fragment hiervon T101, KS1/4, 9.2.27, RAF, ZCE025 oder ein antigenerkennendes Fragment hiervon ist.

**16.** Verfahren zur Herstellung einer immunreaktiven Zusammensetzung nach einem der Ansprüche 1 bis 15, worin das Immunglobulin oder Fragment hiervon ein bifunktioneller Antikörper oder ein Fragment

hiervon ist.

**17.** Verfahren zur Herstellung einer immunreaktiven Zusammensetzung nach einem der Ansprüche 1 bis 15, worin das Immunglobulin oder Immunglobulinfragment hiervon ein chimärer Antikörper oder ein Fragment hiervon ist.

**18.** Verfahren zur Herstellung einer immunreaktiven Zusammensetzung nach Anspruch 16, worin der bifunktionelle Antikörper oder das Fragment hiervon ein synthetischer bifunktioneller Antikörper oder ein Fragment hiervon ist.

**19.** Verfahren zur Herstellung einer immunreaktiven Zusammensetzung nach Anspruch 18 worin der synthetische bifunktionelle Antikörper oder das Fragment hiervon ein synthetisch kovalent quervernetzter Antikörper oder ein Fragment hiervon ist.

**20.** Verfahren zur Herstellung einer immunreaktiven Zusammensetzung nach Anspruch 17, worin der chimäre Antikörper oder das Fragment hiervon ein humanisierter Antikörper oder ein Fragment hiervon ist.

**21.** Verfahren zur Herstellung einer immunreaktiven Zusammensetzung nach Anspruch 17, worin der chimäre Antikörper ein chimärer bifunktioneller Antikörper oder ein Fragment hiervon ist.

**22.** Verfahren zur Herstellung einer immunreaktiven Zusammensetzung nach Anspruch 21, worin der chimäre bifunktionelle Antikörper ein synthetisch kovalent quervernetzter chimärer bifunktioneller Antikörper oder ein Fragment hiervon ist.

**23.** Verfahren zur Herstellung einer immunreaktiven Zusammensetzung nach Anspruch 21, worin der chimäre bifunktionelle Antikörper oder das Fragment hiervon humanisiert ist.

**24.** Verfahren zur Herstellung einer immunreaktiven Zusammensetzung nach Anspruch 22, worin der synthetische Antikörper oder das Fragment hiervon humanisiert ist.

**25.** Verfahren zur Herstellung einer immunreaktiven Zusammensetzung nach einem der Ansprüche 1 bis 24, worin das kovalent gebundene Immunglobulin oder Fragment hiervon reduzierte Nierenaufnahmeeigenschaften aufweist.

**26.** Verfahren zur Herstellung einer immunreaktiven Zusammensetzung nach Anspruch 25, worin das Immunglobulin oder ein Fragment hiervon eine erhöhte Plasmahalbwertszeit aufweist.

**27.** Verfahren nach einem der Ansprüche 1 bis 26, worin das Reduktionsmittel für die freie Aldehydgruppe Natriumborhydrid ist und die Reaktion bei etwa 0 °C bis etwa 25 °C ausgeführt wird.

**28.** Verfahren zur Herstellung einer immundiagnostischen oder immuntherapeutischen Zusammensetzung, gekennzeichnet durch Mischen einer immunreaktiven Zusammensetzung nach einem der Ansprüche 1 bis 26 mit einem pharmazeutisch annehmbaren Träger, Verdünnungsmittel oder Hilfsstoff hierfür.

**Revendications**
**Revendications pour les Etats contractants suivants : AT, BE, CH, DE, FR, GB, IT, LI, LU, NL, SE**

**1.** Composition immunoréactive essentiellement monomère avec un potentiel immunogénique diminué, ladite composition comprenant une immunoglobuline ou un fragment reconnaissant un antigène de celle-ci, liée par covalence à un dérivé de polysaccharide de faible poids moléculaire.

**2.** Composition immunoréactive selon la revendication 1, dans laquelle ladite immunoglobuline ou ledit fragment reconnaissant un antigène de celle-ci conserve son immunoréactivité et ses caractéristiques de localisation d'une manière essentiellement non altérée par rapport à l'immunoglobuline libre ou le fragment reconnaissant un antigène de celle-ci.

3. Composition immunoréactive selon la revendication 1 ou 2, dans laquelle l'immunoglobuline à liaison covalente ou son fragment possède des caractéristiques pharmacocinétiques essentiellement non altérées ou améliorées.

4. Composition immunoréactive selon la revendication 3, dans laquelle le dérivé de polysaccharide de faible poids moléculaire est un polysaccharide oxydé.

5. Composition immunoréactive selon la revendication 4, dans laquelle le polysaccharide oxydé est un dextrane oxydé de faible poids moléculaire.

6. Composition immunoréactive selon la revendication 5, dans laquelle le poids moléculaire moyen du dextrane oxydé est compris entre environ 3000 et environ 16.000.

7. Composition immunoréactive selon la revendication 6, dans laquelle le poids moléculaire moyen du dextrane oxydé est compris entre environ 6000 et environ 16.000.

8. Composition immunoréactive selon la revendication 7, dans laquelle le poids moléculaire moyen du dextrane oxydé est égal à environ 6.000.

9. Composition immunoréactive selon l'une quelconque des revendications 5 à 8, comprenant en moyenne environ 1 à environ 20 molécules de dextrane oxydé par molécule d'immunoglobuline ou de fragment de celle-ci reconnaissant un antigène.

10. Composition immunoréactive selon la revendication 9, dans laquelle l'immunoglobuline ou le fragment de celle-ci, reconnaissant un antigène est un anticorps polyclonal ou monoclonal ou un fragment de celui-ci reconnaissant un antigène.

11. Composition immunoréactive selon la revendication 10, dans laquelle l'immunoglobuline ou le fragment de celle-ci, est fixée à un médicament ou à un agent chélatant les métaux.

12. Composition immunoréactive selon la revendication 11, dans laquelle le médicament est l'adriamycine , le méthotrexate, la vinblastine ou la désacétylvinblastine ou la cisplatine.

13. Composition immunoréactive selon la revendication 10, dans laquelle l'immunoglobuline ou le fragment de celle-ci, reconnaissant un antigène est un anticorps monoclonal ou un fragment de celui-ci reconnaissant un antigène.

14. Composition immunoréactive selon l'une quelconque des revendications 1 à 13, dans laquelle l'immunoglobuline,ou le fragment de celle-ci reconnaissant un antigène reconnaît un antigène associé à une tumeur.

15. Composition immunoréactive selon l'une quelconque des revendications 1 à 14, dans laquelle l'immunoglobuline, ou le fragment de celle-ci, reconnaissant un antigène est T101, KS1/4, 9.2.27, RAF, ZCE025, ou un fragment de celui-ci, reconnaissant un antigène.

16. Formulation pour application immuno-diagnostique ou immunothérapeutique comprenant comme ingrédient actif une composition immunoréactive selon l'une quelconque des revendications 1 à 15, 20 à 30 ou 32 à 40 associée à un support, un diluant ou un excipient pharmaceutiquement acceptable pour celle-ci.

17. Composition immunoréactive selon l'une quelconque des revendications 1 à 15 ou 20 à 28, à utiliser en immunothérapie.

18. Procédé de préparation d'une composition immunoréactive selon l'une quelconque des revendications 1 à 15, qui comprend la réduction par un agent réducteur approprié d'un groupe aldéhyde libre présent sur la fraction de polysaccharide de l'immunoglobuline ou d'un fragment de celle-ci, reconnaissant un antigène, lié(e) par covalence, ladite immunoglobuline ou le fragment de celle-ci, reconnaissant un antigène, ainsi lié(e) ayant été préparés en faisant réagir une immunoglobuline libre, ou un fragment

d'immunoglobuline avec un polysaccharide oxydé et en réduisant ensuite les adducts des bases de Schiff ainsi obtenus par un agent faiblement réducteur.

**19.** Procédé selon la revendication 30, dans lequel l'agent réducteur approprié est le borohydrure de sodium.

**20.** Composition immunoréactive selon l'une quelconque des revendications 1 à 15, dans laquelle l'immunoglobuline, ou le fragment de celle-ci, est un anticorps bifonctionnel ou un fragment de celui-ci.

**21.** Composition immunoréactive selon l'une quelconque des revendications 1 à 15, dans laquelle l'immunoglobuline, ou le fragment immunoglobuline de celle-ci, est un anticorps chimérique ou un fragment de celui-ci.

**22.** Composition immunoréactive selon la revendication 20, dans laquelle l'anticorps bifonctionnel ou le fragment de celui-ci est un anticorps bifonctionnel synthétique ou un fragment de celui-ci.

**23.** Composition immunoréactive selon la revendication 22, dans laquelle l'anticorps bifonctionnel synthétique, ou le fragment de celui-ci, est un anticorps réticulé par covalence synthétique ou un fragment de celui-ci.

**24.** Composition immunoréactive selon la revendication 21, dans laquelle l'anticorps chimérique, ou le fragment de celui-ci, est un anticorps humanisé ou un fragment de celui-ci.

**25.** Composition immunoréactive selon la revendication 21, dans laquelle l'anticorps chimérique est un anticorps bifonctionnel chimérique ou un fragment de celui-ci.

**26.** Composition immunoréactive selon la revendication 25, dans laquelle l'anticorps bifonctionnel chimérique est un anticorps synthétique bifonctionnel chimérique réticulé par covalence ou un fragment de celui-ci.

**27.** Composition immunoréactive selon la revendication 25, dans laquelle l'anticorps bifonctionnel chimérique, ou le fragment de celui-ci, est humanisé.

**28.** Composition immunoréactive selon la revendication 26, dans laquelle l'anticorps synthétique, ou le fragment de celui-ci, est humanisé.

**29.** Composition immunoréactive selon l'une quelconque des revendications 1 à 15 ou selon les revendications 20 à 28, dans laquelle l'immunoglobuline à liaison covalente, ou le fragment de celle-ci, présente des caractéristiques d'absorption réduites par les reins.

**30.** Composition immunoréactive selon la revendication 29, dans laquelle l'immunoglobuline, ou le fragment de celle-ci,a une demi-vie accrue dans le plasma.

**31.** Méthode d'amélioration des caractéristiques pharmacocinétiques d'une immunoglobuline, ou d'un fragment de celle-ci, reconnaissant un antigène,qui comprend la liaison covalente d'une immunoglobuline ou d'un fragment de celle-ci reconnaissant un antigène à un dérivé de polysaccharide de faible poids moléculaire.

**32.** Composition immunoréactive essentiellement monomère présentant des caractéristiques pharmacocinétiques améliorées, ladite composition comprenant une immunoglobuline, ou un fragment de celle-ci, reconnaissant un antigène lié par covalence à un dérivé de polysaccharide de faible poids moléculaire.

**33.** Composition selon la revendication 32, dans laquelle ladite immunoglobuline, ou le fragment de celle-ci, reconnaissant un antigène est un anticorps bifonctionnel ou un fragment de celui-ci.

**34.** Composition selon la revendication 32, comprenant un anticorps chimérique ou un fragment de celui-ci.

**35.** Composition selon la revendication 34, qui est un anticorps bi-fonctionnel chimérique ou un fragment de celui-ci.

**36.** Composition selon la revendication 34 qui est humanisée.

**37.** Composition selon la revendication 35 qui est humanisée.

**38.** Composition selon la revendication 32, qui présente des caractéristiques d'absorption réduites par les reins.

**39.** Composition selon la revendication 32, qui a une demi-vie accrue dans le plasma.

**40.** Composition selon la revendication 38, qui a une demi-vie accrue dans le plasma.

**Revendications pour les Etats contractants suivants : ES, GR**

**1.** Procédé de préparation d'une composition immunoréactive essentiellement monomère à potentiel immunogénique diminué, ladite composition comprenant une immunoglobuline ou un fragment de celle-ci reconnaissant un antigène, lié(e) par covalence à un dérivé de polysaccharide de faible poids moléculaire, ledit procédé comprenant la réduction par un agent réducteur approprié d'un groupe aldéhyde libre présent dans la fraction de polysaccharide de l'immunoglobuline, ou du fragment de celle-ci, reconnaissant un antigène, lié(e) par covalence.

**2.** Procédé selon la revendication 1, dans lequel ladite immunoglobuline, ou le fragment de celle-ci, reconnaissant un antigène, lié(e) par covalence est préparée en faisant réagir une immunoglobuline libre, ou un fragment de celle-ci, avec un polysaccharide oxydé de faible poids moléculaire, puis en réduisant par un agent faiblement réducteur l'adduct de base de Schiff ainsi obtenu.

**3.** Procédé de préparation d'une composition immunoréactive selon la revendication 1 ou 2, dans lequel ladite immunoglobuline, ou le fragment de celle-ci reconnaissant un antigène conserve son immunoréactivité et ses propriétés de localisation de manière essentiellement non altérée par rapport à l'immunoglobuline libre, ou au fragment de celle-ci reconnaissant un antigène.

**4.** Procédé de préparation d'une composition immunoréactive selon l'une quelconque des revendications 1 à 3, dans lequel l'immunoglobuline ou le fragment de celle-ci, à liaison covalente, possède des caractéristiques pharmacocinétiques essentiellement non altérées ou améliorées.

**5.** Procédé de préparation d'une composition immunoréactive selon la revendication 1, 3 ou 4, dans lequel le dérivé polysaccharide est un dextrane oxydé de faible poids moléculaire.

**6.** Procédé de préparation d'une composition immunoréactive selon la revendication 5, dans lequel le poids moléculaire moyen du dextrane oxydé est compris entre environ 3000 et environ 16.000.

**7.** Procédé de préparation d'une composition immunoréactive selon la revendication 6, dans lequel le poids moléculaire moyen du dextrane oxydé est compris entre environ 6.000 et environ 16.000.

**8.** Procédé de préparation d'une composition immunoréactive selon la revendication 7, dans lequel le poids moléculaire moyen du dextrane oxydé est égal à environ 6.000.

**9.** Procédé de préparation d'une composition immunoréactive selon l'une quelconque des revendications 5 à 8, qui comprend en moyenne environ une à environ 20 molécules de dextrane oxydé par molécule d'immunoglobuline ou du fragment de celle-ci, reconnaissant un antigène.

**10.** Procédé de préparation d'une composition immunoréactive selon la revendication 9, dans lequel l'immunoglobuline ou le fragment de celle-ci reconnaissant un antigène est un anticorps polyclonal ou monoclonal, ou un fragment de celui-ci reconnaissant un antigène.

**11.** Procédé de préparation d'une composition immunoréactive selon la revendication 10, dans lequel l'immunoglobuline ou le fragment de celle-ci, reconnaissant un antigène est fixé(e) à un médicament ou à un agent chélatant les métaux.

**12.** Procédé de préparation d'une composition immunoréactive selon la revendication 11, dans lequel le médicament est l'adriamycine, le méthotrexate, la vinblastine, la désacétylvinblastine ou la cisplatine.

**13.** Procédé de préparation d'une composition immunoréactive selon l'une quelconque des revendications 1 à 9, dans lequel l'immunoglobuline ou le fragment de celle-ci, reconnaissant un antigène est un anticorps monoclonal ou un fragment de celui-ci, reconnaissant un antigène .

**14.** Procédé de préparation d'une composition immunoréactive selon l'une quelconque des revendications 1 à 13, dans lequel l'immunoglobuline ou le fragment de celle-ci reconnaissant un antigène reconnaît un antigène associé à une tumeur.

**15.** Procédé de préparation d'une composition immunoréactive selon l'une quelconque des revendications 1 à 14, dans lequel l'immunoglobuline ou le fragment de celle-ci, reconnaissant un antigène est T101, KS1/4, 9.2.27, RAF, ZCE025 ou un fragment de ceux-ci reconnaissant un antigène.

**16.** Procédé de préparation d'une composition immunoréactive selon l'une quelconque des revendications 1 à 15, dans lequel l'immunoglobuline ou le fragment de celle-ci est un anticorps bifonctionnel ou un fragment de celui-ci.

**17.** Procédé de préparation d'une composition immunoréactive selon l'une quelconque des revendications 1 à 15, dans lequel l'immunoglobuline ou le fragment immunoglobuline de celle-ciest un anticorps chimérique ou un fragment de celui-ci.

**18.** Procédé de préparation d'une composition immunoréactive selon la revendication 16, dans lequel l'anticorps bifonctionnel ou le fragment de celui-ci, est un anticorps bifonctionnel synthétique ou un fragment de celui-ci.

**19.** Procédé de préparation d'une composition immunoréactive selon la revendication 18, dans lequel l'anticorps bifonctionnel synthétique ou le fragment de celui-ci, est un anticorps synthétique réticulé par covalence ou un fragment de celui-ci.

**20.** Procédé de préparation d'une composition immunoréactive selon la revendication 17, dans lequel l'anticorps chimérique ou le fragment de celui-ci, est un anticorps humanisé ou un fragment de celui-ci.

**21.** Procédé de préparation d'une composition immunoréactive selon la revendication 17, dans lequel l'anticorps chimérique est un anticorps bifonctionnel chimérique ou un fragment de celui-ci.

**22.** Procédé de préparation d'une composition immunoréactive selon la revendication 21, dans lequel l'anticorps bifonctionnel chimérique est un anticorps synthétique chimérique réticulé par covalence ou un fragment de celui-ci.

**23.** Procédé de préparation d'une composition immunoréactive selon la revendication 21, dans lequel l'anticorps bifonctionnel chimérique ou le fragment de celui-ci est humanisé.

**24.** Procédé de préparation d'une composition immunoréactive selon la revendication 22, dans lequel l'anticorps synthétique ou le fragment de celui-ci est humanisé.

**25.** Procédé de préparation d'une composition immunoréactive selon l'une quelconque des revendications 1 à 24, dans lequel l'immunoglobuline à liaison covalente ou le fragment de celle-ci, a des caractéristiques d'absorption réduites par les reins.

**26.** Procédé de préparation d'une composition immunoréactive selon la revendication 25, dans lequel l'immunoglobuline ou le fragment de celle-ci, a une demi-vie accrue dans le plasma.

**27.** Procédé selon l'une quelconque des revendications 1 à 26, dans lequel l'agent réducteur pour le groupe aldéhyde libre est le borohydrure de sodium et dans lequel la réaction est effectuée entre environ 0° C et environ 25° C.

**28.** Procédé de préparation pour une composition immunodiagnostique ou immunothérapeutique qui comprend le mélange d'une composition immunoréactive telle que définie par l'une quelconque des revendications 1 à 26 à un support, un diluant ou un excipient pharmaceutiquement acceptable pour celle-ci.

FIG.1A

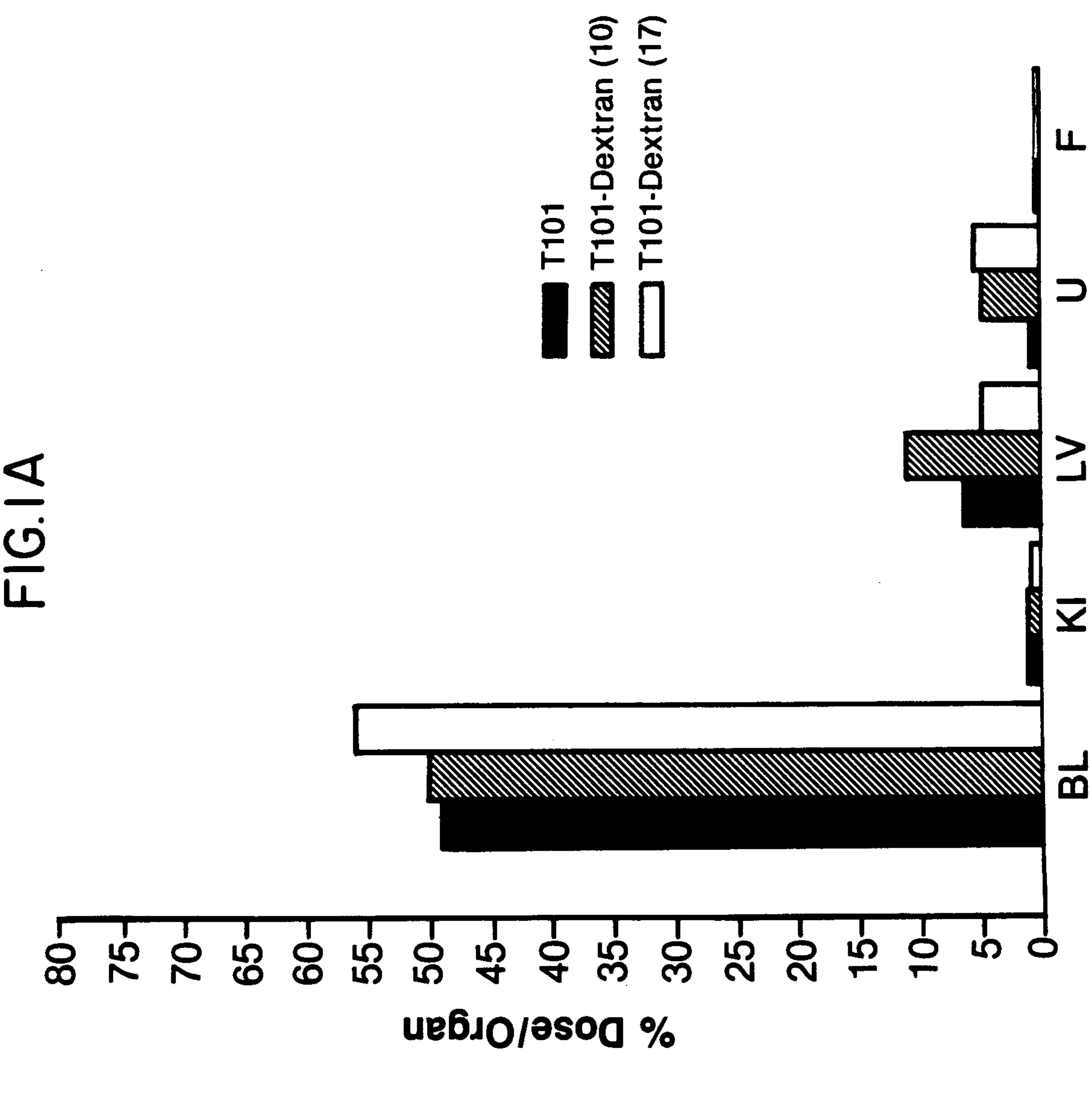
% Dose/Organ
80
75
70
65
60
55
50
45
40
35
30
25
20
15
10
5
0
BL
KI
LV
U
F
T101
T101-Dextran (10)
T101-Dextran (17)

FIG.1B

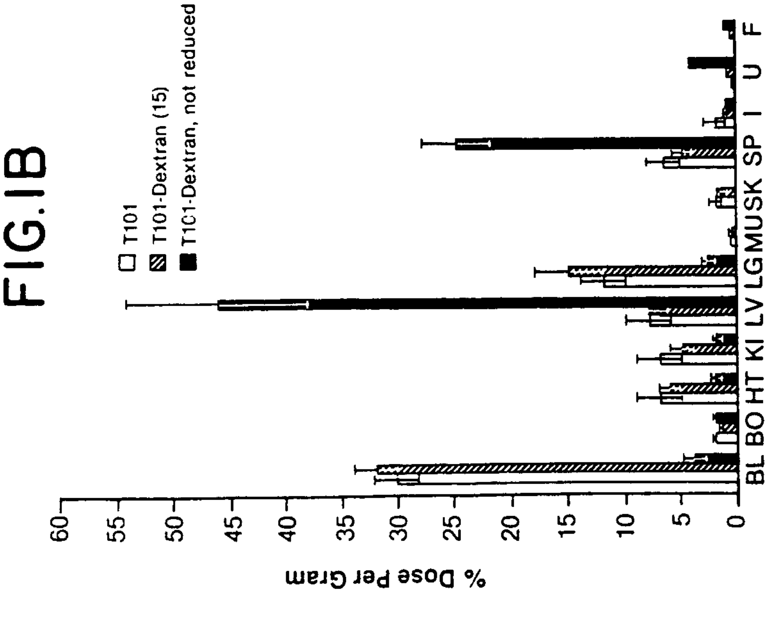


FIG.1C

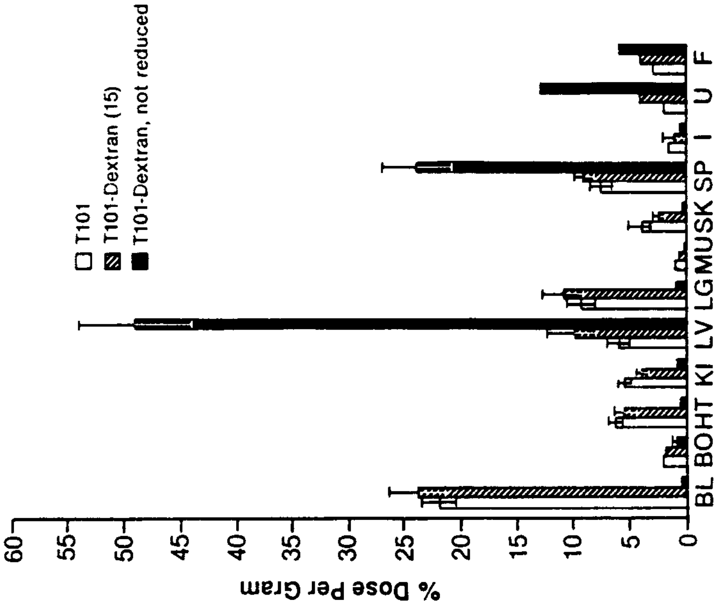


FIG.1D

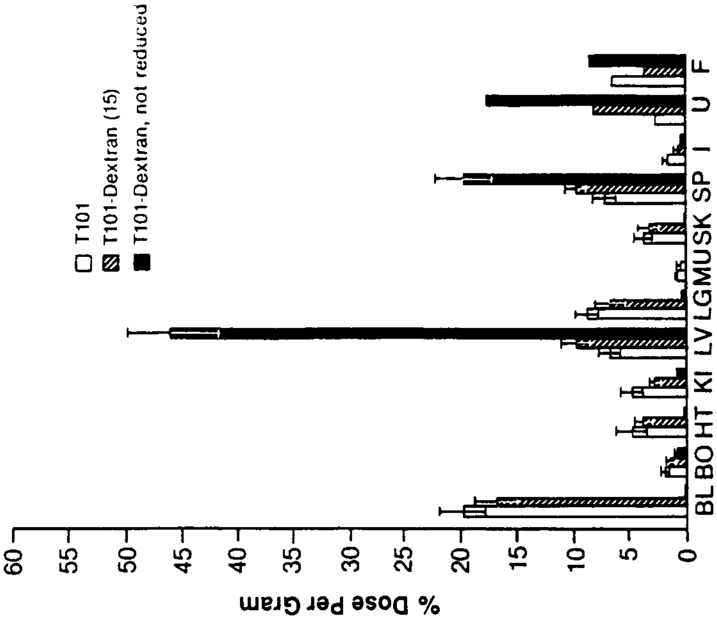

FIG. 2

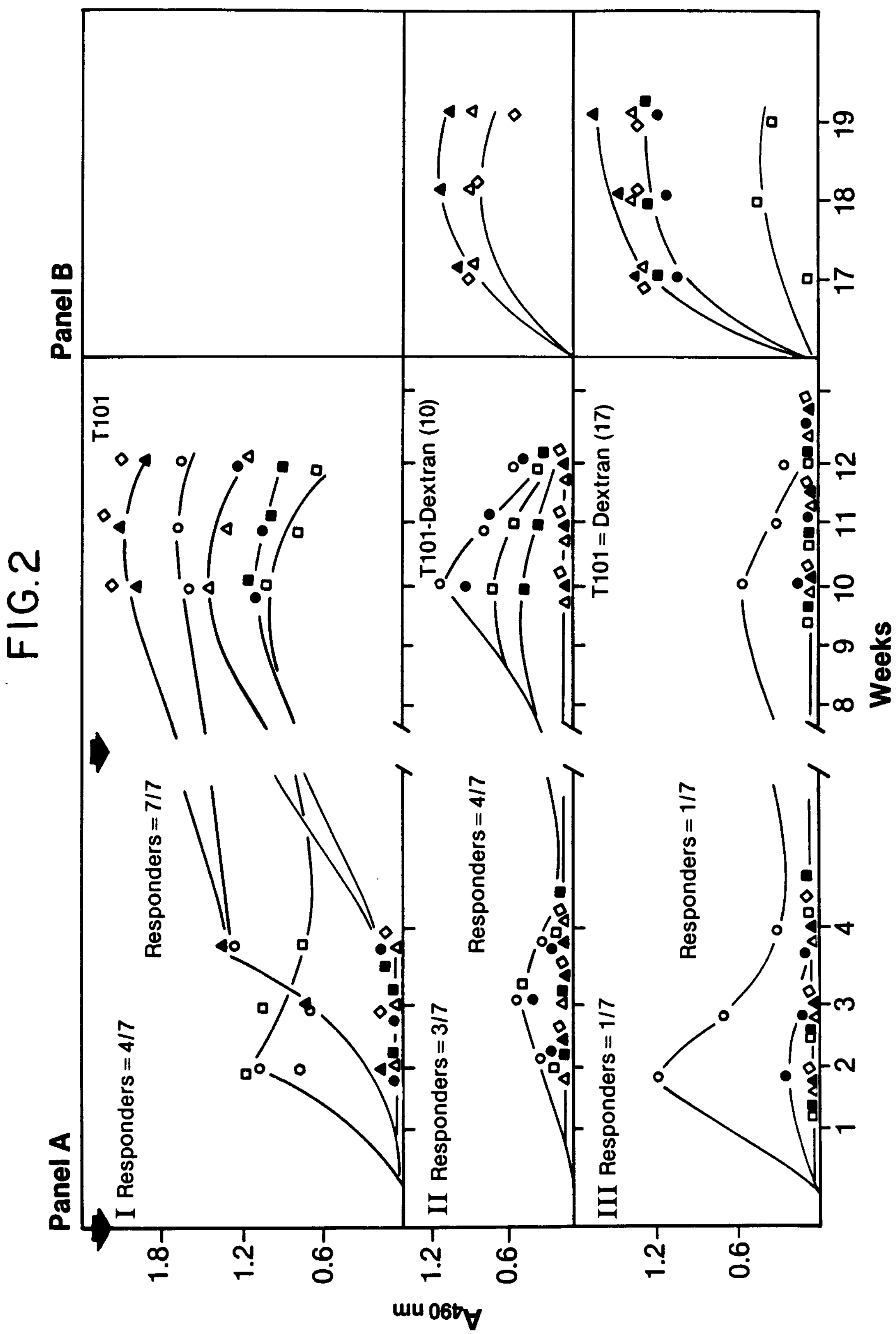
Panel A
Panel B
I Responders = 4/7
Responders = 7/7
T101
II Responders = 3/7
Responders = 4/7
T101-Dextran (10)
III Responders = 1/7
Responders = 1/7
T101 = Dextran (17)
A490 nm
1.8
1.2
0.6
1.2
0.6
1.2
0.6
1
2
3
4
8
9
10
11
12
17
18
19
Weeks

FIG.3

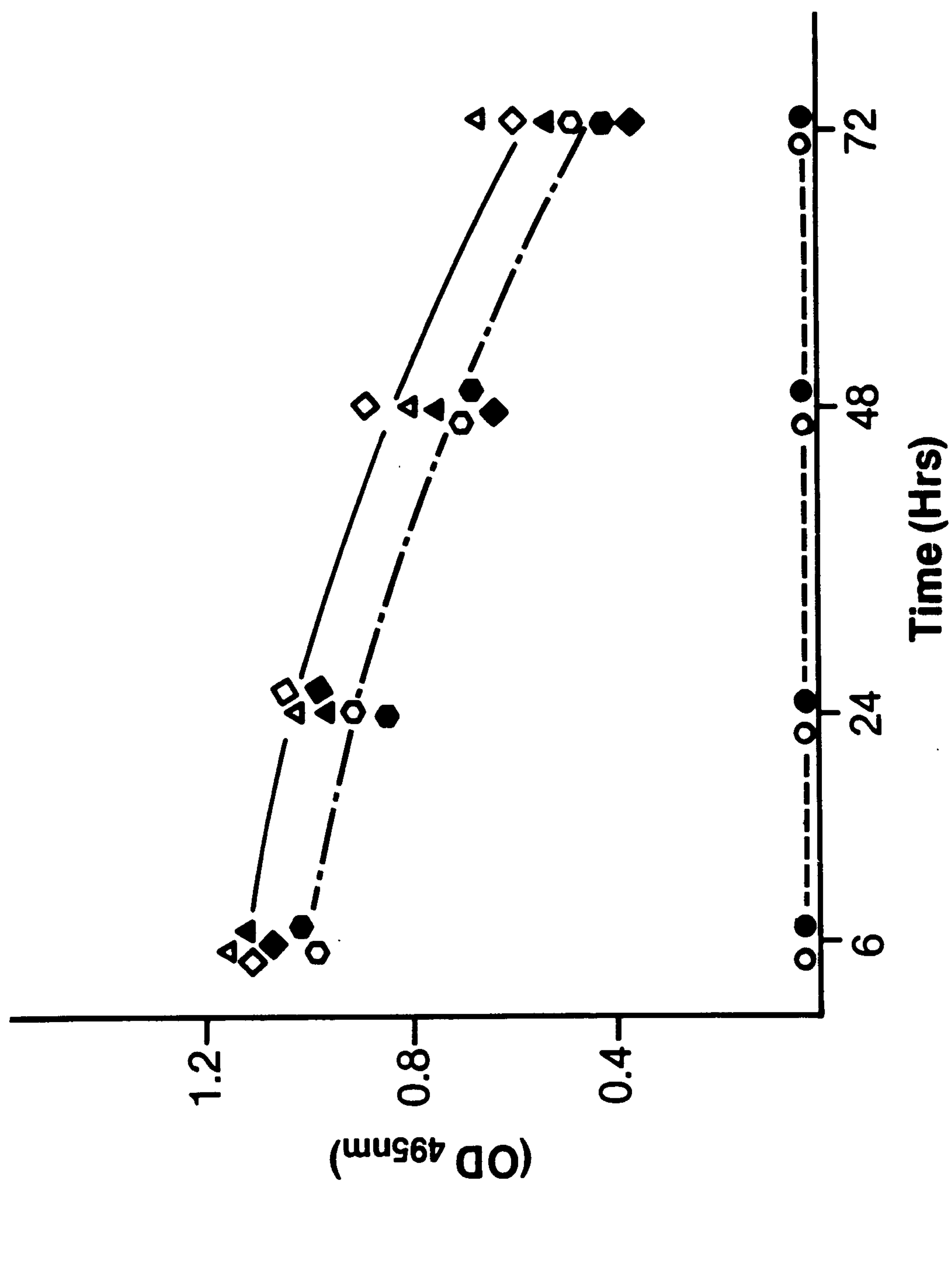

FIG.4C

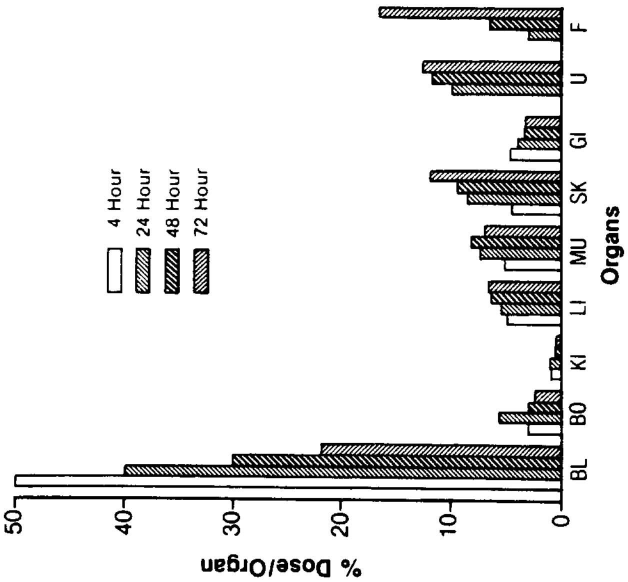


FIG.4B

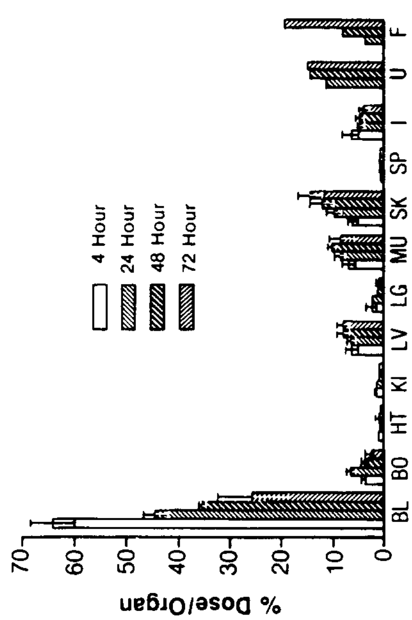


FIG.4A

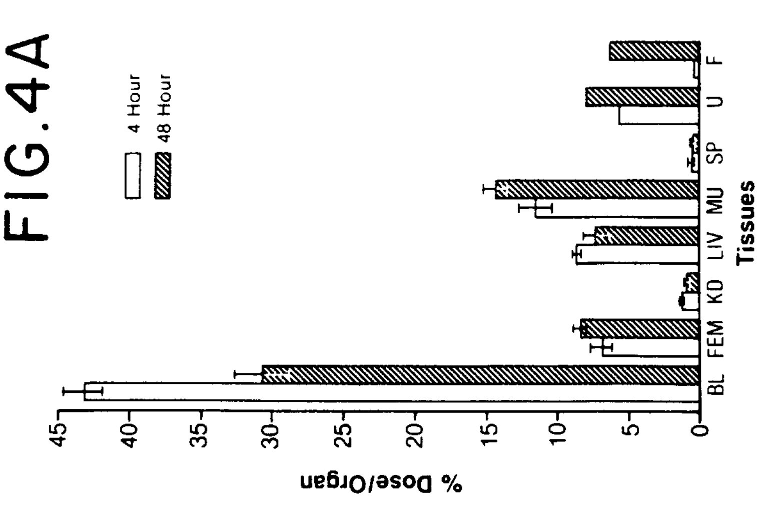

FIG.5A

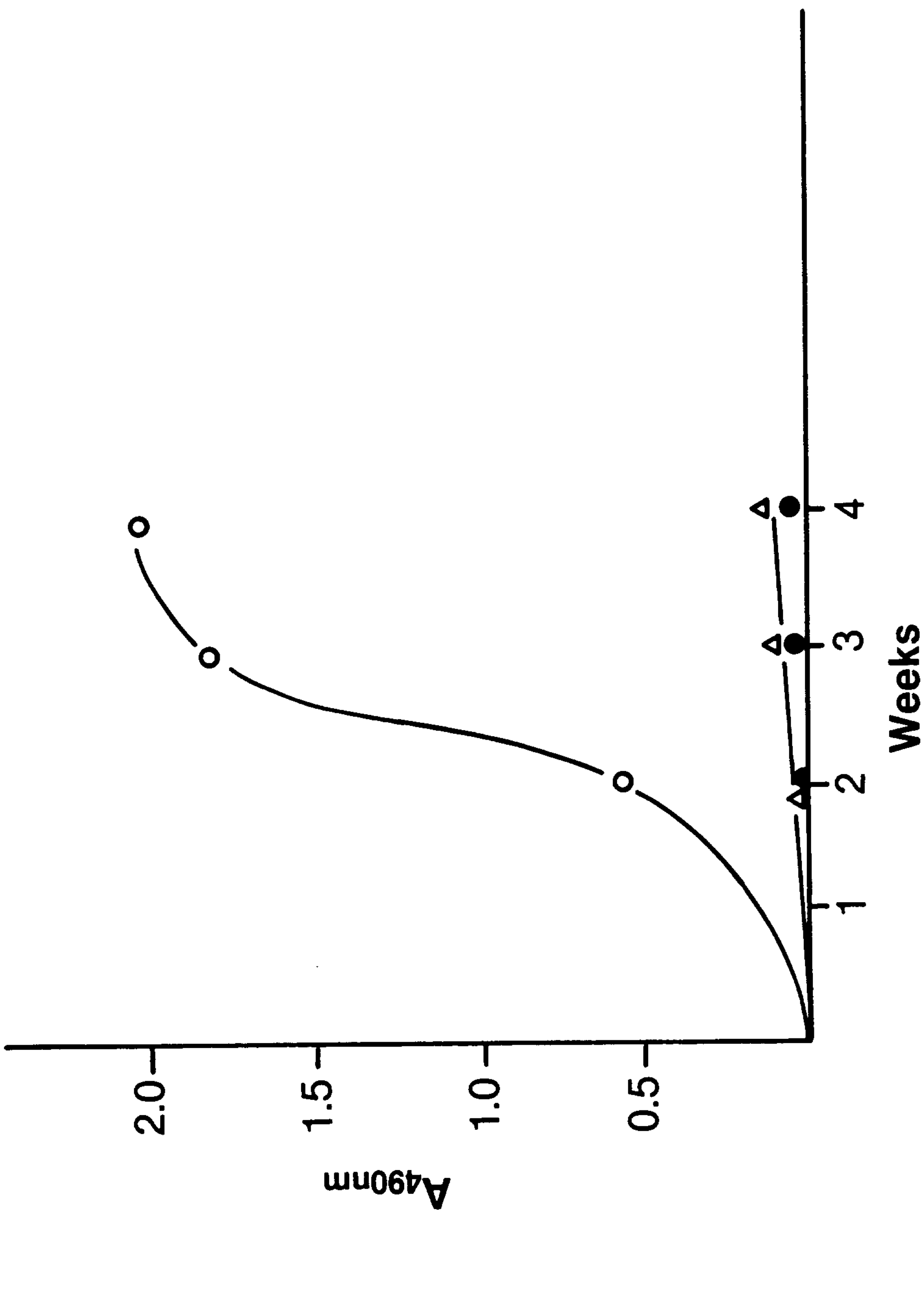

FIG.5B

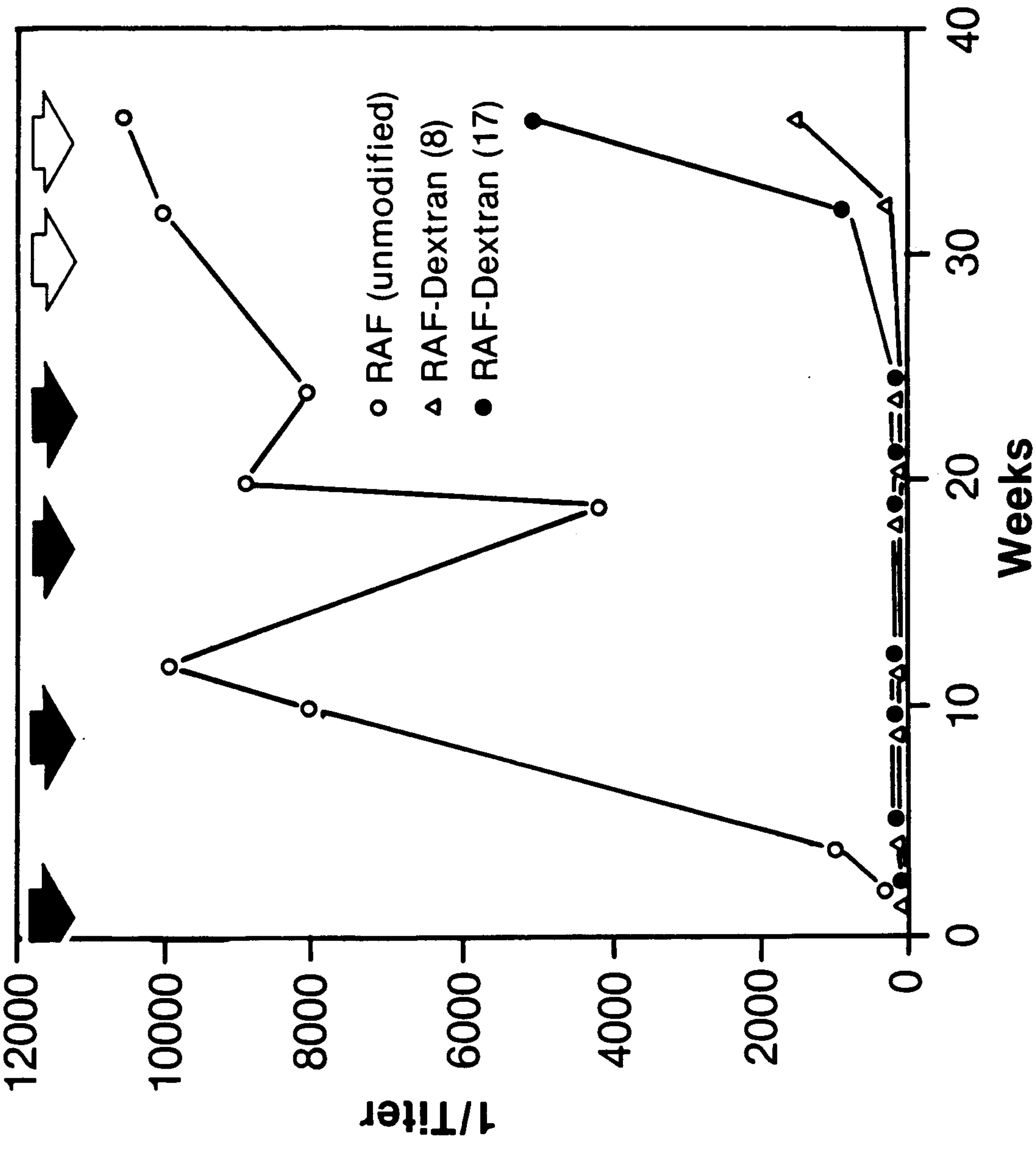
1/Titer
12000
10000
8000
6000
4000
2000
0
0
10
20
30
40
Weeks
RAF (unmodified)
RAF-Dextran (8)
RAF-Dextran (17)

FIG.6

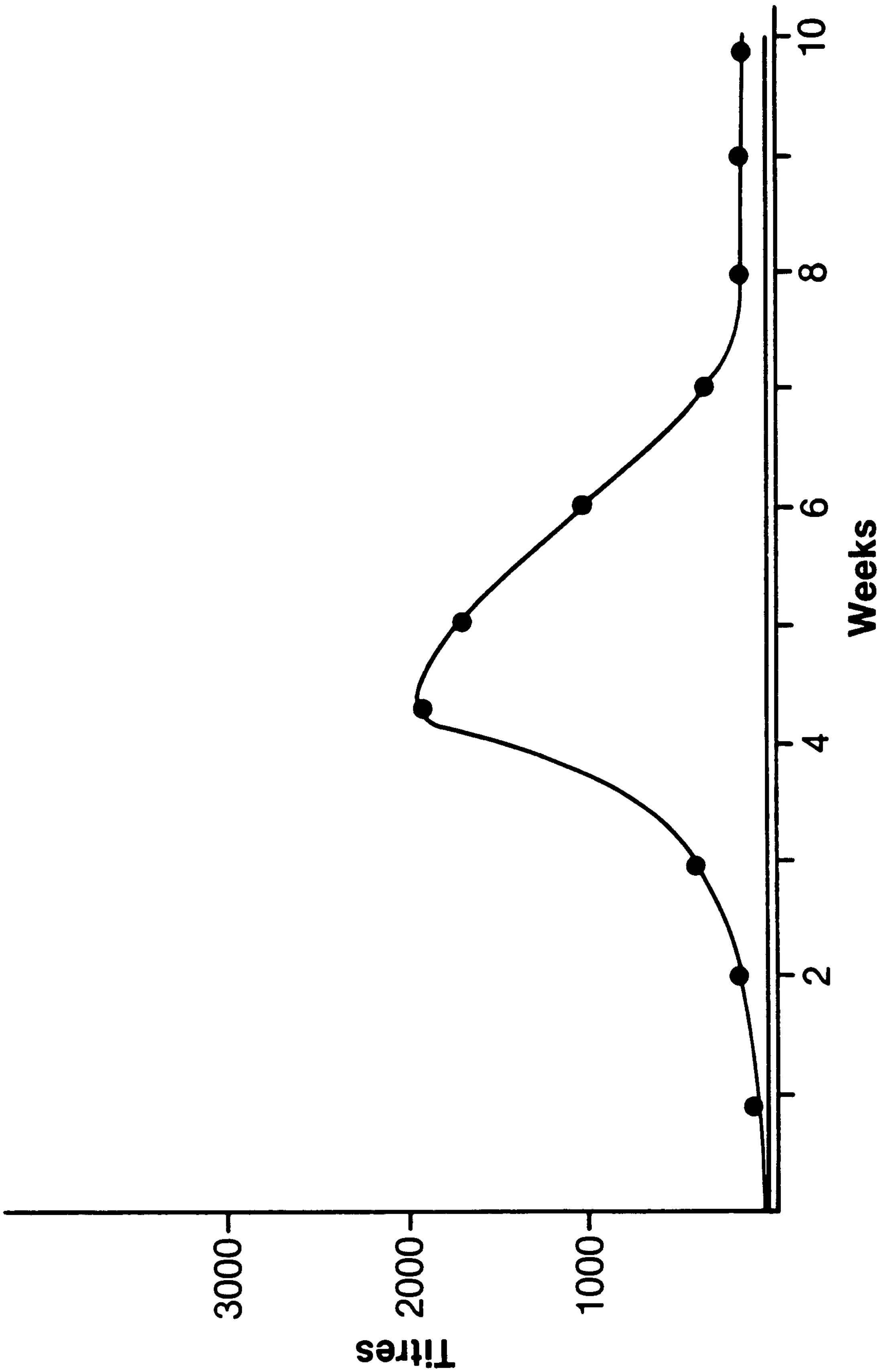

FIG.7

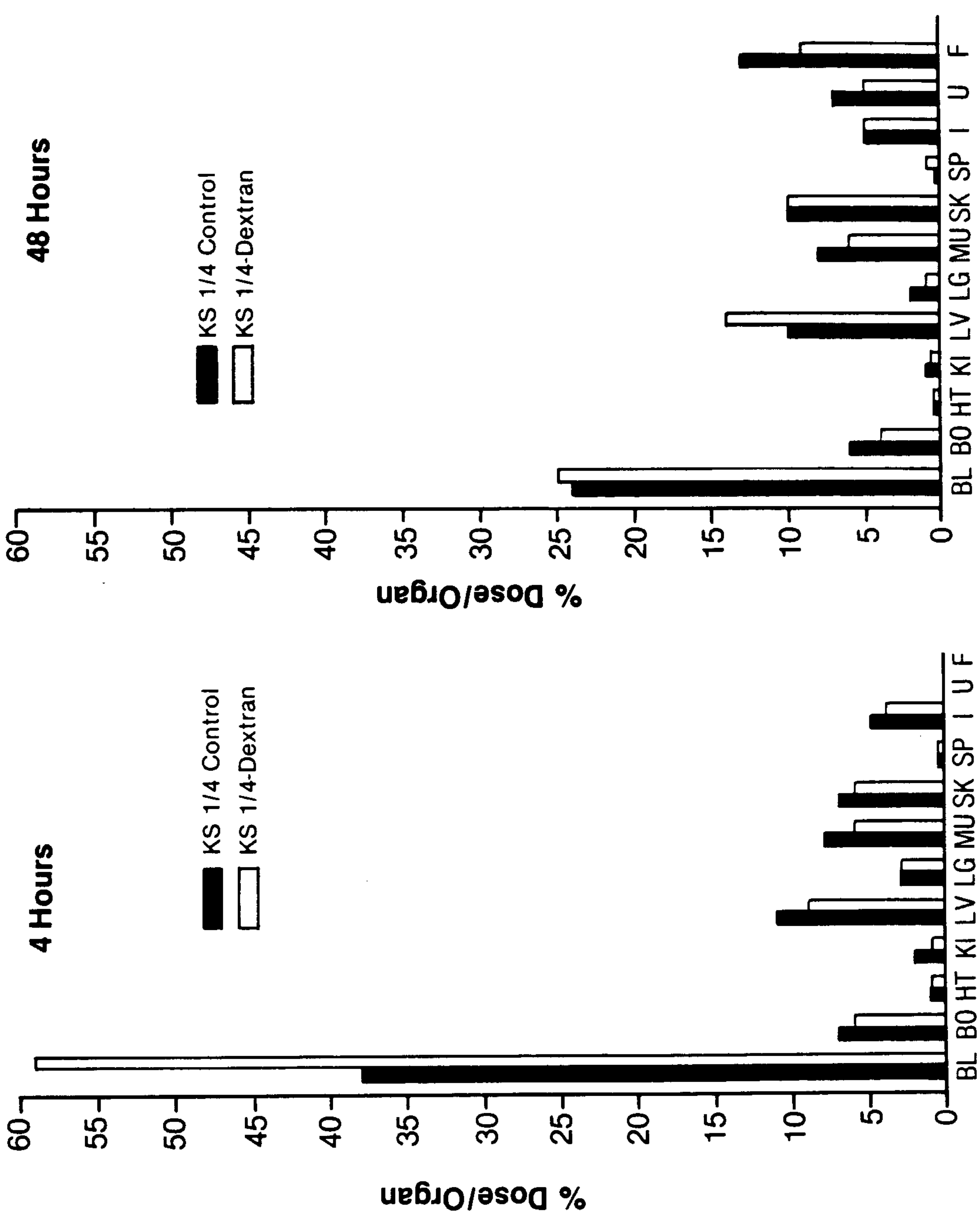

4 Hours
KS 1/4 Control
KS 1/4-Dextran
% Dose/Organ
60
55
50
45
40
35
30
25
20
15
10
5
0
BL BO HT KI LV LG MU SK SP I U F
48 Hours
KS 1/4 Control
KS 1/4-Dextran
% Dose/Organ
60
55
50
45
40
35
30
25
20
15
10
5
0
BL BO HT KI LV LG MU SK SP I U F

FIG.8

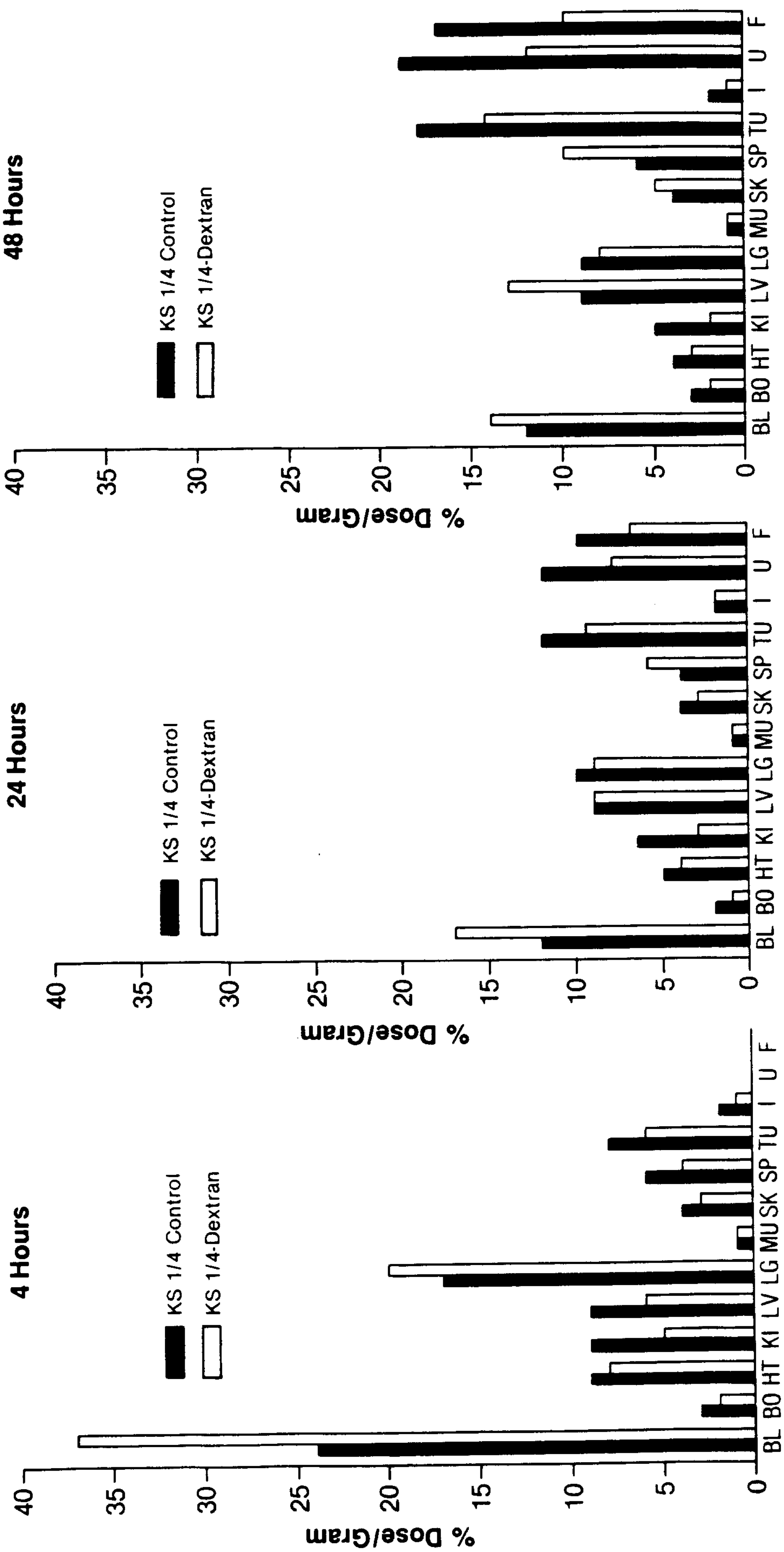
4 Hours
24 Hours
48 Hours
KS 1/4 Control
KS 1/4-Dextran
% Dose/Gram
0
5
10
15
20
25
30
35
40
BL BO HT KI LV LG MU SK SP TU I U F

FIG.9A

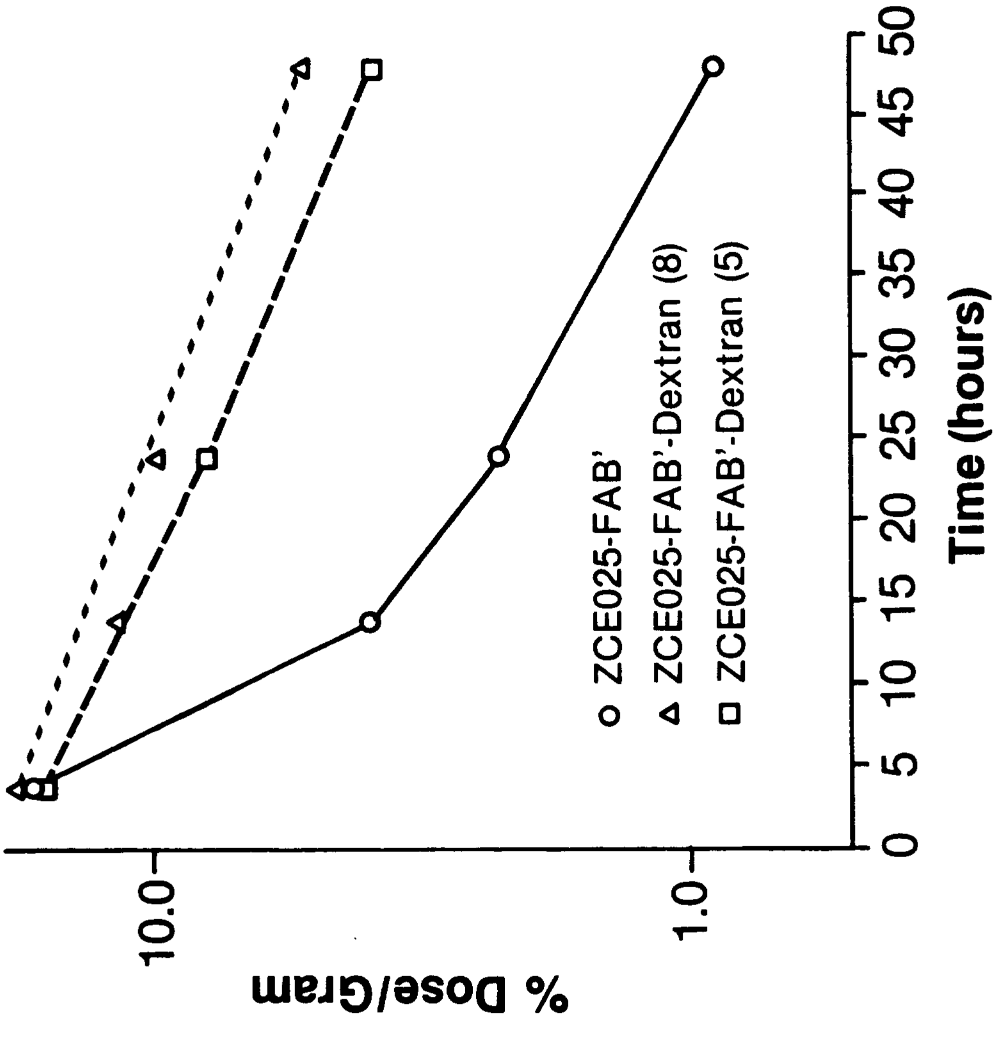

ZCE025-FAB'
ZCE025-FAB'-Dextran (8)
ZCE025-FAB'-Dextran (5)
% Dose/Gram
10.0
1.0
0
5
10
15
20
25
30
35
40
45
50
Time (hours)

FIG.9B

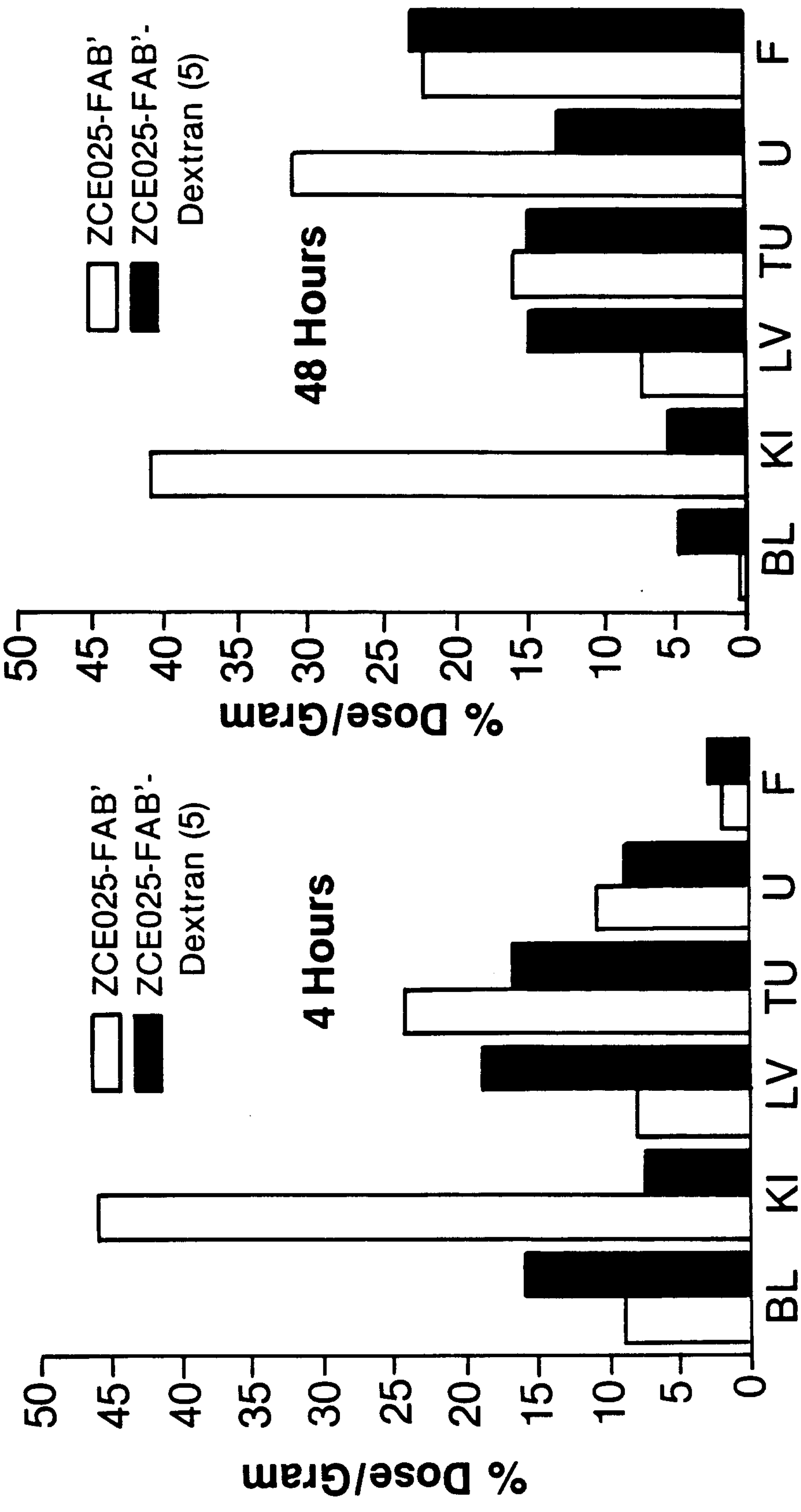
ZCE025-FAB'
ZCE025-FAB'-Dextran (5)
48 Hours
% Dose/Gram
50
45
40
35
30
25
20
15
10
5
0
BL
KI
LV
TU
U
F
ZCE025-FAB'
ZCE025-FAB'-Dextran (5)
4 Hours
% Dose/Gram
50
45
40
35
30
25
20
15
10
5
0
BL
KI
LV
TU
U
F

FIG.10A

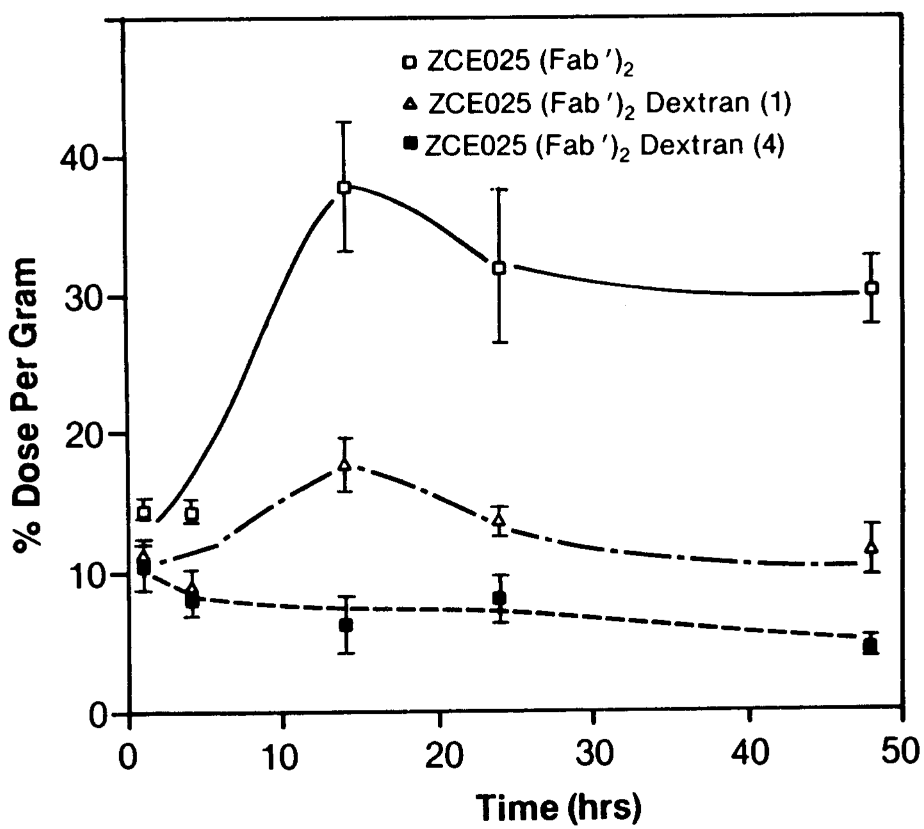


FIG.10B

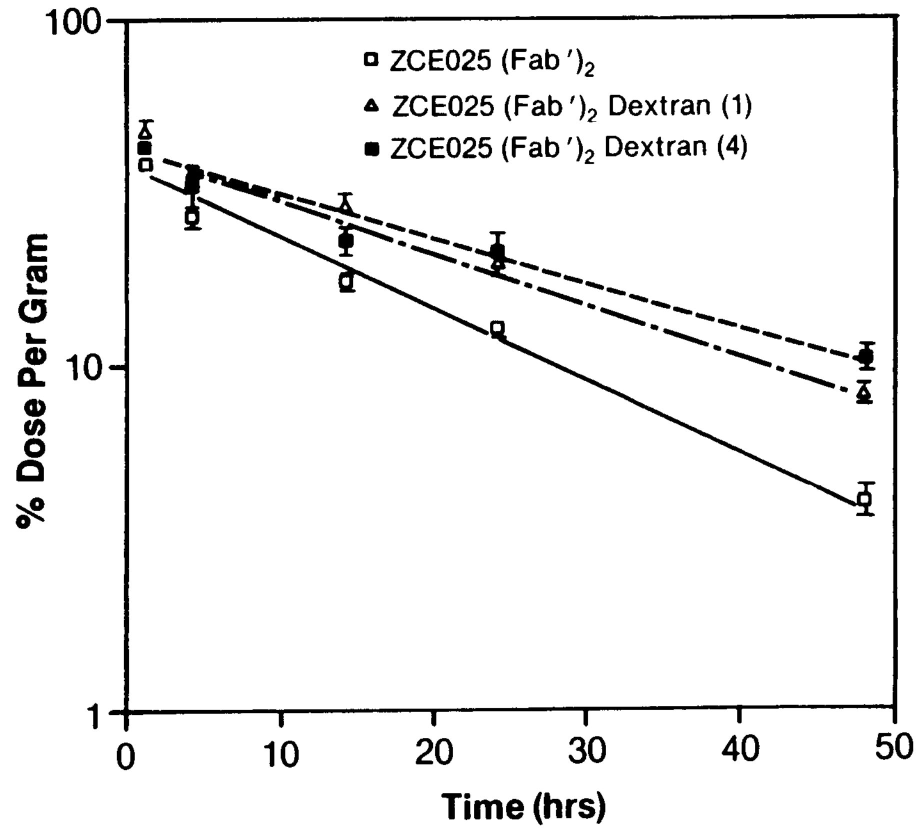

FIG.11

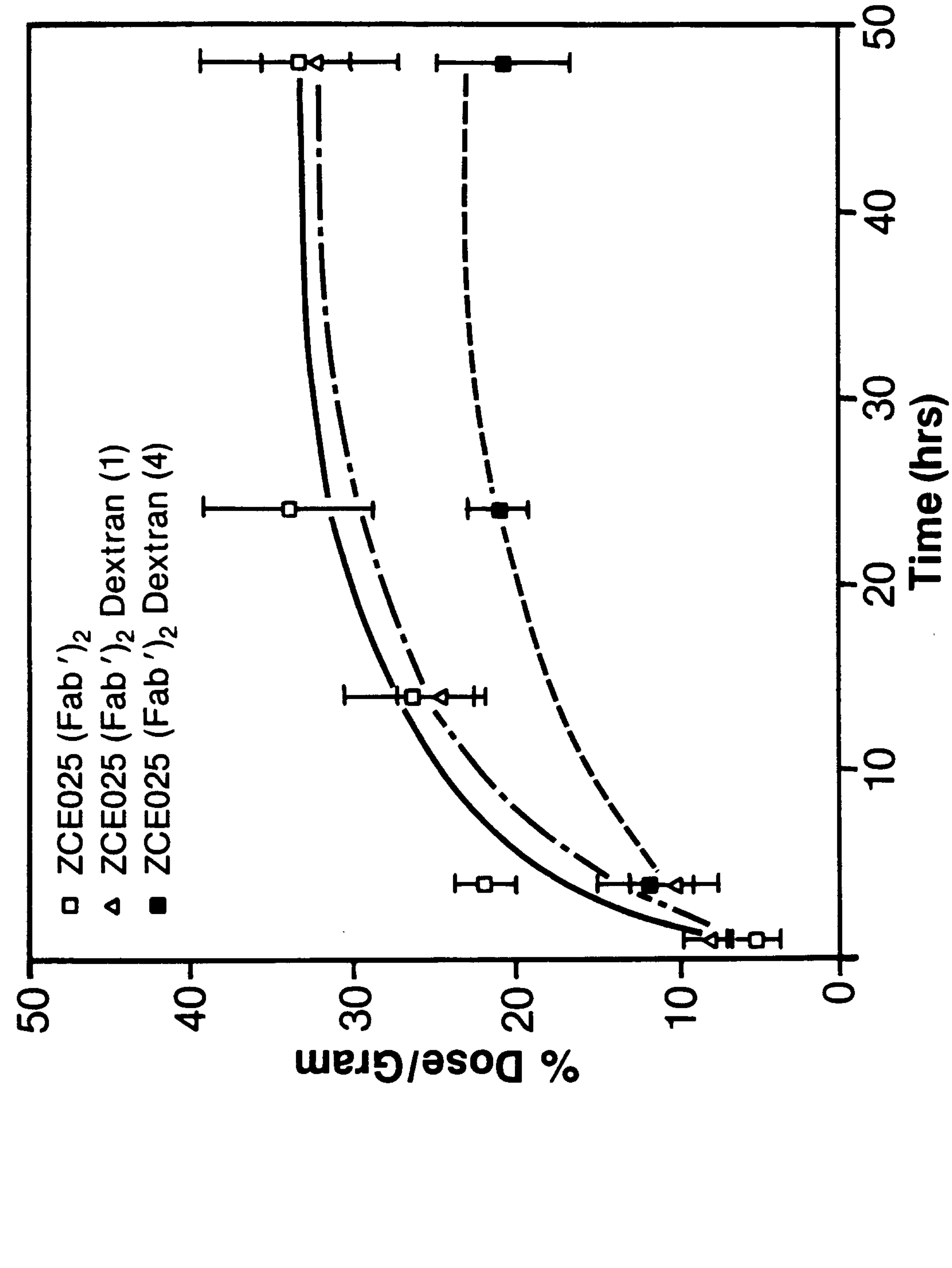
ZCE025 (Fab ')2
ZCE025 (Fab ')2 Dextran (1)
ZCE025 (Fab ')2 Dextran (4)
% Dose/Gram
0
10
20
30
40
50
Time (hrs)
0
10
20
30
40
50

FIG.12A

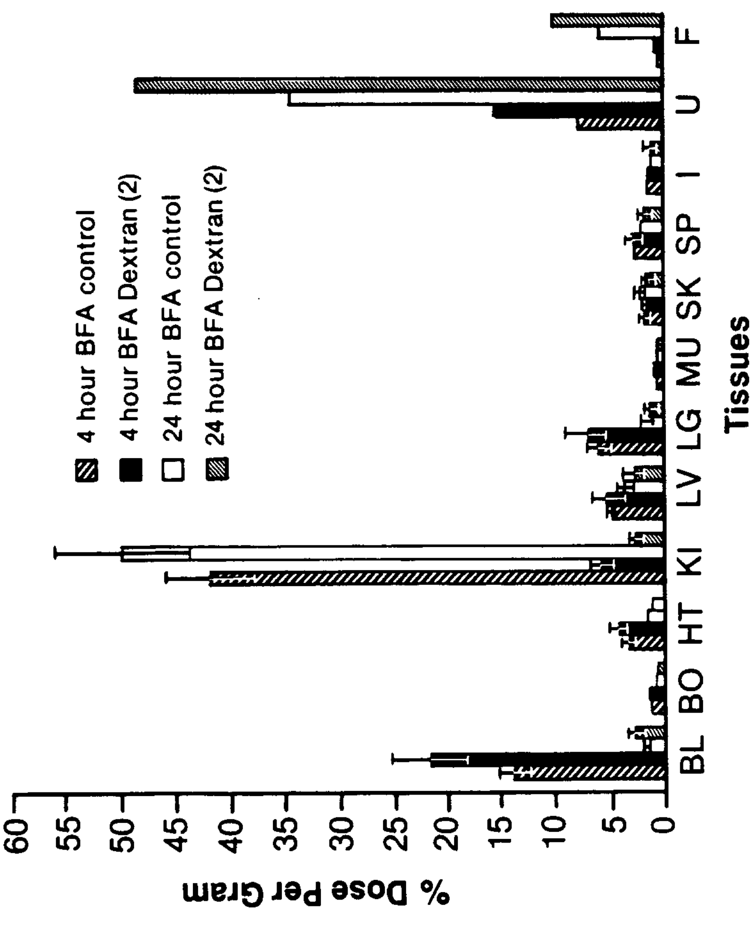


FIG.12B

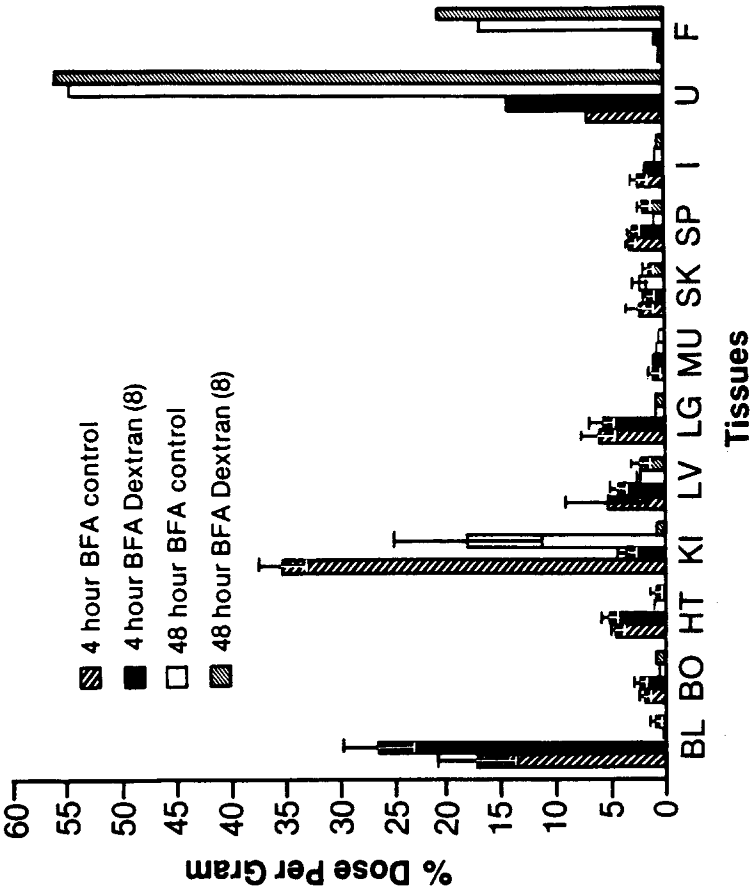

FIG.13

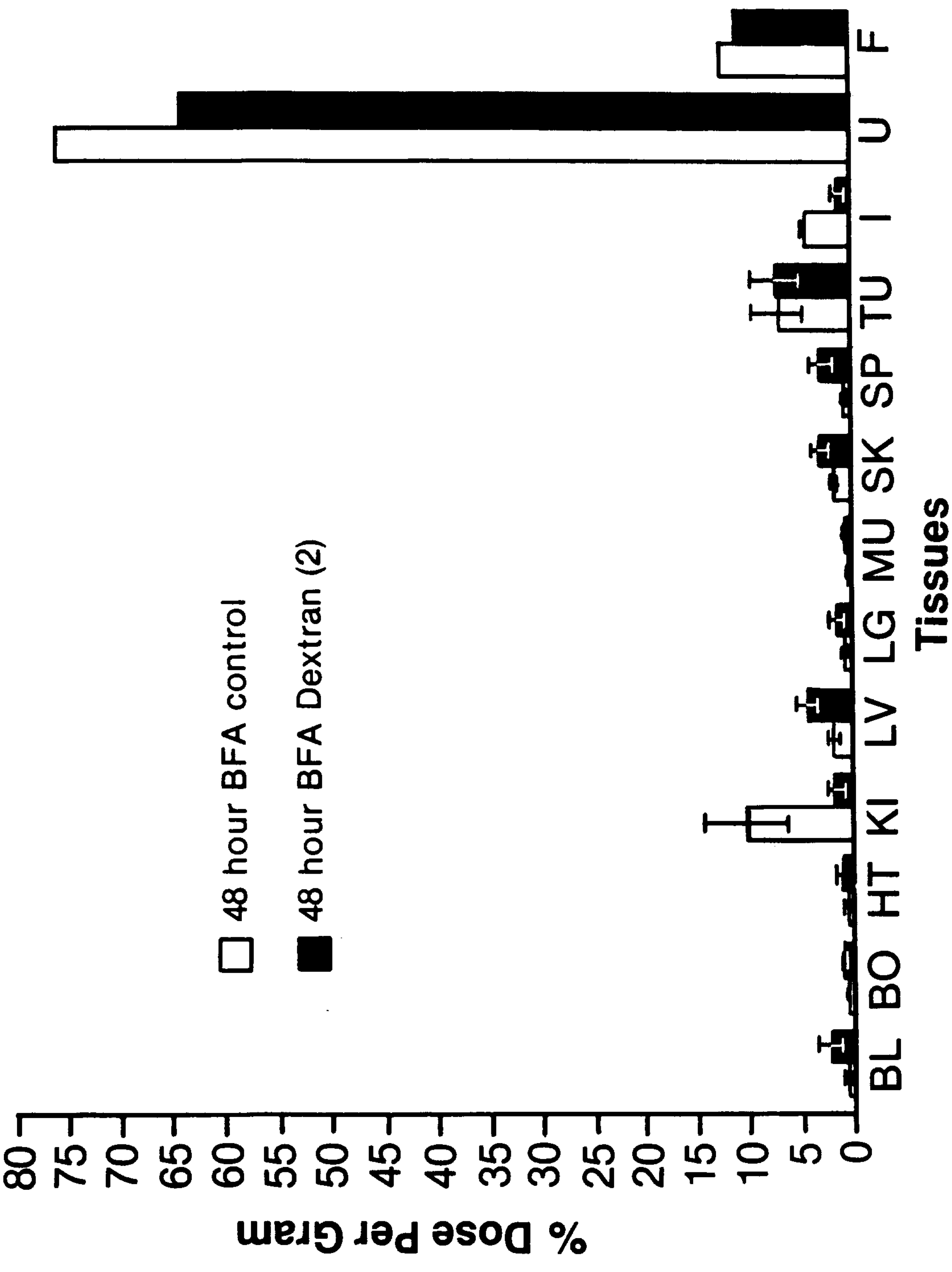
48 hour BFA control
48 hour BFA Dextran (2)
% Dose Per Gram
0
5
10
15
20
25
30
35
40
45
50
55
60
65
70
75
80
BL
BO
HT
KI
LV
LG
MU
SK
SP
TU
I
U
F
Tissues

FIG.14B

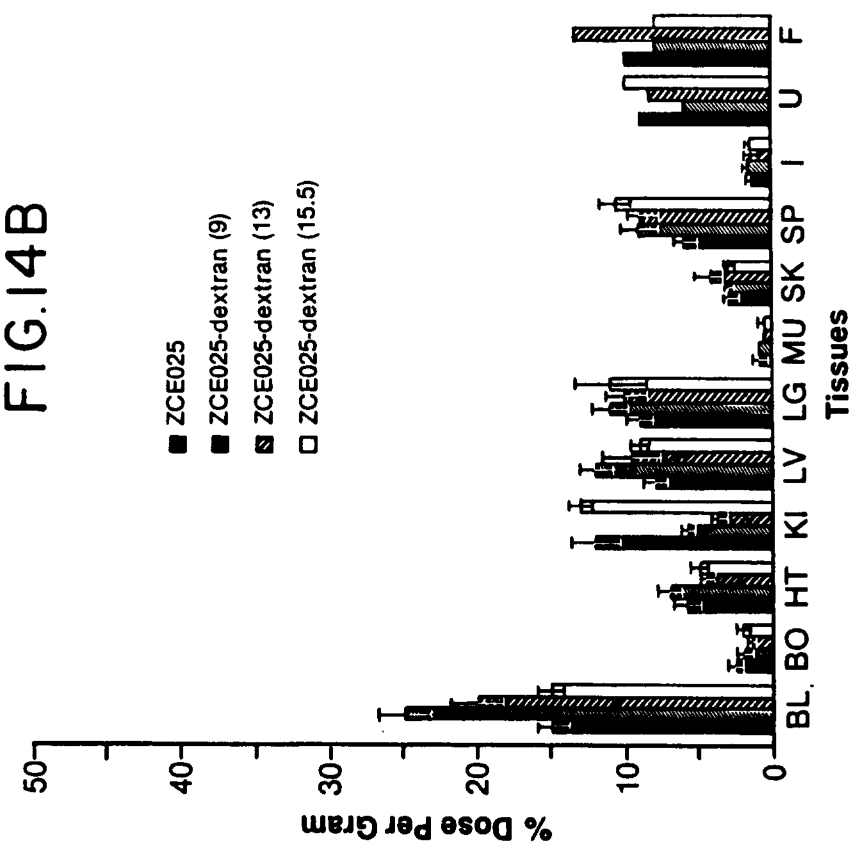


FIG.14A

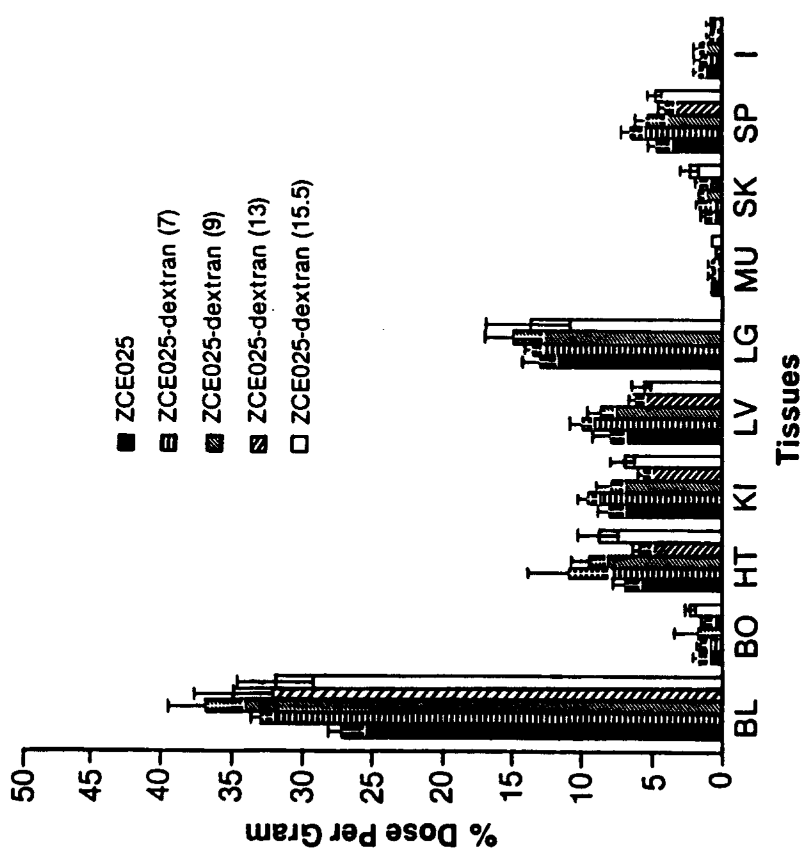

FIG.15

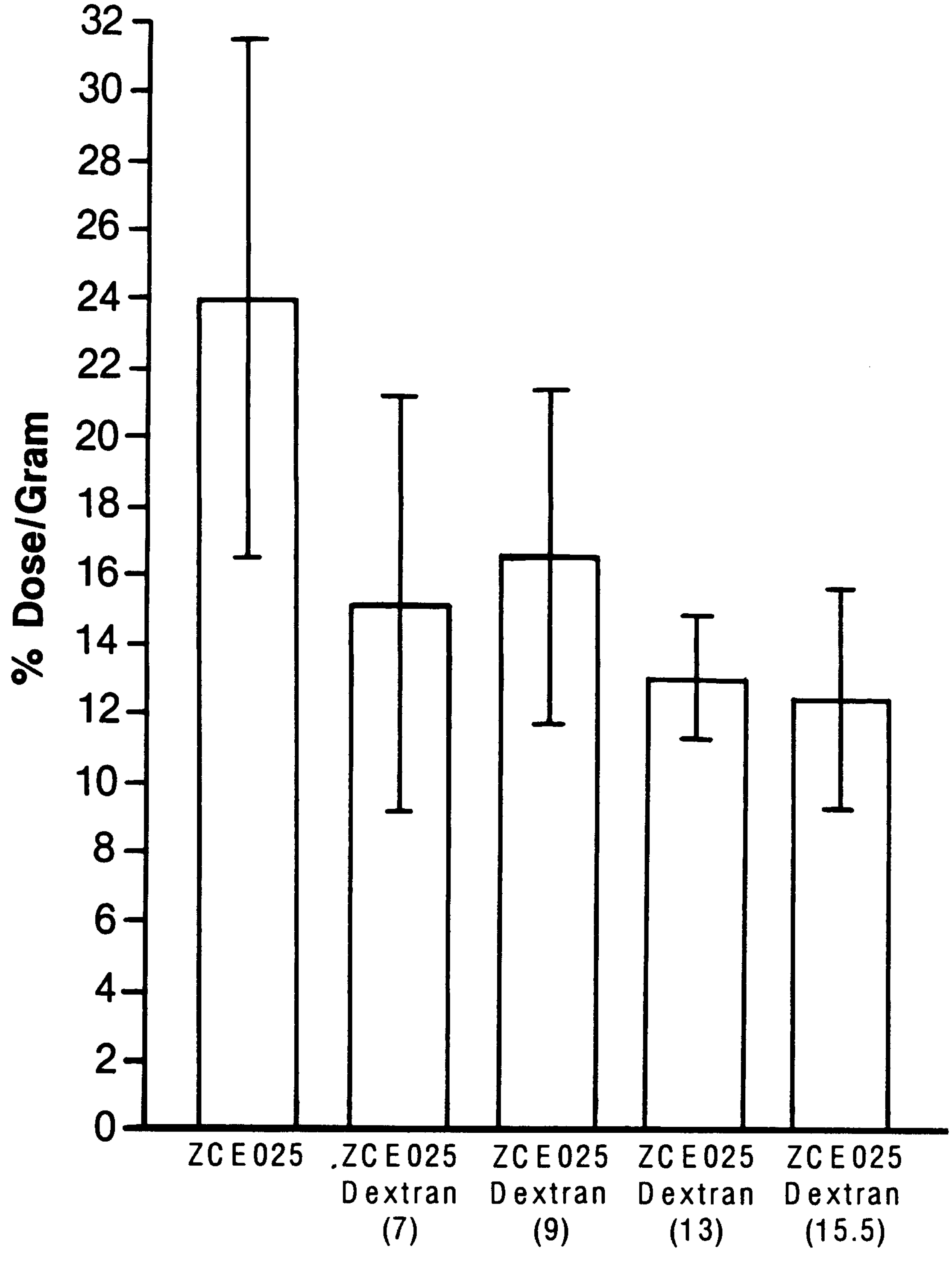
% Dose/Gram
32
30
28
26
24
22
20
18
16
14
12
10
8
6
4
2
0
ZCE025
ZCE025 Dextran (7)
ZCE025 Dextran (9)
ZCE025 Dextran (13)
ZCE025 Dextran (15.5)

FIG.16B

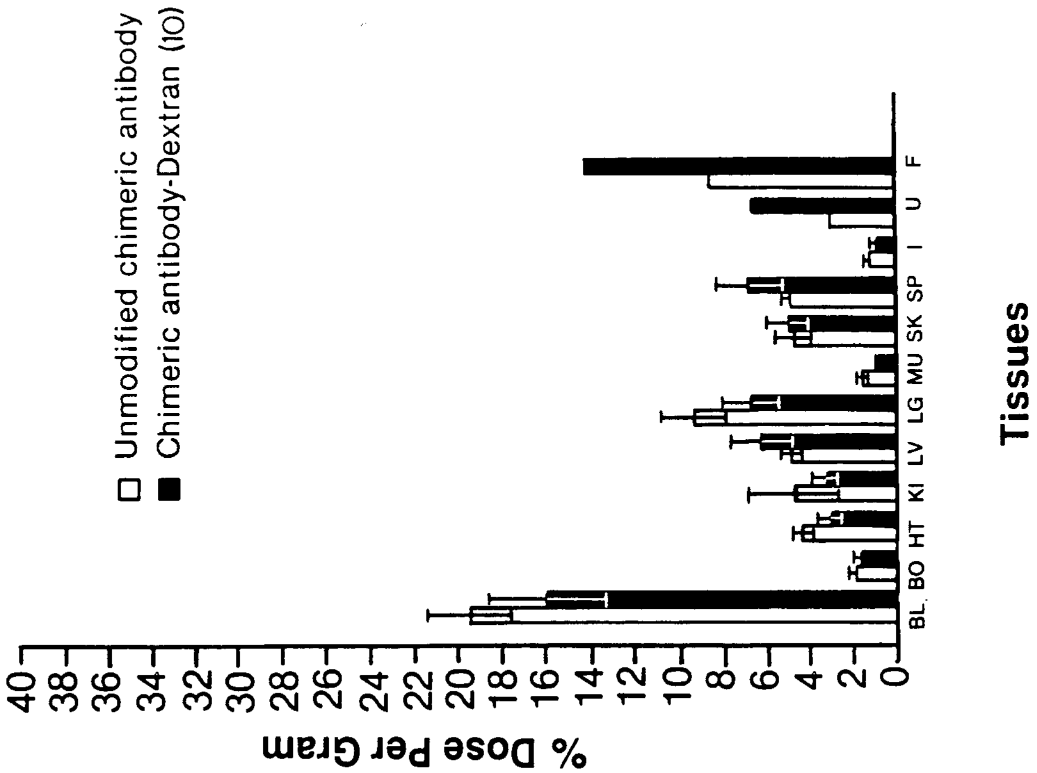


FIG.16A

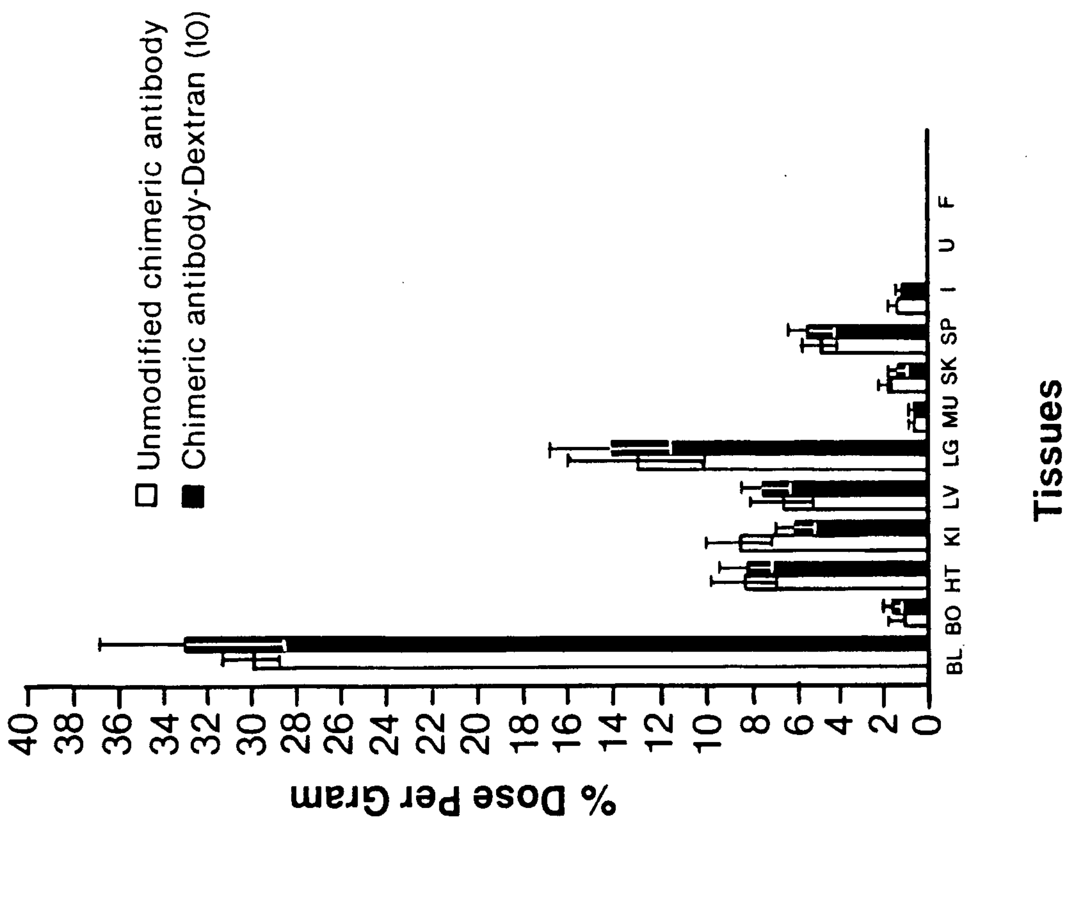

FIG.17

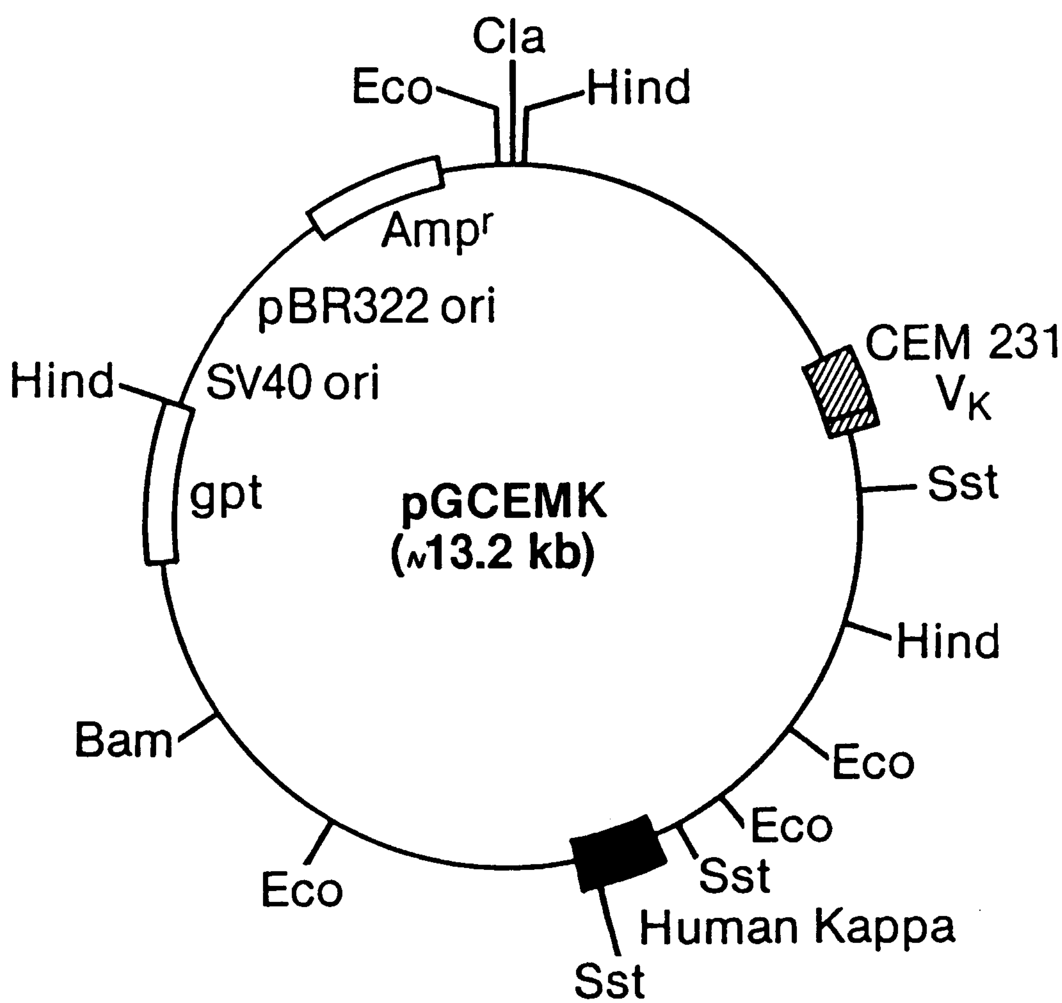
Cla
Eco
Hind
Amp$^r$
pBR322 ori
SV40 ori
Hind
gpt
pGCEMK
(~13.2 kb)
CEM 231
$V_K$
Sst
Hind
Eco
Eco
Sst
Human Kappa
Sst
Bam
Eco

FIG.18

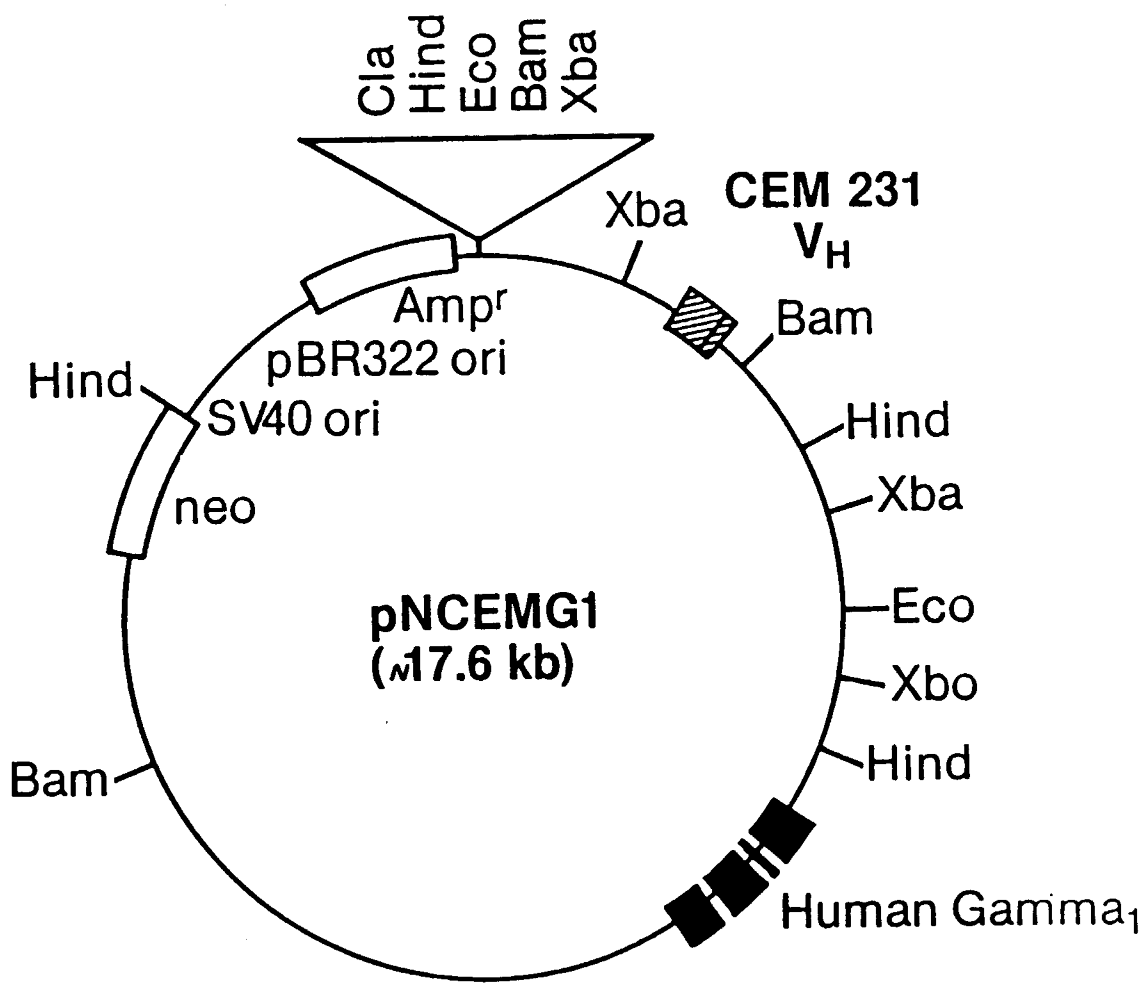
Cla
Hind
Eco
Bam
Xba
Xba
CEM 231
$V_H$
Bam
Hind
Xba
Eco
Xbo
Hind
Human Gamma$_1$
Bam
Hind
SV40 ori
neo
Amp$^r$
pBR322 ori
pNCEMG1
(~17.6 kb)